# EUROPEAN PATENT APPLICATION

(11) **EP 2 786 982 A1**
(43) Date of publication of application: **08.10.2014**
(21) Application number: 12808109.8
(22) Date of filing: 05.07.2012
(51) Int. Cl.: C07C 215/38, C07C 217/48, C07C 217/56, C07C 323/32, C07D 263/32, C07D 207/333, C07D 307/28, C07D 277/24, C07D 277/26, A61K 31/137, A61K 31/421, A61K 31/4164, A61K 31/40, C07C 31/44, A61K 31/341, A61K 31/505, A61K 31/426, A61P 37/02, A61P 37/04, A61P 37/06

(54) **AMINO-PROPYLENE-GLYCOL DERIVATIVES, PREPARATION METHOD AND PHARMACEUTICAL COMPOSITION AND USE THEREOF**

(30) Priority: 06.07.2011 CN 201110188255
(71) Applicant: INSTITUTE OF MATERIA MEDICA, CHINESE ACADEMY OF MEDICAL SCIENCES, Beijing 100050 (CN)
(72) Inventor: HAN, Weijuan, Beijing 100050 (CN); ZHANG, Haijing, Beijing 100050 (CN); WANG, Xiaojian, Beijing 100050 (CN); JIN, Jing, Beijing 100050 (CN); LI, Gang, Beijing 100050 (CN); ZHANG, Yi, Beijing 100050 (CN); XIAO, Qiong, Beijing 100050 (CN); ZHOU, Wanqi, Beijing 100050 (CN); CHEN, Xiaoguang, Beijing 100050 (CN); YIN, Dali, Beijing 100050 (CN)
(74) Representative: Hutter, Anton
(86) International application number: PCT/CN2012/078244
(87) International publication number: WO 2013/004190

(57) **Abstract**

Disclosed are immune-modulators of formula (I), the preparation method thereof, a pharmaceutical composition containing the immune-modulators, and the use of the pharmaceutical composition as a drug, especially as an immune-modulating drug. The compound can be used in immune disorders and immune suppression, and can be used in treating hypo-immunity, rejection after organ transplantation and auto-immune disease.

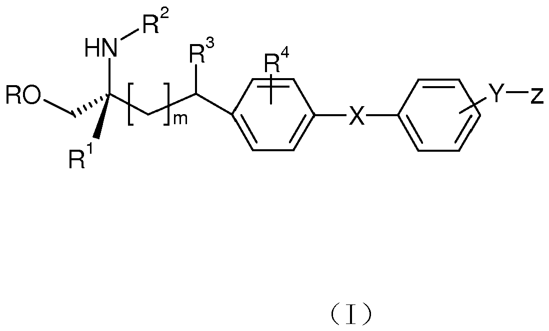

## Description

### Field of the technology

The present invention relates to a novel class of immunomodulators, methods for their preparation, pharmaceutical compositions containing said compounds, their use as drugs, particularly as preventive and therapeutic drugs regulating T lymphocyte-mediated immune diseases belong to medical technology area.

### Background of the technology

Immune response is not only an important defense mechanism for antibody to exclude foreign substances such as bacteria, viruses, and graft, but also an important homeostatic mechanism to prevent autologous cells from mutating and protect against diseases. By affecting the body's immune function, the means of preventing and treating diseases is called immune therapy or immunotherapy.

Immune regulation means that there are stimulation and inhibition between a variety of immune cells and their subsets or between cells and various cytokines in immune response, or both positive phase and negative phase consist of the regulatory network of mutual restraint, completing antigen recognition and response.

Immunomodulators can act on the different aspects of the immune response to play its regulatory role, so that the body's immune response is within the desired range to achieve the purpose of the prevention or treatment of disease. Using useful drugs to facilitate low immune function to return to normal level or prevent immune function deceasing in order to achieve the purpose of prevention is called immune enhancement therapy. Using drugs to suppress the immune function relating to cell proliferation and reduce the immune response is known as immunosuppressive therapy. The drugs are called as immunosuppressants and immunostimulants, collectively immunomodulators.

In clinical therapy, immunosuppressants are mainly used to alleviate rejection reaction after organ transplantation and treat autoimmune diseases. However, the current clinical immunosuppressants have lots of side effects.

The side effects of Glucocorticoid refer to osteonecrosis , poor cataract, edema, hirsutism , high blood sugar , high cholesterol , hypertension, wound healing , myopathy , osteoporosis , peptic ulcer, personality changes , and obesity; The side effects of cyclosporin refer to diarrhea, gingival hyperplasia , headache, hemolytic uremic syndrome, hirsutism , hyperkalemia , high cholesterol , hypertension, hyperuricemia , hypomagnesemia , nausea, renal toxicity , pancreatitis, paralysis, itching , tremor and venous thrombosis ; The side effects of tacrolimus refer to cardiac hypertrophy , low cholesterol , diarrhea , headache , hyperglycemia, hyperkalemia , hypertension , hypomagnesemia , nephrotoxicity , neurotoxicity , nausea , itching and tremor ; sulfur azathioprine effects for cancer , liver toxicity, leukopenia , nausea , pancreatitis and vomiting ; The side effects of mycophenolate mofetil refer to diarrhea , edema , headache , hypertension , bone marrow suppression , nausea, renal toxicity and tremor ; The side effects of leipamicin refer to oral ulcers, joint pain , deep vein thrombosis , edema , headache , high cholesterol, hypertension, interstitial lung disease and Fanconi syndrome (pancytopenia syndrome) and so on .

In summary, the research and development of potent and low toxic immunomodulatory drugs are essential.

In 1990, myriocin (ISP-I), which was found to be a potent immunosuppressive natural product, was isolated from a culture broth of *Isaria sinclairii* by Japanese Fujita *et.al.* ISP-I used to be an antifungal agent also was isolated from fungi broths of Myrioccocum albomyces and Mycelia sterilia, called Myriocin and Thermozymocidin respectively. Lymphocyte proliferation assays effect (MLR) induced by Heterologous lymph glands of rats and cytotoxic T lymphocytes homologous generation experiments (CTL) in vivo indicate that ISP-I is 10-fold the activity of cyclosporine.

In the study of structural modification of ISP-I, Fujita *et. al.* also found that FTY720 has ideal immunosuppressive activity, Currently, a lot of FTY720 derivatives have been reported in relevant literatures. The literatures are as follows. Tetsuro Fujita et. al., Bioorganic & Medicinal Chemistry Letters, 1995, 5,847; Tetsuro Fujita et. al., Bioorganic & Medicinal Chemistry Letters, 1995, 5, 1857; Ryoji Hirose et. al., Bioorganic & Medicinal Chemistry Letters, 1996, 6, 2647; Masatoshi Kiuchi et. al., Bioorganic & Medicinal Chemistry Letters, 1998, 8,101; Tetsuro Fujita e et. al., J. Med. Chem., 1996, 39, 4451; Masatoshi Kiuchi et. al., J. Med. Chem., 2000, 43, 2946. However, all of these literatures are different from the compounds FTY720 derivatives involved in the present invention.

### Content of the invention

After long-term research, the present invention has been found that the new FTY720 derivatives depicted in detail later have excellent immunomodulatory activity, especially in terms of immunosuppressive activity exhibiting excellent medicinal properties. The invention has been completed on the above basis.

One aspect of the invention provides potent and low toxic immune modulators, such as Formula (I) compounds and their stereoisomers.

A further aspect of the present invention refers to pharmaceutical composition comprising the general formula (I) compounds as active ingredients or their stereoisomers.

Further aspect the present invention relates to the use in prevention and / or treatment of immune regulation of formula (I) compounds or pharmaceutical composition containing them.

Further aspect the present invention relates to the methods of prevention and / or treatment of the immune system disease, including that formula (I) compounds or pharmaceutical composition containing them are administered in the host which need to be prevented and / or treated.

The present invention relates to compounds represented by the general formula (I) and the compounds including pharmaceutically acceptable salts and esters. 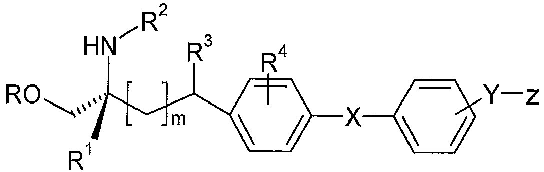 Wherein R is selected from the group consisting of hydrogen, C1-6 alkyl, C1-6 acyl group, sulfonate group,-P (= O) (OR') (OR "), wherein the OR' and OR" are the same or different, R ', and R "are independently selected from hydrogen, C1-6 alkyl, C1-6 acyl group;
R¹ is selected from the group consisting of hydrogen, substituted or unsubstituted C1-6 alkyl group, and the substituents are selected from the group consisting of halogen, carbonyl group, hydroxyl group, mercapto group, cyano group, amino group and sulfonate group;
R² is selected from the group consisting of hydrogen, substituted or unsubstituted C1-8 alkoxy group, and the substituents are selected from the group consisting of halo, carbonyl, hydroxy, mercapto, cyano, amino and phenyl;
R³ is selected from the group consisting of hydrogen or hydroxy;
M is an integer selected from 0 to 4;
R⁴ is selected from the group consisting of hydrogen, hydroxy, mercapto, amino, aldehyde, carboxyl, carbamoyl, halogen, nitro, cyano, C1-6 alkyl, C1-6 alkoxy, C1-6 alkylamino, C1-6 alkylamino group wherein the alkylamino group include mono-and bis-alkylamino, C1-6 alkoxy C1-6 alkyl group, C1-6 acyl, C1-6 acyloxy, C1-6 acylamino group, C1-6 haloalkyl and C2-6 olefins;
X is selected from the group consisting of oxygen, sulfur or single bond, phenyl group is directly linked with phenyl group when X is a single bond;
When X is selected from the group consisting of oxygen and sulfur; Y is selected from the group consisting of C0-8 alkyl group, a C1-8 alkoxy, C2-8 olefin, a five-or six-membered aryl, five-or six-membered heterocycle ring containing 1, 2 or 3 heteroatoms,the heteroatoms can be the same or different and selected from the group consisting of N, O, and S; when Y is selected from the group consisting of C0 alkyl group, it represents Y deletion. In other words, Z is directly connected to the benzene ring;
When X is a single bond; Y is selected from the group consisting of a five-or six-membered aryl, five-or six-membered heterocycle ring containing 1, 2 or 3 heteroatoms,the heteroatoms can be the same or different and selected from the group consisting of N, O, and S; when Y is selected from the group consisting of C0 alkyl group, it represents Y deletion. In other words, Z is directly connected to the benzene ring;
Z is selected from the group consisting of hydrogen, hydroxy, mercapto, amino, aldehyde, carboxyl, carbamoyl, halogen, nitro, cyano, C1-6 alkyl, C1-6 alkoxy, C1-6 alkylthio, C1-6 alkylamino group wherein the alkylamino group include mono-and bis-alkylamino, C1-6 alkoxy C1-6 alkyl group, C1-6 acyl" C1-6 acyloxy, C1-6 acylamino group, C1-6 haloalkyl and C2-6 olefins.

Preferably, five-membered aryl is selected from

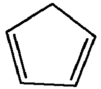

Preferably, six-membered aryl is selected from

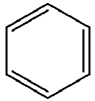

Preferably, five-membered aryl containing 1-4 heteroatoms selected from N, O, or S is selected from

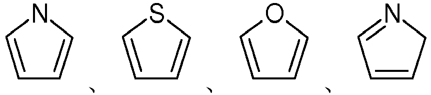

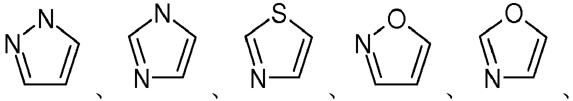

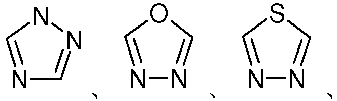

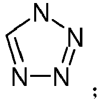

Preferably, six-membered aryl containing 1-4 heteroatoms selected from N, O, or S is selected from

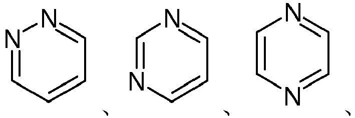

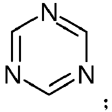

More preferred heterocyclic is selected from

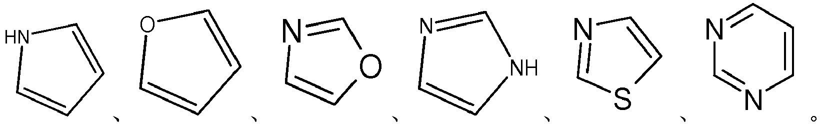

Preferred compounds represented by the formula I and pharmaceutically acceptable salts and esters thereof include but not limit to the compounds shown in IA. 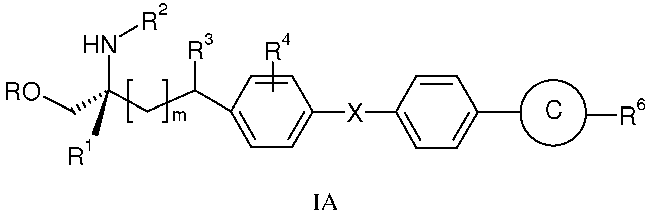 Wherein R is selected from hydrogen, C1-4 alkyl, C1-4 acyl group, sulfonate group,-P (= O) (OR') (OR "), wherein the OR' and OR" are the same or different, R ', and R "are independently selected from hydrogen, C1-4 alkyl, C1-4 acyl group;
R¹ is selected from the group consisting of hydrogen, substituted or unsubstituted C1-4 alkyl group, and the substituents are selected from the group consisting of halogen, carbonyl group, hydroxyl group, mercapto group, cyano group, amino group and sulfonate group;
R² is selected from the group consisting of hydrogen, substituted or unsubstituted C1-6 alkoxy group, and the substituents are selected from the group consisting of halo, carbonyl, hydroxy, mercapto, cyano, amino and phenyl;
R³ is selected from the group consisting of hydrogen or hydroxy;
M is an integer selected from 1 to 3;
R⁴ is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino, C1-4 alkylamino group wherein the alkylamino group include mono-and bis-alkylamino, C1-4 alkoxy C1-4 alkyl group, C1-4 acyl, C1-4 acyloxy, C1-4 acylamino group, C1-4 haloalkyl and C2-4 olefins;
X is selected from the group consisting of oxygen and sulfur;
C ring is selected from 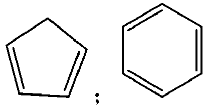 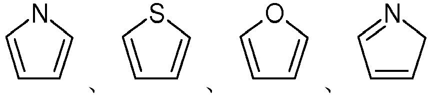 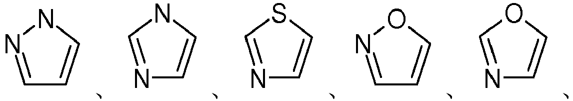 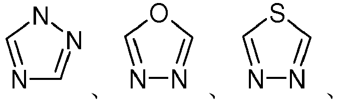 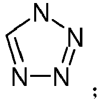 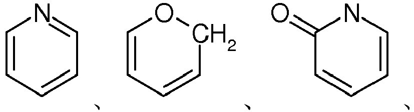 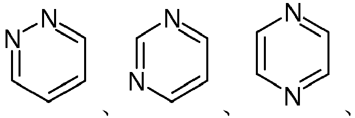 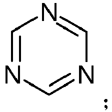 R⁶ is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino, C1-4 alkylamino group wherein the alkylamino group include mono-and bis-alkylamino, C1-4 alkoxy C1-4 alkyl group, C1-4 acyl, C1-4 acyloxy, C1-4 acylamino group, C1-4 haloalkyl and C2-4 olefins;

Preferred compounds represented by the formula I and pharmaceutically acceptable salts and esters thereof include but not limit to the compounds shown in IB. 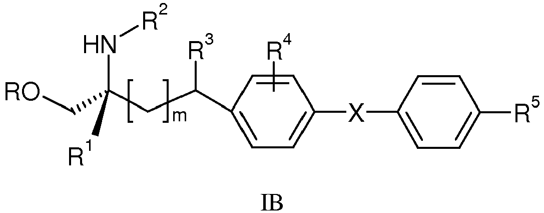 Wherein R is selected from hydrogen, C1-4 alkyl, C1-4 acyl group, sulfonate group,-P (= O) (OR') (OR "), wherein the OR' and OR" the same or different, R', and R "are independently selected from hydrogen, C1-4 alkyl, C1-4 acyl group;
R¹ is selected from the group consisting of hydrogen, substituted or unsubstituted C1-4 alkyl group, and the substituents are selected from the group consisting of halogen, carbonyl group, hydroxyl group, mercapto group, cyano group, amino group and sulfonate group;
R² is selected from the group consisting of hydrogen, substituted or unsubstituted C1-6 alkoxy group, and the substituents are selected from the group consisting of halo, carbonyl, hydroxy, mercapto, cyano, amino and phenyl;
R³ is selected from the group consisting of hydrogen or hydroxy;
M is an integer selected from 1 to 3;
R⁴ is selected from the group consisting of hydrogen, halogen, hydroxy, C1-4 alkyl, C1-4 alkoxy, C1-4 acyl, C1-4 acyloxy, C1-4 alkylthio, amino, C1-4 alkoxy group alkylamino group wherein the alkylamino group include mono-and bis-alkylamino, C1-4 acylamino group, C1-4 haloalkyl, mercapto, C1-4 alkylthio and C2-4 olefins;
X is selected from the group consisting of oxygen and sulfur;
R⁵ is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino, C1-4 alkylamino group wherein the alkylamino group include mono-and bis-alkylamino, C1-4 alkoxy C1-4 alkyl group, C1-4 acyl, C1-4 acyloxy, C1-4 acylamino group, C1-4 haloalkyl or C2-4 olefins;

Preferred compounds represented by the formula I and pharmaceutically acceptable salts and esters thereof include but not limit to the compounds shown in IC. 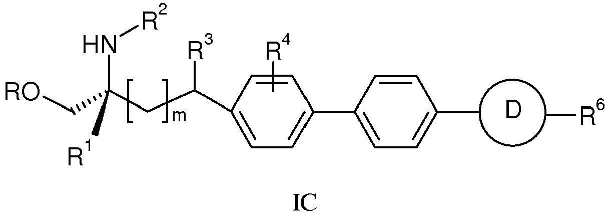 Wherein R is selected from hydrogen, C1-4 alkyl, C1-4 acyl group, sulfonate group,-P (= O) (OR') (OR "), wherein the OR' and OR" the same or different, R', and R "are independently selected from hydrogen, C1-4 alkyl, C1-4 acyl group;
R¹ is selected from the group consisting of hydrogen, substituted or unsubstituted C1-4 alkyl group, and the substituents are selected from the group consisting of halogen, carbonyl group, hydroxyl group, mercapto group, cyano group, amino group and sulfonate group;
R² is selected from the group consisting of hydrogen, substituted or unsubstituted C1-6 alkoxy group, and the substituents are selected from the group consisting of halo, carbonyl, hydroxy, mercapto, cyano, amino and phenyl;
R³ is selected from the group consisting of hydrogen or hydroxy;
m is an integer selected from 1 to 3;
R⁴ is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino, C1-4 alkylamino group wherein the alkylamino group include mono-and bis-alkylamino, C1-4 alkoxy C1-4 alkyl group, C1-4 acyl, C1-4 acyloxy, C1-4 acylamino group, C1-4 haloalkyl or C2-4 olefins;
D ring is selected from 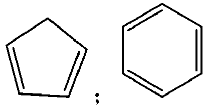 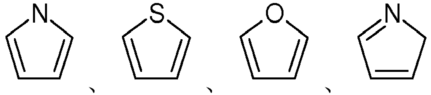 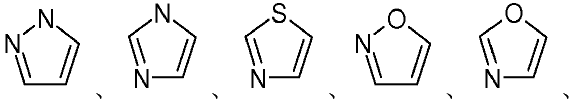 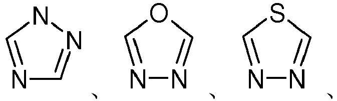 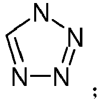 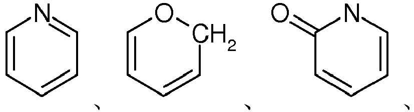 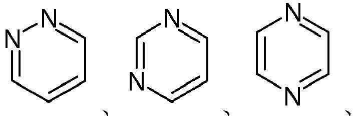 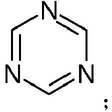 R⁶ is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino, C1-4 alkylamino group wherein the alkylamino group include mono-and bis-alkylamino, C1-4 alkoxy C1-4 alkyl group, C1-4 acyl, C1-4 acyloxy, C1-4 acylamino group, C1-4 haloalkyl or C2-4 olefins;

Preferred compounds represented by the formula IA and pharmaceutically acceptable salts and esters thereof include but not limit to the below compounds. 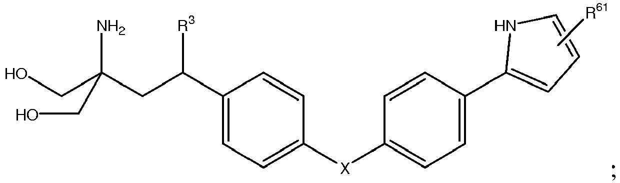 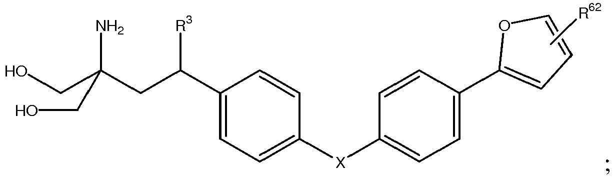 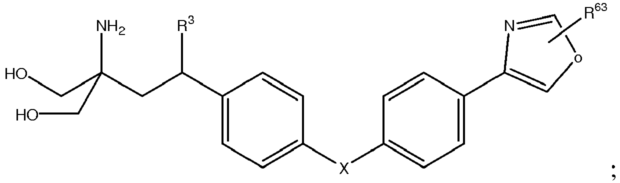 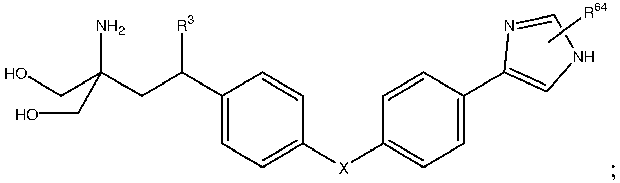 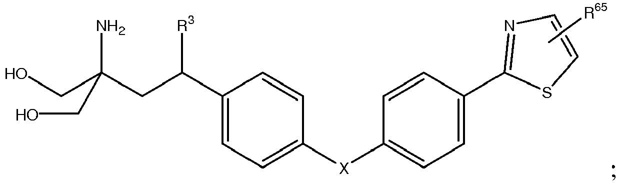 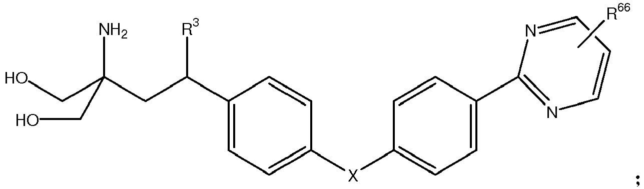 R³ is selected from the group consisting of hydrogen or hydroxy;
X is selected from the group consisting of oxygen and sulfur;
R⁶¹, R⁶², R⁶³, R⁶⁴, R⁶⁵ and R⁶⁶ are independently selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino, C1-4 alkylamino group wherein the alkylamino group include mono-and bis-alkylamino, C1-4 alkoxy C1-4 alkyl group, C1-4 acyl, C1-4 acyloxy, C1-4 acylamino group, C1-4 haloalkyl or C2-4 olefins;

Further preferred compounds represented by the formula IA and pharmaceutically acceptable salts and esters thereof include but not limit to the below compounds.

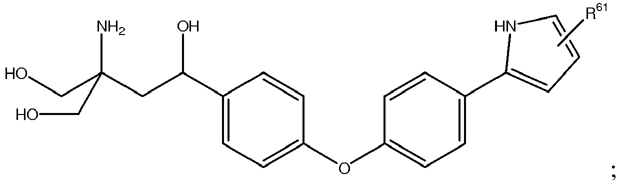

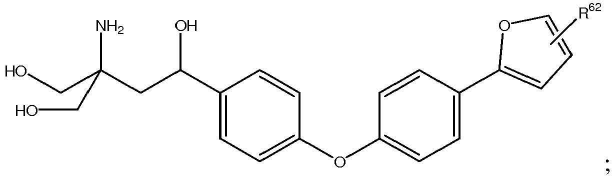

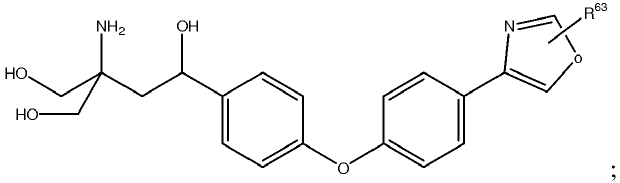

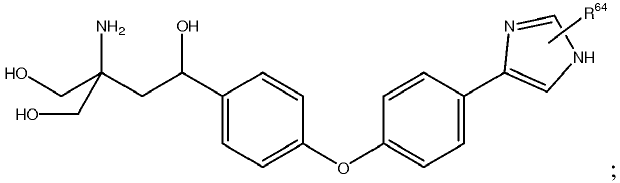

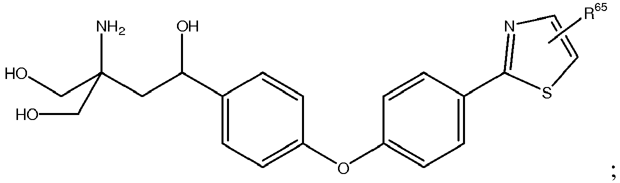

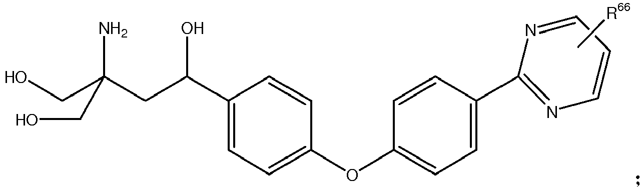

R⁶¹, R⁶², R⁶³, R⁶⁴, R⁶⁵ and R⁶⁶ are independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino , ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group amino group, tert-butyl amino group, vinyl group, propenyl, allyl, butenyl;

Further preferred compounds represented by the formula IA and pharmaceutically acceptable salts and esters thereof include but not limit to the below compounds.

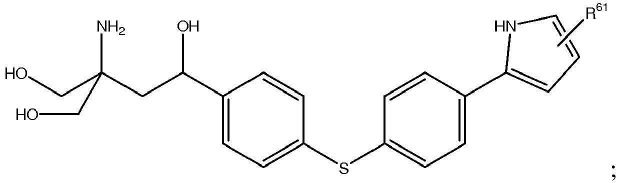

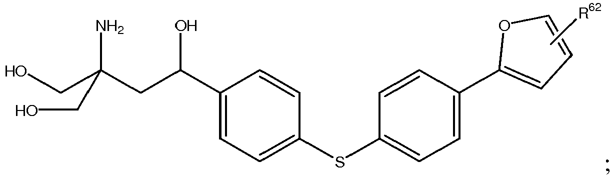

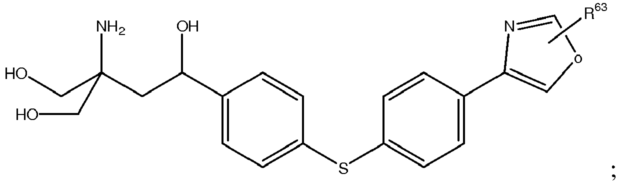

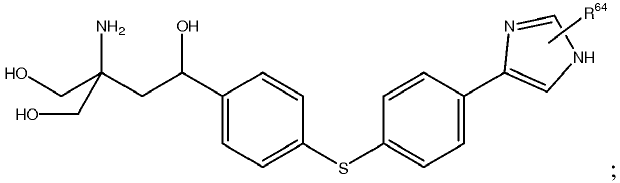

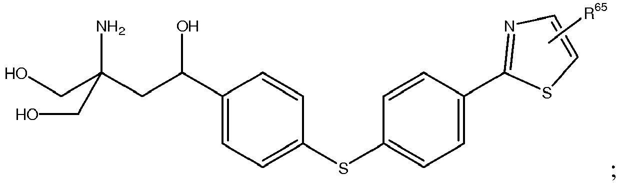

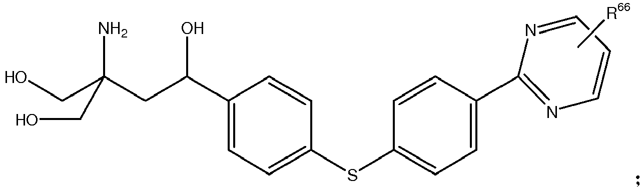

R⁶¹, R⁶², R⁶³, R⁶⁴, R⁶⁵ and R⁶⁶ are independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino , ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group amino group, tert-butyl amino group, vinyl group, propenyl, allyl, butenyl;

Further preferred compounds represented by the formula IA and pharmaceutically acceptable salts and esters thereof include but not limit to the below compounds.

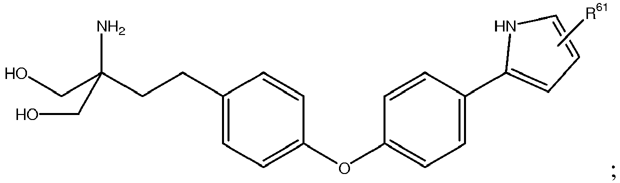

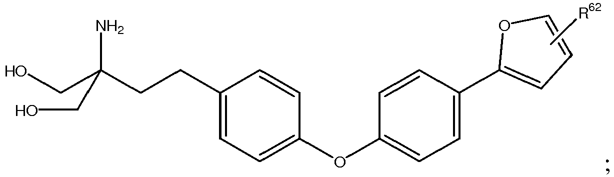

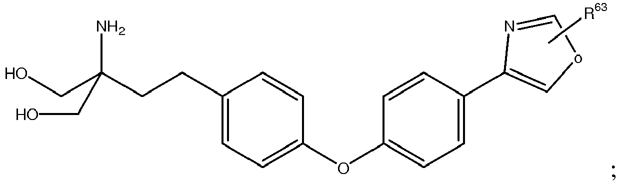

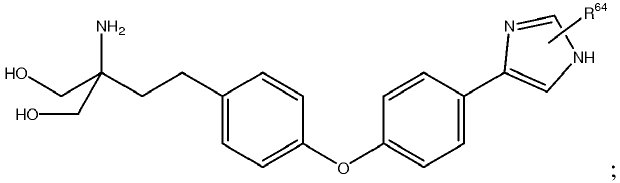

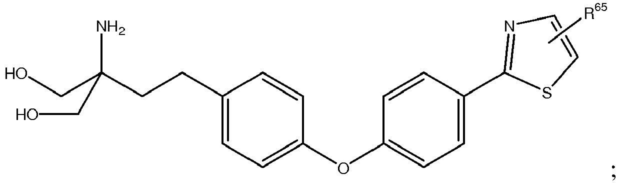

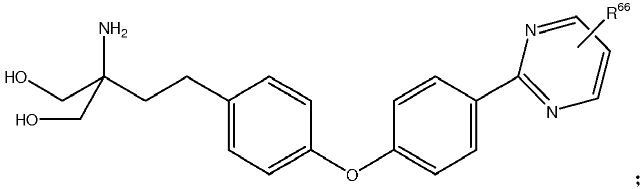

R⁶¹, R⁶², R⁶³, R⁶⁴, R⁶⁵ and R⁶⁶ are independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino , ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group amino group, tert-butyl amino group, vinyl group, propenyl, allyl, butenyl;

Further preferred compounds represented by the formula IA and pharmaceutically acceptable salts and esters thereof include but not limit to the below compounds.

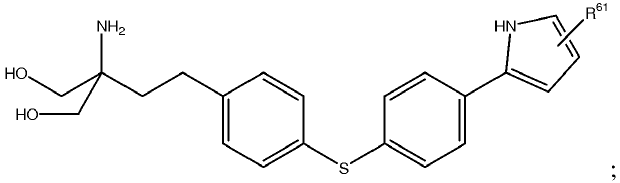

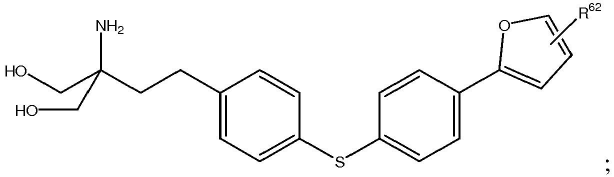

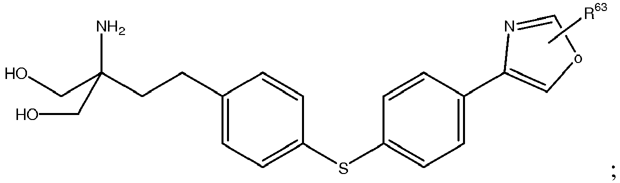

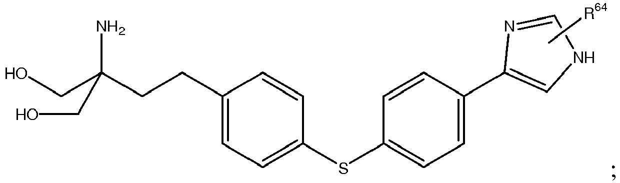

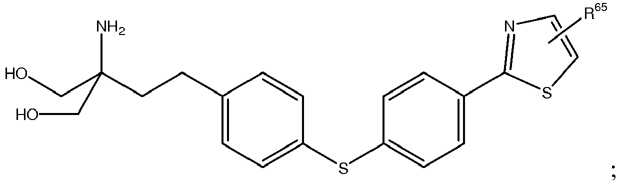

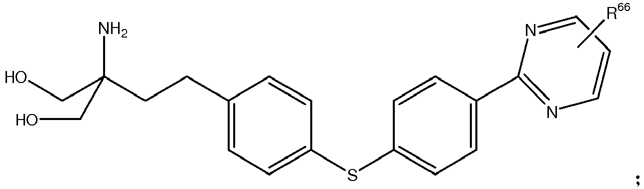

R⁶¹, R⁶², R⁶³, R⁶⁴, R⁶⁵ and R⁶⁶ are independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino , ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group amino group, tert-butyl amino group, vinyl group, propenyl, allyl, butenyl;

Preferred compounds represented by the formula IB and pharmaceutically acceptable salts and esters thereof include but not limit to the below compounds. 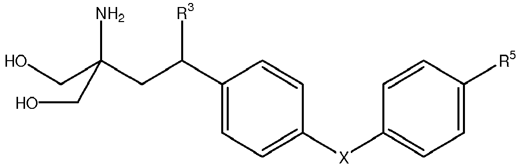 R³ is selected from the group consisting of hydrogen or hydroxy;
R⁵ is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino, C1-4 alkylamino group wherein the alkylamino group include mono-and bis-alkylamino, C1-4 alkoxy C1-4 alkyl group, C1-4 acyl, C1-4 acyloxy, C1-4 acylamino group, C1-4 haloalkyl or C2-4 olefins;

Further preferred compounds represented by the formula IB and pharmaceutically acceptable salts and esters thereof include but not limit to the below compounds.

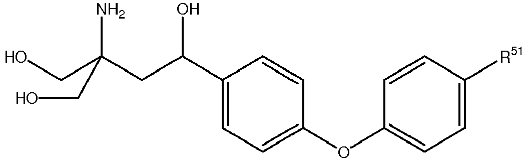

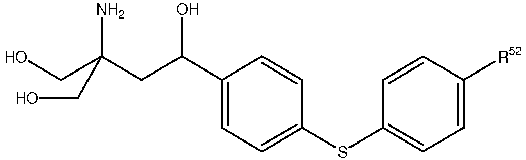

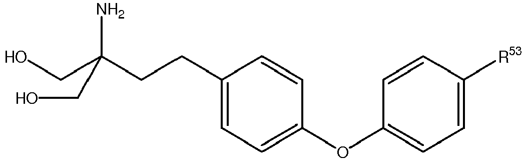

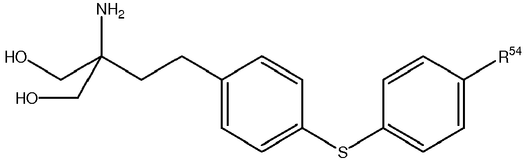

R⁵¹, R⁵², R⁵³ and R⁵⁴ are independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino , ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group amino group, tert-butyl amino group, vinyl group, propenyl, allyl, butenyl.

Preferred compounds represented by the formula IC and pharmaceutically acceptable salts and esters thereof include but not limit to the below compounds. 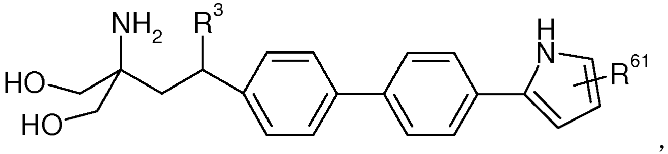 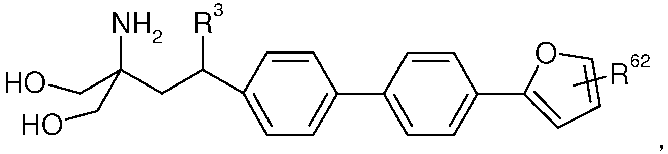 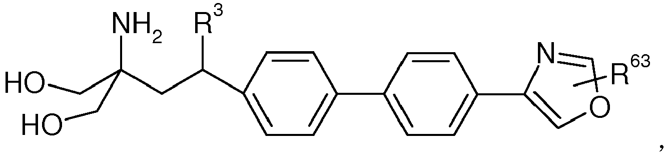 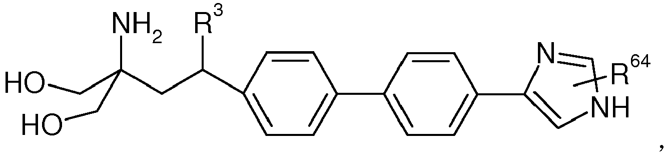 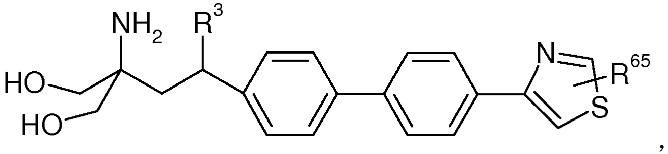 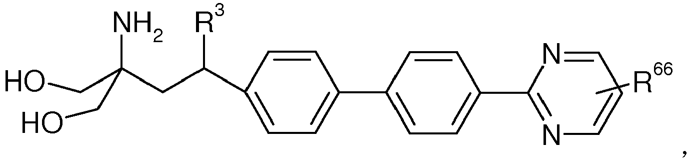 R³ is selected from the group consisting of hydrogen or hydroxy;
R⁶¹, R⁶², R⁶³, R⁶⁴, R⁶⁵ and R⁶⁶ are independently selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino, C1-4 alkylamino group wherein the alkylamino group include mono-and bis-alkylamino, C1-4 alkoxy C1-4 alkyl group, C1-4 acyl, C1-4 acyloxy, C1-4 acylamino group, C1-4 haloalkyl or C2-4 olefins;

Further preferred compounds represented by the formula IC and pharmaceutically acceptable salts and esters thereof include but not limit to the below compounds.

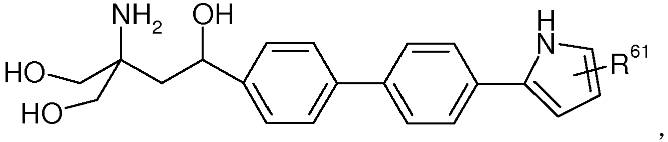

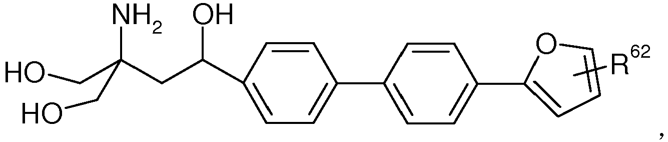

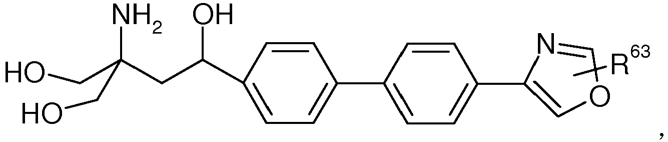

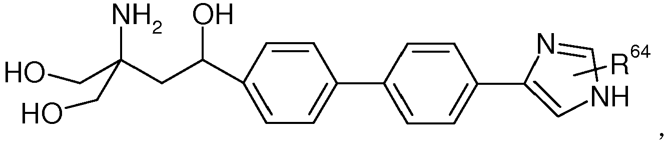

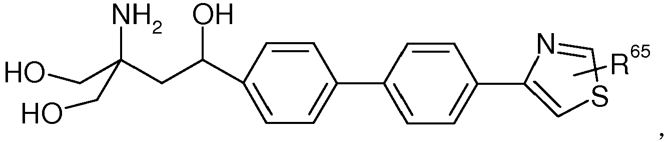

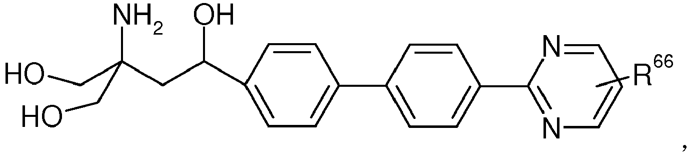

R⁶¹, R⁶², R⁶³, R⁶⁴, R⁶⁵ and R⁶⁶ are independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino , ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group amino group, tert-butyl amino group, vinyl group, propenyl, allyl, butenyl.

Further preferred compounds represented by the formula IC and pharmaceutically acceptable salts and esters thereof include but not limit to the below compounds.

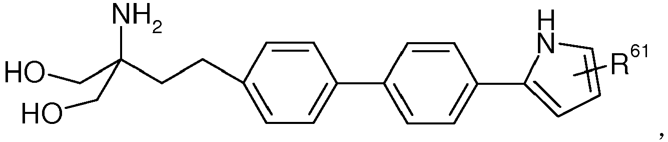

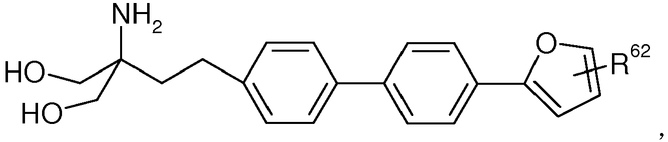

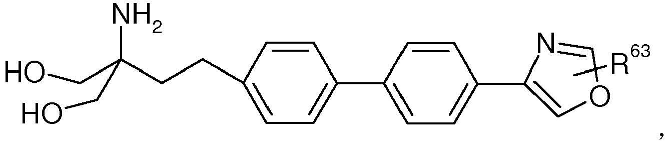

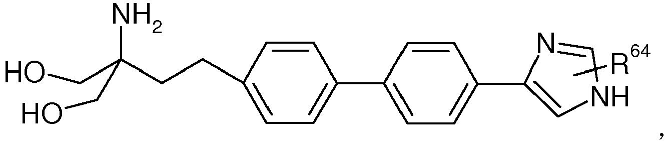

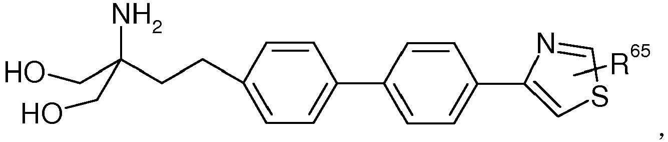

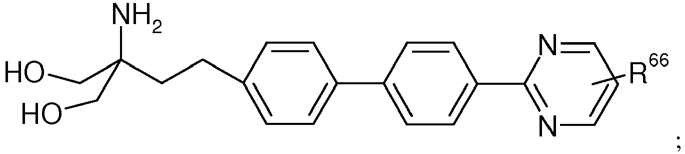

R⁶¹, R⁶², R⁶³, R⁶⁴, R⁶⁵ and R⁶⁶ are independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino , ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group amino group, tert-butyl amino group, vinyl group, propenyl, allyl, butenyl.

In the present invention, the term "alkyl" refers to straight-chain or branched-chain alkyl group containing one or more carbon atoms such as methyl, ethyl, propyl, isopropyl, n-butyl, iso-butyl , sec-butyl, tert-butyl, pentyl, isopentyl, neopentyl, sec-pentyl, hexyl, isohexyl, sec-hexyl, heptyl, octyl, nonyl, decyl and the like.

In the present invention, the term "hydrocarbyl" refers to a free or containing one or more double or triple bonds. The alkyl group is as defined above.

The most preferred compounds are selected from

| | |
|---|---|
| 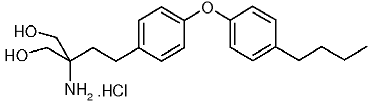 | 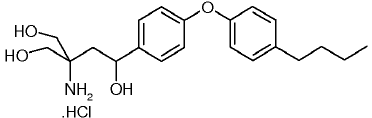 |
| **Compound No. 926** | **Compound No**.**925** |
| 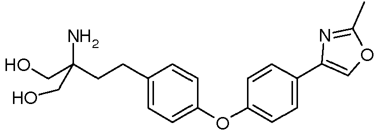 | 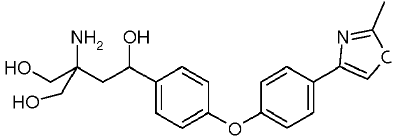 |
| **Compound No**.**933** | **Compound No**.**959** |
| 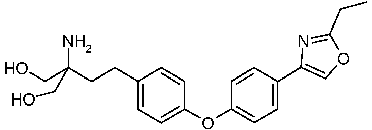 | 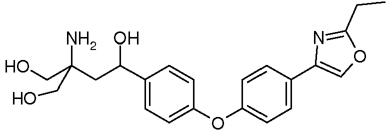 |
| **Compound No**.**934** | **Compound No**.**960** |
| 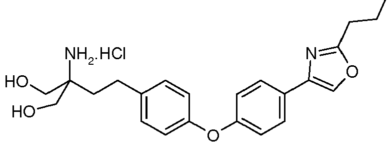 | 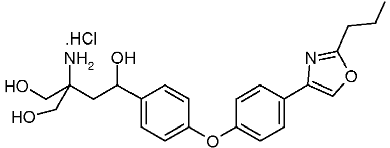 |
| **Compound No**.**978** | **Compound No**.**961** |
| 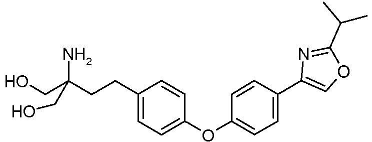 | 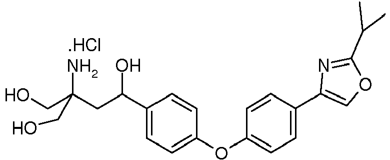 |
| **Compound No**.**935** | **Compound No**.**962** |
| 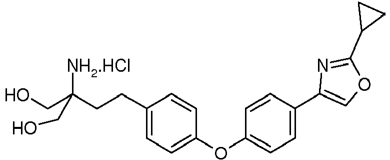 | 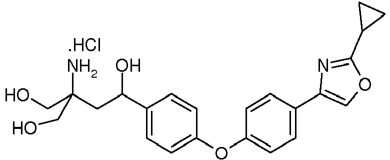 |
| **Compound No**.**955** | **Compound No**.**963** |
| | |
| 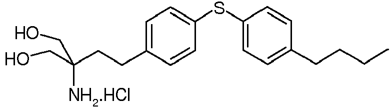 | 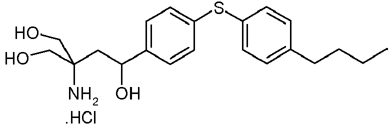 |
| **Compound No**.**958** | **Compound No**.**957** |
| 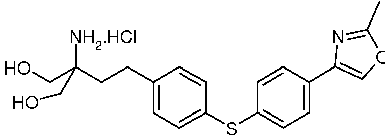 | 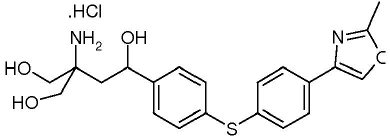 |
| **Compound No.973** | **Compound No.965** |
| 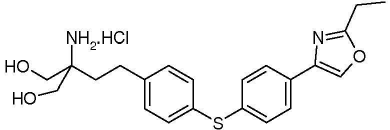 | 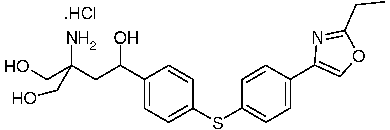 |
| **Compound No.974** | **Compound No.966** |
| 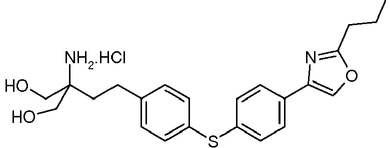 | 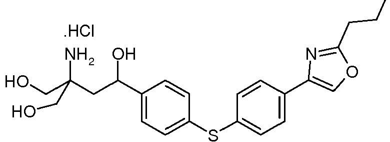 |
| **Compound No.975** | **Compound No.967** |
| 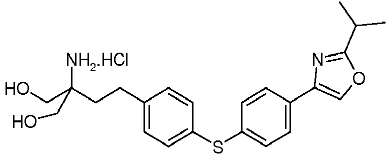 | 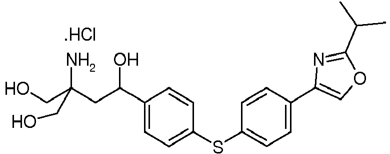 |
| **Compound No.976** | **Compound No.968** |
| 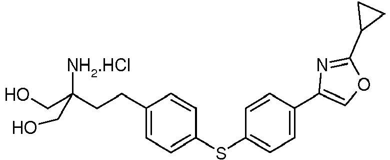 | 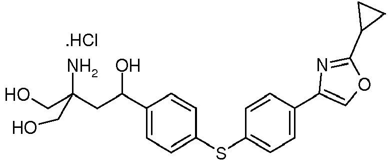 |
| **Compound No.977** | **Compound No.969** |
| | |
| 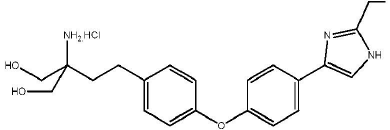 | 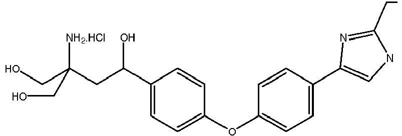 |
| **Compound No.979** | |
| 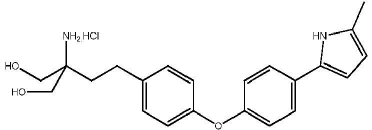 | 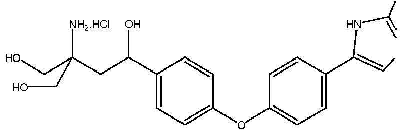 |
| **Compound No.980** | |
| 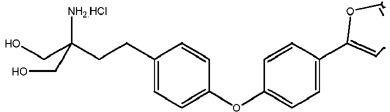 | 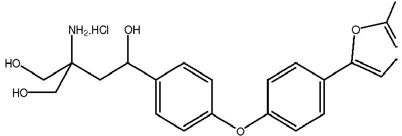 |
| **Compound No.981** | |
| 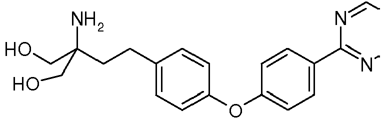 | 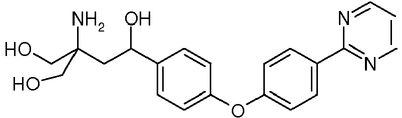 |
| | **Compound No**.**1301** |
| 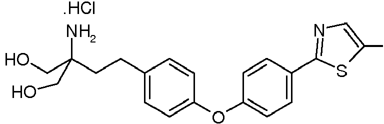 | 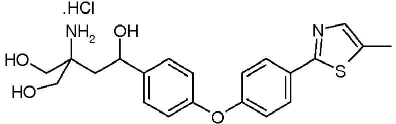 |
| | **Compound No**.**983** |
| | |
| 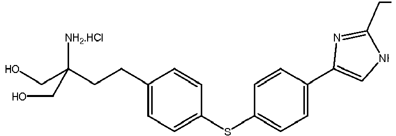 | 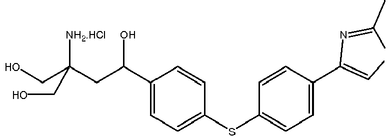 |
| 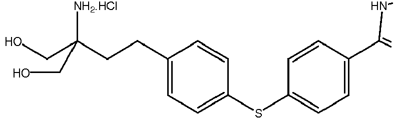 | 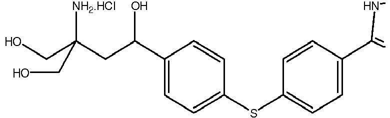 |
| 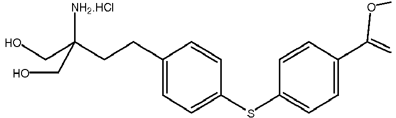 | 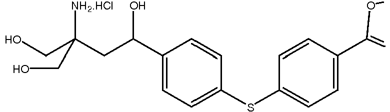 |
| 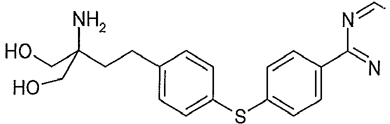 | 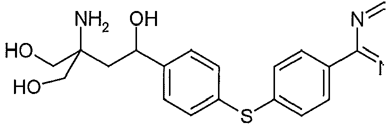 |
| 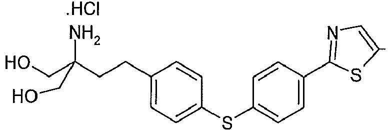 | 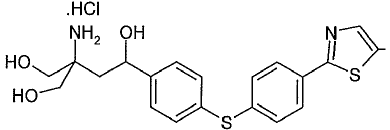 |
| | |
| 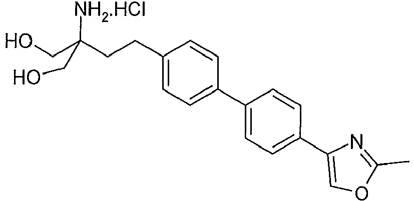 | |
| **Compound No**.**932** | |
| 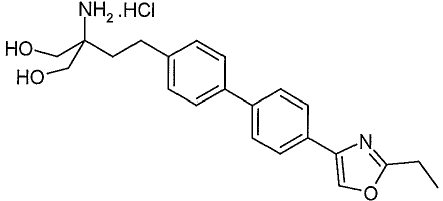 | |
| **Compound No**.**927** | |
| 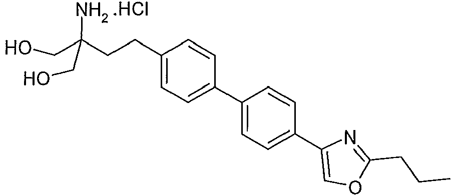 | |
| **Compound No**.**930** | |
| 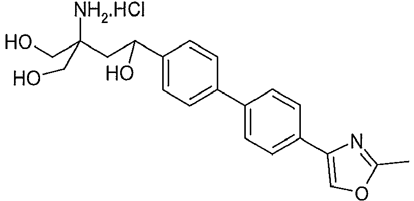 | |
| 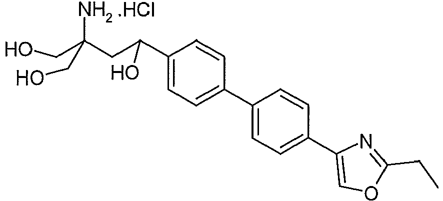 | |
| 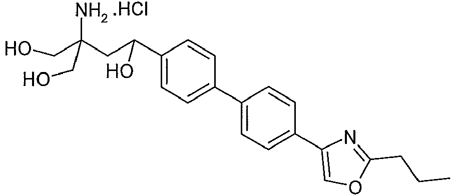 | |

The present invention relates to the general formula (1) compounds in the form of pharmaceutically acceptable salts, and / or solvates thereof simultaneously.

Examples of the general formula (1) include inorganic acid salts such as hydrochloride, hydrobromide, sulfate and phosphate, and organic acid salts such as acetate, fumarate, maleate, benzoate, citrate, succinate, malate, methanesulfonate, benzenesulfonate and tartrate. When the general formula (1) compounds are applied in the form of salt, the salts tends to be acceptable pharmaceutically. The present invention also includes the general formula (1) compounds or their hydrates and solvates in the form of salt thereof.

According to the present invention, general formula (I) compounds can exist in the form of isomers, and the typical said compounds of the present convention include the isomers.

The general formula (I) compounds exist cis-trans isomerism of the double bond. Asymmetric center contains the R configuration or the S configuration. The present invention includes all possible stereoisomers and mixtures of two or more isomers. If cis / trans isomers exist, the present invention refers to the forms of cis and trans and mixtures of these forms. If needed, the individual isomers can be isolated or prepared by stereoselective synthesis by conventional methods.

The compounds of this invention can be prepared by the following method (method A as shown in the schematic), such as method A

### A-1: Synthetic route of R³=OH

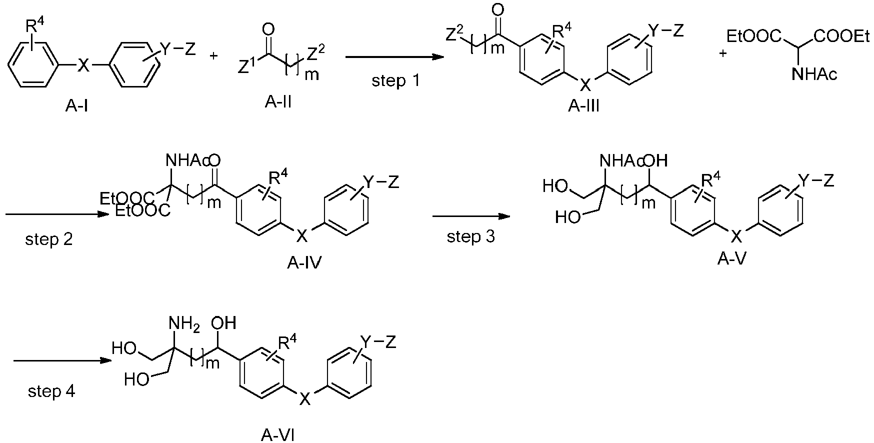

Step 1 is Friedel-Crafts acylation reaction. Formula (A-III)compounds can be prepared by the reactions between formula(A- I) compounds and the formula (A- II) compounds with the Lewis acid catalyst in any suitable reaction solvent (e.g., methylene chloride, carbon disulfide). Required Lewis acids for the reactions are selected from any suitable acids, preferably aluminum chloride.

Step 2 is condensation reaction. Formula (A-IV)compounds can be prepared by the reactions between formula(A-III) compounds and diethyl acetamidomalonate in any suitable reaction solvent (e.g., ethanol, tetrahydrofuran). Required bases for the reactions select from any suitable bases, preferably sodium alkoxide, sodium hydroxide.

Step 3 is reduction reaction. Formula (A- V)compounds can be prepared by the reduction of formula(A-IV) compounds with the reducing agents in any suitable reaction solvent (e.g., ethanol, water). Preferred reducing agents are selected from the metal reducing agents; preferably metal reducing agents are selected from lithium aluminium hydride, sodium borohydride, lithium borohydride or diborane.

Step 4 is hydrolysis reaction. Formula (A-VI)compounds can be prepared by the hydrolysis of formula(A- V) compounds in any suitable reaction solvent (e.g., methanol, ethanol)). This reaction can be catalyzed by acids or bases, which are common in organic synthesis reactions.

### A-2: synthetic route of R³=H

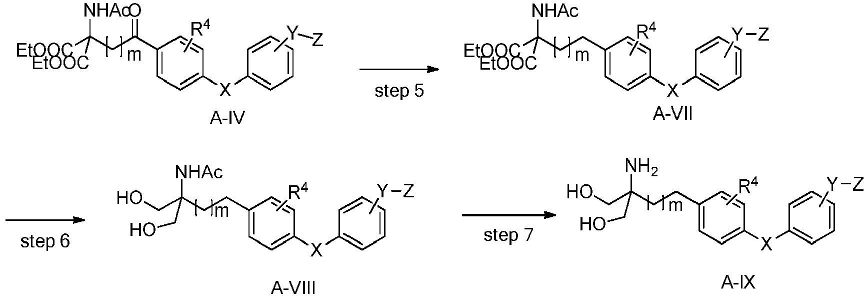

Step 5 is reduction reaction. Formula (A-VII) compounds can be prepared by the reduction of formula(A-VI) compounds with any suitable catalyst in any suitable reaction solvent (e.g., dichloromethane). Triethylsilane, for example, is required the reducing agent for the reactions. Lewis acid, for example titanium tetrachloride, is the required catalyst for the reaction.

Step 6 is reduction reaction. The reaction condition is the same as step 3. Formula (A-VIII)compounds can be prepared by the reactions of formula(A-VII) compounds with the reducing agents in any suitable reaction solvent (e.g., ethanol, water)..

Step 7 is hydrolysis reaction. The reaction condition is the same as step 4. Formula (A-IX) compounds can be prepared by the hydrolysis of formula (A-VII) compounds in any suitable reaction solvent (e.g., methanol, ethanol).

### A-3: synthetic route of esterification of hydroxyl.

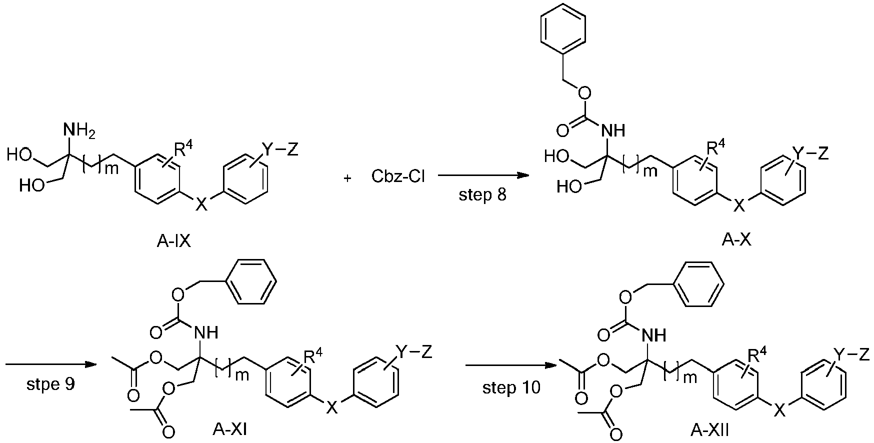

Step 8 is acylation reaction. Formula (A-X)compounds can be prepared by the reactions between formula(A-IX) compounds and Cbz-Cl with the base catalyst in any suitable reaction solvent (e.g., ethyl acetate, water). Required bases for the reactions are selected from any suitable bases, such as sodium bicarbonate, sodium hydroxide, potassium hydroxide and the like.

Step 9 is esterification reaction. Formula (A-XI)compounds can be prepared by the reactions between formula(A- X) compounds and acetic anhydride or acetyl chloride with the base catalyst in any suitable reaction solvent. Required bases for the reactions are selected from any suitable bases, such as s triethylamine, pyridine and the like.

Step 10 is reduction reaction. Formula (A-XII)compounds can be prepared by hydrogenation of formula(A-XI) compounds with suitable catalyst in any suitable reaction solvent (e.g., methanol, ethanol). Required catalyst for the reactions is selected from any suitable catalysts, for example palladium on activated carbon.

### A-4: Synthetic route of phosphorylation

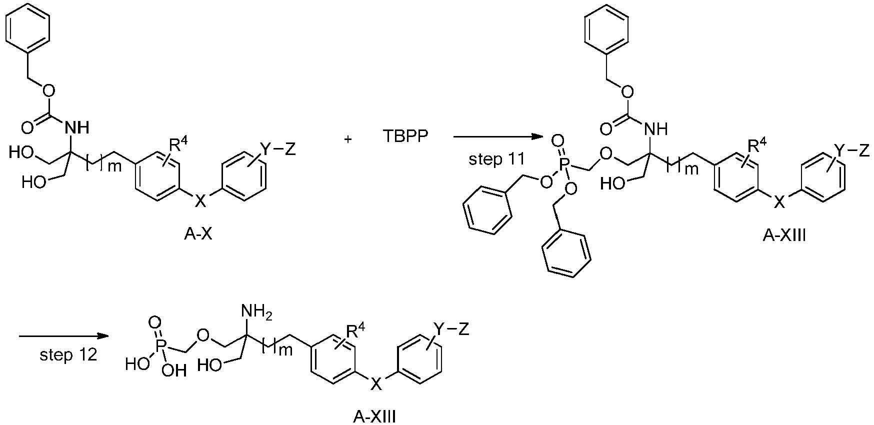

Step 11 is esterification reaction. Formula (A- XIII)compounds can be prepared by the reactions between formula(A- X) compounds and phosphorylating reagent with suitable catalyst in any suitable reaction solvent (dichloromethane). Required catalyst for the reactions is selected from any suitable catalysts, such as silver oxide and Hex4N.

Step 12 is reduction reaction. The reaction condition is the same as step 10. Formula (A-XIV) compounds can be prepared by hydrogenation of formula (A-XIII) compounds with suitable catalyst in any suitable reaction solvent (e.g., methanol, ethanol). Required catalyst for the reactions is selected from any suitable catalysts, for example palladium on activated carbon.

In the above reaction diagram, Z¹ and Z² are common leaving groups in organic synthesis, which can be the same or different. For example, halogen atoms (e.g. chlorine, bromine, iodine, etc.). Ac is acetyl, Et is ethyl. The other symbols are as previously defined.

The compounds of this invention can be prepared by the following method (method B as shown in the schematic), such as method B

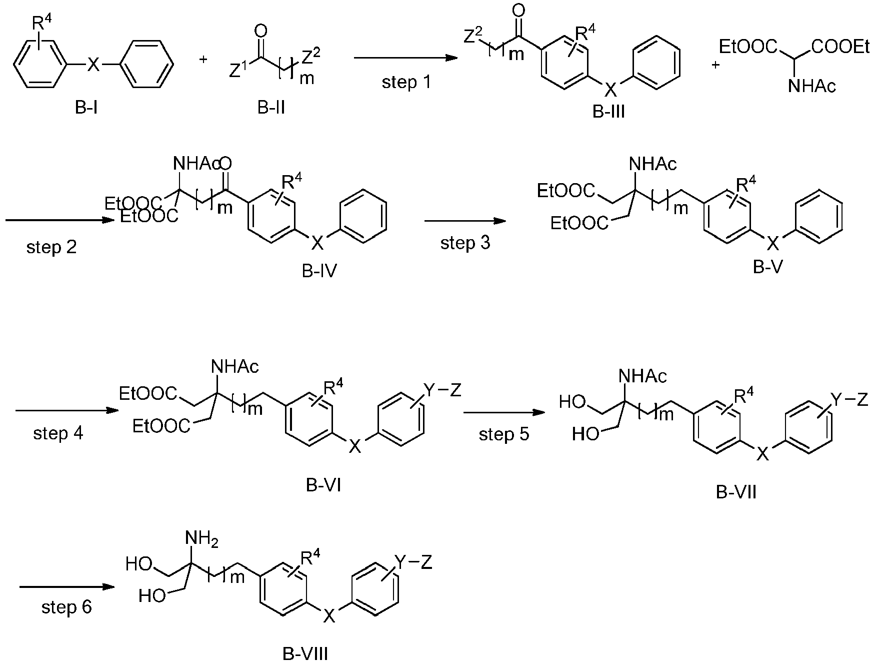

In the above reaction diagram, Z¹ and Z² are common leaving groups in organic synthesis, which can be the same or different. For example, halogen atoms (e.g. chlorine, bromine, iodine, etc.). Ac is acetyl, Et is ethyl. The other symbols are as previously defined.

Step 1 is Friedel-Crafts acylation reaction. Formula (B-III)compounds can be prepared by the reactions between formula(B- I) compounds and the formula (B- II) compounds with the Lewis acid catalyst in any suitable reaction solvent (e.g., methylene chloride, carbon disulfide). Required Lewis acids for the reactions are selected from any suitable acids, preferably aluminum chloride.

Step 2 is condensation reaction. Formula (B-IV)compounds can be prepared by the reactions between formula(B-III) compounds and diethyl acetamidomalonate in any suitable reaction solvent (e.g., ethanol, tetrahydrofuran). Required bases for the reactions select from any suitable bases, preferably sodium alkoxide, sodium hydroxide.

Step 3 is reduction reaction. Formula (B- V)compounds can be prepared by the reactions of formula(B-IV) compounds with the reducing agents in any suitable reaction solvent (e.g., ethanol, water). Preferred reducing agents are selected from the metal reducing agents; preferably metal reducing agents are selected from lithium aluminium hydride, sodium borohydride, lithium borohydride or diborane.

Steps 4 are both Friedel-Crafts reaction and condensation reaction. Formula (B-VI) compounds can be prepared by Friedel-Crafts reaction of formula (B- V) compounds followed by condensation reaction. Friedel-Crafts reaction can be conducted with the Lewis acid catalyst in any suitable reaction solvent (e.g., methylene chloride, carbon disulfide). Required Lewis acids for the reactions are selected from any suitable acids, preferably aluminum chloride. condensation reaction can be carried out with suitable acids or bases or catalysts in any suitable solvent.

Step 5 is reduction reaction. Formula (B-VII) compounds can be prepared by the reactions of formula (B-VI) compounds with the reducing agents in any suitable reaction solvent (e.g., ethanol, water). Preferred reducing agents are selected from the metal reducing agents; preferably metal reducing agents are selected from lithium aluminium hydride, sodium borohydride, lithium borohydride or diborane.

Step 6 is hydrolysis reaction. Formula (B-VIII)compounds can be prepared by the hydrolysis of formula (B-VII) compounds in any suitable reaction solvent (e.g., methanol, ethanol)). This reaction can be catalyzed by acids or bases, which are common in organic synthesis reactions.

The present invention relates to pharmaceutical compositions containing this invention compounds as the active ingredient. The pharmaceutical compositions can be prepared by methods known in this field. Combining compounds represented by the present invention with one or more pharmaceutically acceptable solid or liquid excipients and / or adjuvants is made into any formulation suitable for human or animal. The weight of the compounds of this invention in pharmaceutical composition accounts for 0.1 to 95%.

Compounds or their pharmaceutical compositions of the present invention can be administered in unit dosage form. The route of administration can be divided into intestinal and parenteral, such as oral, intravenous, intramuscular, subcutaneous, nasal, oral mucosa, eyes, lungs and respiratory tract, skin, vagina, rectum and so on.

Dosage form can be a liquid, a solid or semi-solid dosage forms. Liquid dosage forms can be solutions (including true solutions and colloid solutions), emulsions (including o / w type, w / o type and multiple emulsions), suspensions, injections (including aqueous injections, powder and infusion), eye drops , nasal drops, lotions and liniments etc.; solid dosage forms can be tablets (including conventional tablets, enteric-coated tablets, tablets, dispersible tablets, chewable tablets, effervescent tablets, orally disintegrating tablets), capsules (including hard capsules, soft capsules, enteric capsules), granules, powders, pellets, pills, suppositories, films, patches, gas (powder) aerosols, sprays, etc.; semi-solid dosage forms may be ointments, gels, pastes and the like.

The compounds of this invention can be made into the normal preparation, sustained release formulations, controlled release formulations, targeting formulations and various particulate delivery systems.

In order to making compounds of the invention into tablets, various known excipients can be widely used in this field. Such as diluents, binders, wetting, agents, disintegrants, lubricants and glidants. The diluent can be starch, dextrin , sucrose , glucose, lactose, mannitol , sorbitol , xylitol , microcrystalline cellulose, calcium sulfate , calcium hydrogen phosphate, calcium carbonate, etc. ; wetting agents can be water , ethanol , iso- propanol and the like ; binder can be starch , dextrin , syrup , honey , glucose solution , microcrystalline cellulose, acacia mucilage , clear glue, sodium carboxymethyl cellulose, methyl cellulose, hydroxypropylmethyl cellulose, ethyl cellulose , acrylic resins, carbomer , polyvinyl pyrrolidone, polyethylene glycol and the like ; disintegrating agents can be dry starch, microcrystalline cellulose, low -substituted hydroxypropyl cellulose, cross -linked poly vinyl pyrrolidone, crosslinked sodium carboxymethyl cellulose, sodium carboxymethyl starch , sodium bicarbonate and citric acid, polyoxyethylene sorbitan fatty acid esters, sodium lauryl sulfate and the like; lubricants and glidants agent can be talc, silicon dioxide , stearate, tartaric acid, liquid paraffin , polyethylene glycol and the like.

The tablets can be further made into tablets, such as sugar-coated tablets, film-coated tablets, enteric coated tablets, or double tablets and multilayer tablets.

In order to making dosing unit into capsules, the active ingredients of the present invention compounds combine with diluent and glidants, the mixture was directly put into a hard capsule or soft capsule. With diluent, binder, disintegrant, the active ingredients of the present invention compounds can be made into granules or pellets, then place into a hard gelatin capsules or soft capsules. The species of diluen, adhesives, wetting agents, disintegrants, glidants used for the preparation of tablets of the present invention compounds can also be applied for preparing capsules of the present invention compounds.

In order to making compounds of the invention into injection, water, ethanol, isopropanol, propylene glycol or their mixture can be used as solvent, the common and appropriate amount of solubilizer, co-solvents, pH regulating agents, osmotic pressure adjusting agent can be added in the field. Solubilizers or co-solvents can be poloxamer, lecithin, hydroxypropyl-β-cyclodextrin; pH regulating agent can be a phosphate, acetate, hydrochloric acid, sodium hydroxide and so on; osmotic pressure regulating agent can be sodium chloride, mannitol, glucose, phosphate, acetate and the like. Mannitol and glucose can be added as proppant when freeze-dried powders need to be prepared.

In addition, if desired, coloring agents, preservatives, perfumes, flavoring agents or other additives to pharmaceutical formulations can be added.

To achieve the purpose of treatment and enhance the therapeutic effect, the drug or pharmaceutical composition of this present invention can be administered by any known methods of administration.

Dose of the pharmaceutical composition of the present invention compounds can be varied in a wide range according to the property and severity of prevention or treatment of the diseases, individual situation of patients or animals and the administration and the formulation. In general, a suitable daily dosage range for the present invention compounds is 0.001-150mg/Kg weight, preferably 0.1-100mg/Kg weight, more preferably 1-60mg/Kg weight, and most preferably 2-30mg/Kg weight. The above dosage can be one unit or be divided into several administered dosage units, depending on the doctor's clinical experience and the use of other therapeutic regimen.

The compounds or their compositions of the invention can be administered alone, or in combination with other therapeutic drugs or symptomatic drugs. When the compounds of the present invention have synergistic effects with other therapeutic agents, their dosage should be adjusted according to the actual situation.

### EXAMPLES

### EXAMPLE 1

This example described the preparation of 2-amino-2-(4-(4-(2-ethyl-1H-imidazol-4-yl)phenoxy)phenethyl)propane-1,3-diol hydrochloride

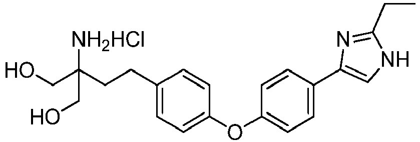

### (1-1) Preparation of 2-chloro-1-(4-phenoxyphenyl)ethanone

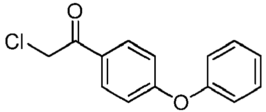

Chloroacetyl chloride (13.3 g, 117.5 mmol) was added dropwise to a cooled solution (0°C) of diphenyl ether (20 g, 117.5 mmol) in dry CH₂Cl₂ (200mL), then AlCl₃ (16.5 g, 123.4 mmol) was added in portions. The solution was allowed to return to room temperature and stirred for further 4 h, then poured slowly into HCl-ice mixture. The organic layer was separated and the aqueous phase was extracted with CH₂Cl₂ three times. The combined organic layers were washed with H₂O to neutral, dried over Na₂SO₄, filtered and concentrated, yielding the crude product (27g) as light yellow oil. The product was used in the next step without purification.

### (1-2) Preparation of diethyl 2-acetamido-2-(2-oxo-2-(4-phenoxyphenyl)ethyl) malonate

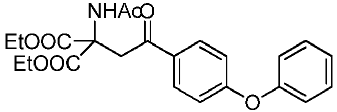

NaH (4.5g, 131mmol) was added to dry THF (300mL) at room temperature. After 30 min, diethyl acetamidomalonate (29.7g, 137mmol) was added in portions and the mixture was stirred for a further 5h. Then a solution of 2-chloro-1-(4-phenoxyphenyl)ethanone (27g, 109mmol) in THF was added. The mixture was heated to reflux for further 12h and concentrated. The residue was diluted with EtOAc, washed with H₂O to neutral, dried over Na₂SO₄, filtered and concentrated. The residue was purified by silica gel column chromatography to afford the title compound (30g) as colorless syrup.
¹HNMR(MERCURY 300MHz, CDCl₃) δ 7.93(d, J=8.7Hz, 2H, 2ArH)7.40(t, J =8.0Hz, 2H, 2ArH)7.21(t, J=7.5Hz, 1H, 1ArH)7.10(brs, 1H, NH)7.06(d, J =7.8Hz, 2H, 2ArH)6.98(d, J=9.0Hz, 2H, 2ArH)4.27(q, J=7.2Hz, 4H, 2CH₂)4.22(s, 2H, CH₂)1.97(s, 3H, CH₃)1.23(t, J=7.2Hz, 6H, 2CH₃) ESI(m/z)428 (M+H⁺)450(M+Na⁺)

### (1-3) Preparation of diethyl 2-acetamido-2-(4-phenoxyphenethyl)malonate

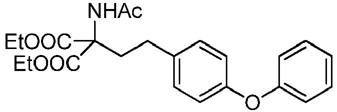

A solution of diethyl 2-acetamido-2-(2-oxo-2-(4-phenoxyphenyl)ethyl) malonate (10.2g, 23.9mmol) in CH₂Cl₂ (38mL) was added dropwise to a solution of Et₃SiH (10.5g, 90.7mmol) in CH₂Cl₂ (100mL) at room temperature under N₂ protection. TiCl₄ (17.2g, 90.7mmol) was added to the solution with a syringe and the reaction mixture was stirred overnight, then poured slowly into HCl-ice mixture. The organic layer was separated and the aqueous phase was extracted with CH₂Cl₂ three times. The combined organic layers were washed with H₂O to neutral, dried over Na₂SO₄, filtered and concentrated. The crude product was used in the next step without purification.

### (1-4) Preparation of diethyl 2-acetamido-2-(4-(4-(2-chloroacetyl)phenoxy)phenethyl) malonate

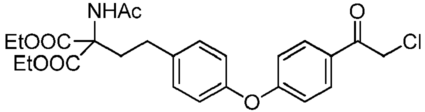

Chloroacetyl chloride (4.7 g, 41.2 mmol 1) in CH₂Cl₂(20mL) was added dropwise to a cooled solution (0°C) of diethyl 2-acetamido-2-(4-phenoxyphenethyl) malonate (15.5 g, 37.5 mmol) in dry CH₂Cl₂(200mL), then AlCl₃ (25 g, 188 mmol) was added in portions. The solution was allowed to return to room temperature and stirred for further 5 h, then poured slowly into HCl-ice mixture. The organic layer was separated and the aqueous phase was extracted with CH₂Cl₂ three times. The combined organic layers were washed with H₂O to neutral, dried over Na₂SO₄, filtered and concentrated. The residue was purified by silica gel column chromatography to afford the title compound (4.1g).
¹HNMR(MERCURY 300MHz, CDCl₃) δ 7.93(d, J=8.7Hz, 2H, 2ArH)7.19(d, J =8.7Hz, 2H, 2ArH)6.98(d, J=8.7Hz, 4H, 4ArH)6.81(brs, 1H, NH)4.65(s, 2H, 1CH₂)4.28-4.20(m, 4H, 2CH₂)2.70(dd, J=11.4Hz, 7.2Hz, 2H, 1CH₂)2.50(dd, J=9.3Hz, 5.1Hz, 2H, 1CH₂)2.04(s, 3H, 1CH₃)1.25(t, J=7.2Hz, 6H, 2CH₃) ESI(m/z)490(M+H⁺)512(M+Na⁺)

### (1-5)Preparation of diethyl 2-acetamido-2-(4-(4-(2-(propionyloxy)acetyl)phenoxy) phenethyl)malonate

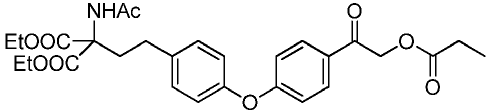

To a solution of diethyl 2-acetamido-2-(4-(4-(2-chloroacetyl)phenoxy) phenethyl)malonate (1.2g, 2.4mmol) in CH₃CN (12mL) was added propionic acid (0.41g, 5.5mmol) and Et₃N(0.51g, 5.1mmol). The mixture was heated to reflux for 2h, then concentrated. The residue was diluted with CH₂Cl₂, washed with H₂O to neutral, dried over Na₂SO₄, filtered and concentrated, yielding the crude product (1.1g) as yellow syrup.
¹HNMR(MERCURY 300MHz, CDCl₃) δ 7.88(d, J=8.7Hz, 2H, 2ArH)7.18(d, J =8.7Hz, 2H, 2ArH)6.98(d, J=8.7Hz, 4H, 4ArH)6.80(brs, 1H, NH)5.30(s, 2H, CH₂)4.27-4.20(m, 4H, 2CH₂)2.70(dd, J=10.8Hz, 6.9Hz, 2H, 1CH₂)2.56-2.46(m, 4H, 2CH₂)2.03(s, 3H, 1CH₃)1.29-1.19(m, 9H, 3CH₃)
ESI(m/z)528(M+H⁺)550(M+Na⁺)

### (1-6)Preparation of diethyl 2-acetamido-2-(4-(4-(2-ethyl-1H-imidazol-4-yl)phenoxy) phenethyl)malonate

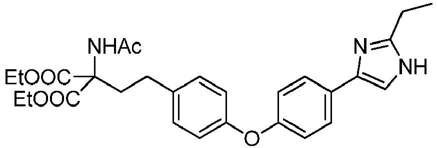

To a solution of diethyl 2-acetamido-2-(4-(4-(2-(propionyloxy)acetyl)phenoxy) phenethyl)malonate (1.0 g, 1.9 mmol) in xylene(20mL) was added acetamide (0.29 g, 3.8 mmol). The mixture was heated to reflux for three days, then concentrated. The residue was purified by silica gel column chromatography to afford the title compound (0.2g) as yellow oil.
¹HNMR(MERCURY 300MHz, CDCl₃) δ 7.63(d, J=8.4Hz, 2H, 2ArH)7.13(s, 1H, 1ArH)7.09(d, J=8.1Hz, 2H, 2ArH)6.96(d, J=8.4Hz, 2H, 2ArH)6.91(d, J=8.1Hz, 2H, 2ArH)6.80(brs, 1H, NH)4.23-4.18(m, 4H, 2CH₂)2.81(q, J=7.8Hz, 2H, CH₂)2.69(dd, J=9.0Hz, 6.6Hz, 2H, 1CH₂)2.46(dd, J=8.4Hz, 7.8Hz, 2H, 1CH₂)2.01(s, 3H, 1CH₃)1.32(t, J=7.5Hz, 3H, 1CH₃)1.25(t, J=7.2Hz, 6H, 2CH₃)ESI(m/z)508(M+H⁺)

### (1-7) Preparation of N-(4-(4-(4-(2-ethyl-1H-imidazol-4-yl)phenoxy)phenyl) -1-hydroxy-2- (hydroxymethyl)butan-2-yl)acetamide

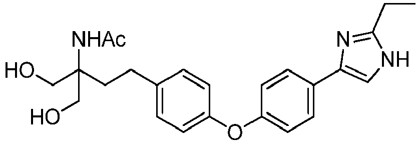

To a solution of diethyl 2-acetamido-2-(4-(4-(2-ethyl-1H-imidazol-4-yl) phenoxy)phenethyl)malonate (0.18g, 0.36mmol) in 95% EtOH(3mL) was added K₂HPO₄ (0.64g, 2.8mmol) in distilled water (0.64 mL) and NaBH₄ (0.07g, 1.8mmol) in aq. 10% NaOH(0.5mL). The mixture was stirred for further 6 h at room temperature and concentrated. The residue was extracted with EtOAc, washed with H₂O to neutral, dried over Na₂SO₄, filtered and concentrated. The crude product was used in the next step without purification.

### (1-8) Preparation of 2-amino-2-(4-(4-(2-ethyl-1H-imidazol-4-yl)phenoxy)phenethyl) propane-1,3-diol hydrochloride

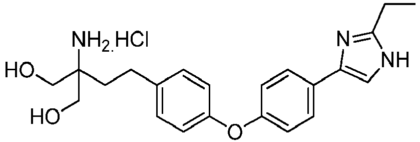

To a solution of *N*-(4-(4-(4-(2-ethyl-1H-imidazol-4-yl)phenoxy)phenyl)-1-hydroxy -2-(hydroxymethyl)butan-2-yl)acetamide in MeOH(10mL) was added NaOH(0.015, 0.36mmol) and heated to reflux for 6h, then filtered to remove insoluble substance. The solution was added HCl-EtOH solution until pH=3-4 and concentrated. The residue was purified by silica gel column chromatography to afford the title compound (0.1g) as light yellow solid.
¹HNMR(MERCURY 300MHz, CD₃OD) δ 7.63(d, J=8.4Hz, 2H, 2ArH)7.62(s, 1H, 1ArH)7.23(d, J=8.4Hz, 2H, 2ArH)6.99(d, J=8.4Hz, 2H, 2ArH)6.93(d, J=8.7Hz, 2H, 2ArH)3.64(s, 4H, 2CH₂)2.99(q, J=7.8Hz, 2H, 1CH₂)2.66-2.60(m, 2H, 1CH₂)1.94-1.88(m, 2H, 1CH₂)1.37(t, J=7.5Hz, 3H, 1CH₃) ¹³CNMR(400MHz, CD₃OD) δ 160.39, 155.90, 150.90, 138.61, 134.17, 130.98, 128.36, 122.72, 120.88, 119.61, 114.77, 62.49, 62.05, 34.71, 29.42, 20.40, 11.89
ESI(m/z)382(M+H⁺)HRMS calcd.for C₂₃H₂₈N₃O₃(M+H⁺)382.2125, found382.2120

### EXAMPLE 2

This example described the preparation of 2-amino-2-(4-(4-(5-methyl-1*H*-pyrrol-2-yl) phenoxy)phenethyl)propane-1,3-diol hydrochloride

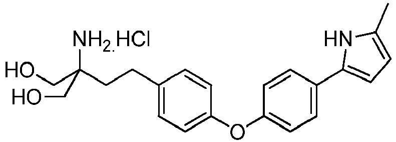

### (2-1) The preparation of diethyl 2-acetamido-2-(4-phenoxyphenethyl)malonate was the same as described above.

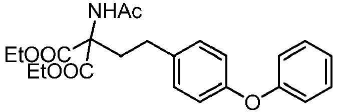

### (2-2) Preparation of diethyl 2-acetamido-2-(4-(4-(4-oxopentanoyl)phenoxy) phenethyl)malonate

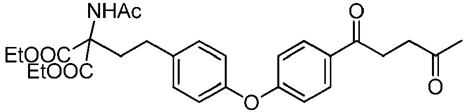

Levulinic acid chloride (1.9 g, 15.2 mmol) was added dropwise to a cooled solution (0°C) of diethyl 2-acetamido-2-(4-phenoxyphenethyl) malonate (6.0 g, 14.5 mmol) in dry CH₂Cl₂(200mL), then AlCl₃ (11.7 g, 87 mmol) was added in portions. The solution was allowed to return to room temperature and stirred for further 2 h, then poured slowly into HCl-ice mixture. The organic layer was separated and the aqueous phase was extracted with CH₂Cl₂ three times. The combined organic layers were washed with H₂O to neutral, dried over Na₂SO₄, filtered and concentrated, yielding the crude compound (7.1g) as yellow syrup. The crude product was used in the next step without purification.

### (2-3) Preparation of diethyl 2-acetamido-2-(4-(4-(5-methyl-1H-pyrrol-2-yl)phenoxy) phenethyl)malonate

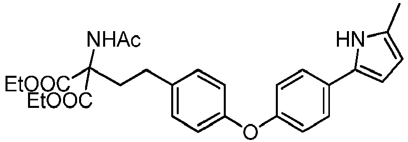

To a solution of diethyl 2-acetamido-2-(4-(4-(4-oxopentanoyl)phenoxy) phenethyl)malonate (3.6 g, 7.0 mmol) in EtOH/CHCl₃ (50mL, 2:3) was added ammonium acetate (10.8 g, 140 mmol). The mixture was heated to 50°C for 3h, then concentrated. The residue was purified by silica gel column chromatography to afford the title compound (2.1g) as light yellow syrup.
1HNMR (MERCURY 300 MHz, CDC13) δ 8.07 (brs, 1H, NH) 7.37 (d, J = 8.4 Hz, 2H, 2ArH) 7.10 ((d, J = 8.4 Hz, 2H, 2ArH) 6.93 (dd, J = 10.2, 8.7 Hz, 4H, 4ArH) 6.79 (brs, 1H, NH) 6.31 (brs, 1H, 1ArH) 5.93 (brs, 1H, 1ArH) 4.25-4.15 (m, 4H, 2CH2) 2.68 (dd, J = 10.5, 7.2 Hz, 2H, 1CH2) 2.45 (dd, J = 9.0, 7.2 Hz, 2H, 1CH2) 2.32 (s, 3H, CH3) 2.01 (s, 3H, CH3) 1.25 (t, J = 7.2 Hz, 6H, 2CH3)
ESI(m/z) 493 (M+H+)

### (2-4) Preparation of N-(1-hydroxy-2-(hydroxymethyl)-4-(4-(4-(5-methyl-1H-pyrrol -2-yl)phenoxy)phenyl)butan-2-yl)acetamide

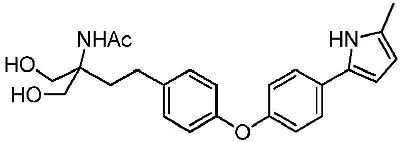

To a solution of diethyl 2-acetamido-2-(4-(4-(5-methyl-1H-pyrrol-2-yl)phenoxy) phenethyl)malonate (2.1 g, 4.2 mmol) in 95% EtOH(30 mL) was added K₂HPO₄ (7.6 g, 33.2 mmol) in distilled water (7.6 mL) and NaBH₄ (0.82 g, 21.6 mmol) in aq. 10% NaOH(5.5mL). The mixture was stirred for further 6 h at room temperature and concentrated. The residue was extracted with EtOAc, washed with H₂O to neutral, dried over Na₂SO₄, filtered and concentrated, yielding the crude product (1.6g) as light yellow sponge.
¹HNMR (MERCURY 300 MHz, CDCl₃) δ 8.14 (brs, 1H, NH) 7.37 (d, *J* = 8.4 Hz, 2H, 2ArH) 7.15 ((d, *J* = 8.4 Hz, 2H, 2ArH) 6.93 (dd, *J* = 10.2, 6.9 Hz, 4H, 4ArH) 6.31 (brs, 1H, 1ArH) 5.93 (brs, 1H, 1ArH) 3.85 (d, *J =* 11.7 Hz, 2H, 1CH₂) 3.62 (d, *J =* 11.1 Hz, 2H, 1CH₂) 2.62 (t, *J* = 8.4 Hz, 2H, 1CH₂) 2.32 (s, 3H, CH₃) 2.04-1.93 (m, 5H, 1CH₃, 1CH₂)
ESI(m/z) 409 (M+H⁺)

### (2-5) Preparation of 2-amino-2-(4-(4-(5-methyl-1H-pyrrol-2-yl)phenoxy)phenethyl) propane-1,3-diol hydrochloride

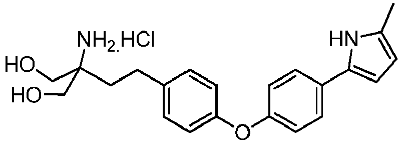

To a solution of *N*-(1-hydroxy-2-(hydroxymethyl)-4-(4-(4-(5-methyl-1H-pyrrol -2-yl)phenoxy)phenyl)butan-2-yl)acetamide (1.6 g, 4.2 mmol) in MeOH(10mL) was added NaOH(0.17 g, 4.3 mmol) and heated to reflux for 2h, then filtered to remove insoluble substance. The solution was added HCl-EtOH solution until pH=3-4 and concentrated. The residue was purified by silica gel column chromatography to afford the title compound (1.1g) as light yellow solid.
¹HNMR (MERCURY 300 MHz, CD₃OD) δ 7.42 (d, *J* = 8.4 Hz, 2H, 2ArH) 7.16 ((d, *J* = 8.4 Hz, 2H, 2ArH) 6.85 (d, *J* = 7.8 Hz, 2H, 2ArH) 6.84 (d, *J* = 8.7 Hz, 2H, 2ArH) 6.18 (d, *J =* 3.0 Hz, 1H, 1ArH) 5.73 (d, *J =* 2.7 Hz, 1H, 1ArH) 3.63 (brs, 4H, 2CH₂) 2.62-2.56 (m, 2H, 1CH₂) 2.20 (s, 3H, CH₃) 1.92-1.86 (m, 2H, 1CH₂)
¹³CNMR (400 MHz, CD₃OD) δ 157.50, 156.15, 137.15, 131.30, 130.81, 130.61, 130.08, 125.50, 120.15, 119.66, 108.03, 106.03, 62.53, 62.02, 34.80, 29.35, 12.92 ESI (m/z) 367 (M+H⁺) HRMS calcd. for C₂₂H₂₆N₂O₃ (M+H⁺) 367.2016, found 367.2026

### EXAMPLE 3

This example described the preparation of 2-amino-2-(4-(4-(5-methylfuran-2-yl) phenoxy)phenethyl)propane-1,3-diol hydrochloride

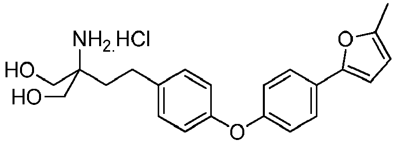

### (3-1) Preparation of diethyl 2-acetamido-2-(4-(4-(4-oxopentanoyl) phenoxy)phenethyl)malonate was the same as described above.

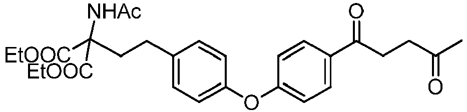

### (3-2) Preparation of diethyl 2-acetamido-2-(4-(4-(5-methylfuran-2-yl) phenoxy)phenethyl)malonate

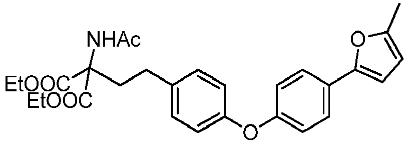

To a solution of diethyl diethyl 2-acetamido-2-(4-(4-(4-oxopentanoyl) phenoxy)phenethyl)malonate (3.5 g, 6.9 mmol) in toluene (30mL) was added *p*-toluenesulfonic acid (0.12 g, 0.7 mmol). The mixture was heated to 80°C for 5h, then concentrated. The residue was purified by silica gel column chromatography to afford the title compound (0.2g).
¹HNMR (MERCURY 300 MHz, CDCl₃) δ 7.57 (d, *J* = 9.0 Hz, 2H, 2ArH) 7.11 ((d, *J* = 8.1 Hz, 2H, 2ArH) 6.96 (dd, *J* = 11.1, 8.7 Hz, 4H, 4ArH) 6.79 (brs, 1H, NH) 6.44 (d, *J* = 3.0 Hz, 1H, 1ArH) 6.03 (brs, 1H, 1ArH) 4.29-4.18 (m, 4H, 2CH₂) 2.69 (dd, *J* = 10.8, 6.9 Hz, 2H, 1CH₂) 2.45 (dd, *J* = 15.3, 8.4 Hz, 2H, 1CH₂) 2.36 (s, 3H, CH₃) 2.02 (s, 3H, CH₃) 1.25 (t, *J* = 7.2 Hz, 6H, 2CH₃)
ESI(m/z) 494 (M+H⁺)

### (3-3) Preparation of N-(1-hydroxy-2-(hydroxymethyl)-4-(4-(4-(5-methylfuran-2-yl) phenoxy)phenyl)butan-2-yl)acetamide

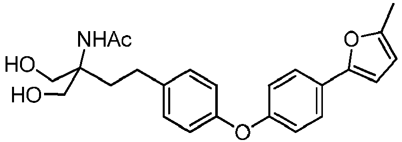

To a solution of diethyl 2-acetamido-2-(4-(4-(5-methyl-1H-pyrrol-2-yl)phenoxy) phenethyl)malonate (0.2 g, 0.4 mmol) in 95% EtOH(3 mL) was added K₂HPO₄ (0.73 g, 3.2 mmol) in distilled water (0.73 mL) and NaBH₄ (0.08 g, 2.1 mmol) in aq. 10% NaOH(0.53 mL). The mixture was stirred for further 6 h at room temperature and concentrated. The residue was extracted with EtOAc, washed with H₂O to neutral, dried over Na₂SO₄, filtered and concentrated, yielding the crude product (0.16g) as light yellow sponge.
¹HNMR (MERCURY 300 MHz, CDCl₃) δ 7.57 (d, *J* = 9.0 Hz, 2H, 2ArH) 7.16 ((d, *J* = 7.8 Hz, 2H, 2ArH) 6.95 (dd, *J* = 12.0, 7.2 Hz, 4H, 4ArH) 6.44 (d, *J* = 3.3 Hz, 1H, 1ArH) 6.033 (d, *J* = 2.1 Hz, 1H, 1ArH) 3.87 (d, *J* = 11.4 Hz, 2H, 1CH₂) 3.64 (d, *J* = 11.7 Hz, 2H, 1CH₂) 2.63 (t, *J* = 8.4 Hz, 2H, 1CH₂) 2.36 (s, 3H, CH₃) 2.04-1.94 (m, 5H, 1CH₃, 1CH₂)

### (3-4) Preparation of 2-amino-2-(4-(4-(5-methylfuran-2-yl) phenoxy)phenethyl) propane-1,3-diol hydrochloride

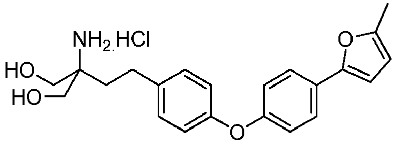

To a solution of *N*-(1-hydroxy-2-(hydroxymethyl)-4-(4-(4-(5-methylfuran-2-yl) phenoxy)phenyl)butan-2-yl)acetamide (0.16 g, 0.4 mmol) in MeOH(10mL) was added NaOH(0.02 g, 0.4 mmol) and heated to reflux for 6h, then filtered to remove insoluble substance. The solution was added HCl-EtOH solution until pH=3-4 and concentrated. The residue was purified by silica gel column chromatography to afford the title compound (0.13 g) as light yellow solid.
¹HNMR (MERCURY 300 MHz, CD₃OD) δ 7.53 (d, *J* = 8.7 Hz, 2H, 2ArH) 7.18 ((d, *J* = 8.4 Hz, 2H, 2ArH) 6.89 (d, *J* = 9.0 Hz, 4H, 4ArH) 6.46 (d, *J* = 3.3 Hz, 1H, 1ArH) 6.01 (d, *J* = 2.1 Hz, 1H, 1ArH) 3.64 (brs, 4H, 2CH₂) 2.64-2.58 (m, 2H, 1CH₂) 2.27 (s, 3H, CH₃) 1.93-1.87 (m, 2H, 1CH₂)

### EXAMPLE 4

This example described the preparation of 3-amino-3-(hydroxymethyl) -1-(4-(4-(5-methylthiazol-2-yl)phenoxy)phenyl)butane-1,4-diol hydrochloride

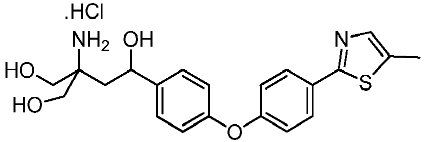

### (4-1) Preparation of 5-methyl-2-(4-phenoxyphenyl)thiazole

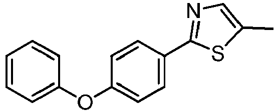

To a solution of 4-phenoxyphenylboronic acid (1.05 g, 4.9 mmol) in toluene/EtOH (6 mL/2 mL) was added 2-bromo-5-methylthiazole(0.87 g, 4.9 mmol) and aq. 2M Na₂CO₃ (6 mL). The mixture was heated to reflux for 6h, then concentrated. The residue was purified by silica gel column chromatography to afford the title compound (1.06 g) as white solid.
¹HNMR (MERCURY 300 MHz, CDCl₃) δ 7.86 (d, *J* = 8.4 Hz, 2H, 2ArH) 7.47 (s, 1H, 1ArH) 7.37 (t, *J* = 7.8 Hz, 2H, 2ArH) 7.15 (t, *J* = 7.2 Hz, 1H, 1ArH) 7.04 (t, *J* = 8.7 Hz, 4H, 4ArH) 2.50 (s, 3H, 1CH₃)
ESI(m/z) 268 (M+H⁺)

### (4-2) Preparation of 2-bromo-1-(4-(4-(5-methylthiazol-2-yl)phenoxy)phenyl)ethanone

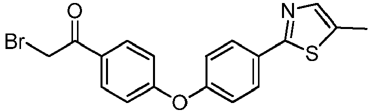

Bromoacetyl bromide (0.84 g, 4.17 mmol) was added dropwise to a cooled solution (0°C) of 5-methyl-2-(4-phenoxyphenyl)thiazole (1.06 g, 3.97 mmol) in dry CH₂Cl₂ (100mL), then AlCl₃ (2.7 g, 5.1 mmol) was added in portions. The solution was allowed to return to room temperature and stirred for further 3 h, then poured slowly into HCl-ice mixture. The organic layer was separated and the aqueous phase was extracted with CH₂Cl₂ three times. The combined organic layers were washed with H₂O to neutral, dried over Na₂SO₄, filtered and concentrated, yielding the crude product as light yellow solid. The crude product was used in the next step without purification.
¹HNMR (MERCURY 300 MHz, CDCl₃) δ 8.00 (d, *J* = 8.7 Hz, 2H, 2ArH) 7.94 (d, *J* = 8.7 Hz, 2H, 2ArH) 7.50 (s, 1H, 1ArH) 7.12 (d, *J* = 8.4 Hz, 2H, 2ArH) 7.07 (d, *J* = 8.7 Hz, 2H, 2ArH) 4.66 (s, 2H, 1CH₂) 2.52 (s, 3H, 1CH₃)
ESI(m/z) 387, 389 (M+H⁺)

### (4-3) Preparation of diethyl 2-acetamido-2-(2-(4-(4-(5-methylthiazol-2-yl) phenoxy)phenyl)-2-oxoethyl)malonate

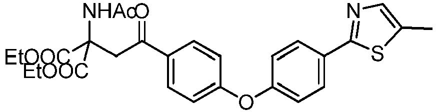

Sodium (0.11 g, 4.8 mmol) was added to absolute EtOH (250 mL). After sodium dissolved completely, diethyl acetamidomalonate (1.13 g, 5.2 mmol) was added in portions at 0 °C. The solution was then allowed to return to room temperature and stirred for further 30min. Then a solution of 2-bromo-1(4-(4-(5-methylthiazol-2-yl)phenoxy)phenyl)ethanone (1.5 g, 4.0 mmol) in THF (10 mL) was added. The mixture was stirred at room temperature for 2 h, then concentrated. The residue was diluted with EtOAc, washed with brine, dried over Na₂SO₄, filtered and concentrated, yielding the crude product as yellow oil. The crude product was used in the next step without purification.
¹HNMR (MERCURY 300 MHz, CDCl₃) δ 7.97 (d, *J* = 8.7 Hz, 2H, 2ArH) 7.92 (d, *J* = 8.7 Hz, 2H, 2ArH) 7.50 (s, 1H, 1ArH) 7.11 (brs, 1H, 1NH) 7.10 (d, *J* = 9.3 Hz, 2H, 2ArH) 7.05 (d, *J* = 8.4 Hz, 2H, 2ArH) 4.30-4.23 (m, 6H, 3CH₂) 2.52 (s, 3H, 1CH₃) 1.97 (s, 3H, 1CH₃) 1.22 (t, *J* = 7.2 Hz, 3H, 1CH₃)
ESI(m/z) 525 (M+H⁺)

### (4-4) Preparation of N-(1,4-dihydroxy-2-(hydroxymethyl)-4-(4-(4-(5-methylthiazol-2-yl)phenoxy)phenyl)butan-2-yl)acetamide

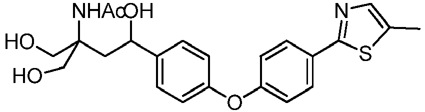

To a solution of diethyl 2-acetamido-2-(2-(4-(4-(5-methylthiazol-2-yl) phenoxy)phenyl)-2-oxoethyl)malonate (2.0 g, 4.0 mmol) in 95% EtOH(28 mL) was added K₂HPO₄ (7.2 g, 32mol) in distilled water (7.2 mL) and NaBH₄ (0.78 g, 21 mmol) in aq. 10% NaOH(5.3 mL). The mixture was stirred for further 6 h at room temperature and concentrated. The residue was extracted with EtOAc, washed with H₂O to neutral, dried over Na₂SO₄, filtered and concentrated, yielding the crude product (0.34g) as light yellow sponge.
¹HNMR (MERCURY 300 MHz, CDCl₃) δ 7.85 (d, *J* = 8.1 Hz, 2H, 2ArH) 7.45 (s, 1H, 1ArH) 7.35 (d, *J* = 8.1 Hz, 2H, 2ArH) 7.02 (d, *J* = 7.5 Hz, 2H, 2ArH) 7.00 (d, *J* = 8.4 Hz, 2H, 2ArH) 4.91 (d, *J* = 10.5 Hz, 1H, 1CH) 3.78 (t, *J* = 12.6 Hz, 2H, 1CH₂) 3.62 (d, *J =* 11.7 Hz, 1H, 1CH₂) 3.50 (d, *J* = 11.7 Hz, 1H, 1CH₂) 2.50 (s, 3H, 1CH₃) 2.39 (d, *J* = 15.3 Hz, 1H, 1CH₂) 2.06 (s, 3H, 1CH₃) 1.84 (dd, *J* = 14.7 Hz, 10.5 Hz, 1H, 1CH₂) ESI(m/z) 443 (M+H⁺)

### (4-5) Preparation of 3-amino-3-(hydroxymethyl) -1-(4-(4-(5-methylthiazol-2-yl) phenoxy)phenyl)butane-1,4-diol hydrochloride

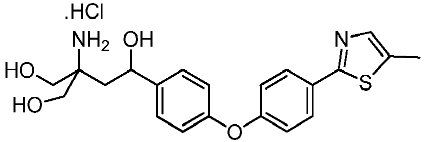

To a solution of N-(1,4-dihydroxy-2-(hydroxymethyl)-4-(4-(4-(5-methylthiazol-2-yl)phenoxy)phenyl)butan-2-yl)acetamide (0.34 g, 0.77 mmol) in MeOH(10mL) was added NaOH(0.03 g, 0.8 mmol) and heated to reflux for 6h, then filtered to remove insoluble substance. The solution was added HCl-EtOH solution until pH=3-4 and concentrated. The residue was purified by silica gel column chromatography to afford the title compound (0.23 g) as light yellow solid.
¹HNMR (MERCURY 300 MHz, CD₃OD) δ 7.87 (d, *J* = 8.4 Hz, 2H, 2ArH) 7.85 (s, 1H, 1ArH) 7.41 (d, *J* = 8.4 Hz, 2H, 2ArH) 7.02 (t, *J* = 8.7 Hz, 4H, 4ArH) 4.99 (dd, *J* = 10.5 Hz, 2.7 Hz, 1H, 1CH) 3.81 (d, *J* = 11.4 Hz, 1H, 1CH₂) 3.76 (d, *J* = 11.4 Hz, 1H, 1CH₂) 3.64 (d, *J* = 11.4 Hz, 1H, 1CH₂) 3.56 (d, *J* = 11.4 Hz, 1H, 1CH₂) 2.52 (s, 3H, 1CH₃) 1.96-1.79 (m, 2H, 1CH₂)

### EXAMPLE 5

This example described the preparation of 3-amino-3-(hydroxymethyl)-1 -(4-(4-(pyrimidin-2-yl)phenoxy)phenyl)butane-1,4-diol

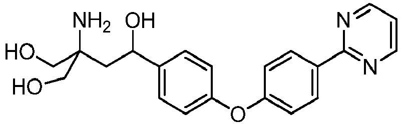

### (5-1) Preparation of 2-(4-phenoxyphenyl)pyrimidine

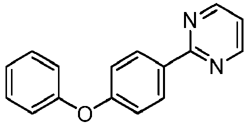

To a solution of 2-bromopyrimidine (159 mg ,1.0 mmol) in 1,2-dimethoxyethane (10mL) was added 4-phenoxyphenylboronic acid(257 mg ,1.2 mmol),water(2 mL), K₂CO₃(27 mg , 0.2 mmol, Pd(PPh₃)₄(23 mg , 0.02 mmol). The mixture was heated to reflux under argon for 18h, then cooled to room temperature. The solution was diluted with EtOAc, washed with H₂O and brine, dried over Na₂SO₄, filtered and concentrated. The residue was purified by silica gel column chromatography (PE/EA=10:1) to afford the title compound (150 mg) as transparent light yellow oil.
¹HNMR (MERCURY 300 MHz, CDCl₃) δ 8.78 (d, *J* = 4.8 Hz, 2H, 2ArH) 8.44 (d, *J* = 8.4 Hz, 2H, 2ArH) 7.38 (t, *J* = 7.5 Hz, 2H, 2ArH) 7.18-7.14 (m, 3H, 3ArH) 7.09 (d, *J* = 8.7 Hz, 2H, 2ArH)
ESI(m/z) 249 (M+H⁺)

### (5-2) Preparation of 2-bromo-1-(4-(4-(pyrimidin-2-yl)phenoxy)phenyl)ethanone

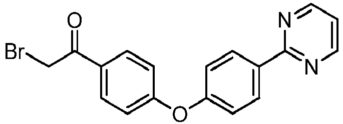

Bromoacetyl bromide (651 mg, 3.226 mmol) was added dropwise to a cooled solution (0°C) of 2-(4-phenoxyphenyl)pyrimidine (800 mg , 3.226 mmol) in dry CH₂Cl₂ (30 mL), then AlCl₃ was added in portions. The solution was allowed to return to room temperature and stirred for further 3 h, then poured slowly into HCl-ice mixture. The organic layer was separated and the aqueous phase was extracted with CH₂Cl₂ three times. The combined organic layers were washed with H₂O to neutral, dried over Na₂SO₄, filtered and concentrated, yielding the crude product (1 g) as light yellow solid.
¹HNMR (MERCURY 300 MHz, CDCl₃) δ 8.82 (d, *J* = 4.8 Hz, 2H, 2ArH) 8.51 (d, *J* = 9.0 Hz, 2H, 2ArH) 8.01 (d, *J* = 8.7 Hz, 2H, 2ArH) 7.23-7.17 (m, 3H, 3ArH) 7.09 (d, *J* = 9.3 Hz, 2H, 2ArH) 4.41 (s, 2H, CH₂)
ESI(m/z) 369, 371 (M+H⁺)

### (5-3) Preparation of diethyl 2-acetamido-2-(2-oxo-2-(4-(4-(pyrimidin-2-yl) phenoxy)phenyl)ethyl)malonate

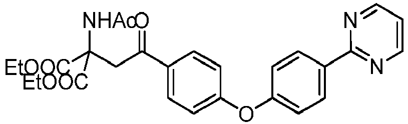

NaH was added to dry THF (300mL) at room temperature. After 30 min, diethyl acetamidomalonate (588 mg , 2.71 mmol) was added in portions. Then a solution of 2-bromo-1-(4-(4-(pyrimidin-2-yl)phenoxy)phenyl)ethanone (1 g , 2.71 mmol) in THF was added. The mixture was stirred at room temperature for 4h and then heated to reflux for further 5h and concentrated. The residue was diluted with EtOAc (20mL), washed with 1NHCl, aq. saturated NaHCO₃, brine, dried over Na₂SO₄, filtered and concentrated. The residue was purified by silica gel column chromatography(PE/EA=5:1) to afford the title compound (1.01g) as light yellow oil. ¹HNMR (MERCURY 300 MHz, CDCl₃) δ 8.81 (d, *J* = 4.5 Hz, 2H, 2ArH) 8.49 (d, *J* = 9.0 Hz, 2H, 2ArH) 7.97 (d, *J* = 8.7 Hz, 2H, 2ArH) 7.21-7.12 (m, 3H, 3ArH) 7.07 (d, *J* = 8.7 Hz, 2H, 2ArH) 4.30-4.23 (m, 6H, 3CH₂) 1.98 (s, 3H, 1CH₃) 1.24 (t, *J* = 6.9 Hz, 3H, 1CH₃)
ESI(m/z) 506 (M+H⁺)

### (5-4) Preparation of N-(1,4-dihydroxy-2-(hydroxymethyl)-4-(4-(4-(pyrimidin-2-yl) phenoxy)phenyl)butan-2-yl)acetamide

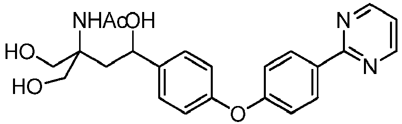

To a solution of diethyl 2-acetamido-2-(2-oxo-2-(4-(4-(pyrimidin-2-yl) phenoxy)phenyl)ethyl)malonate (1.01 g , 2 mmol) in 95% EtOH was added K₂HPO₄ (3.648 g) in distilled water (4 mL) and NaBH₄ (418 mg, 11 mmol) in aq. 10% NaOH(0.25 mL in 2.5 mL H₂O). The mixture was stirred for further 4 h at room temperature and concentrated. The residue was extracted with EtOAc, washed with H₂O to neutral, dried over Na₂SO₄, filtered and concentrated. The residue was purified by silica gel column chromatography(CH₂Cl₂/MeOH=25:1) to afford the title compound (400 mg) as colorless oil.
¹HNMR (MERCURY 300 MHz, CDCl₃) δ 8.69 (d, *J* = 4.8 Hz, 2H, 2ArH) 8.35 (d, *J* = 8.7 Hz, 2H, 2ArH) 7.30 (d, *J* = 8.4 Hz, 2H, 2ArH) 7.12 (t, *J* = 4.8 Hz, 1H, 1ArH) 7.01 (d, *J* = 9.3 Hz, 4H, 4ArH) 4.85 (d, *J* = 10.2 Hz, 1H, 1CH) 3.80-3.47 (m, 4H, 2CH₂) 2.24 (d, *J* = 14.7 Hz, 1H, 1CH₂) 1.97 (s, 3H, 1CH₃) 1.80 (dd, *J* = 15.0 Hz, 10.8 Hz, 1H, 1CH₂)
ESI(m/z) 406 (M-OH⁺)

### (5-5) Preparation of 3-amino-3-(hydroxymethyl)-1-(4-(4-(pyrimidin-2-yl) phenoxy)phenyl)butane-1,4-diol

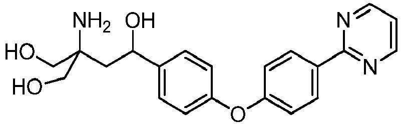

To a solution of *N*-(1,4-dihydroxy-2-(hydroxymethyl)-4-(4-(4-(pyrimidin-2-yl) phenoxy)phenyl)butan-2-yl)acetamide (287 mg , 0.678 mmol) in MeOH(10mL) was added NaOH(34 mg , 0.838 mmol) and heated to reflux for 6h, then filtered to remove insoluble substance. The solution was added HCl-EtOH solution until pH=3-4 and concentrated. The residue was recrystallized with isopropanol/PE to afford the title compound (155 mg) as white solid.
¹HNMR (MERCURY 300 MHz, CD₃OD) δ 8.75 (d, *J* = 4.8 Hz, 2H, 2ArH) 8.32 (d, *J* = 9.0 Hz, 2H, 2ArH) 7.39 (d, *J* = 8.4 Hz, 2H, 2ArH) 7.26 (t, *J* = 4.5 Hz, 1H, 1ArH) 6.99 (dd, *J* = 8.4 Hz, 2.1 Hz, 4H, 4ArH) 4.95 (dd, *J* = 10.2 Hz, 3.0Hz, 1H, 1CH) 3.59-3.47 (m, 4H, 2CH₂) 1.91-1.66 (m, 2H, 1CH₂)

### EXAMPLE 6

This example described the preparation of

| | |
|---|---|
| 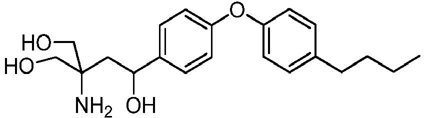 | 3-amino-1-(4-(4-butylphenoxy)phenyl)-3-(hydroxymethyl)butane-1,4-diol |
| 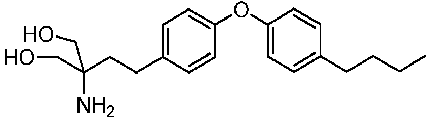 | 2-amino-2-(4-(4-butylphenoxy)phenethyl) propane-1,3-diol |

### (6-1) Preparation of 1-(4-phenoxyphenyl)butan-1-one

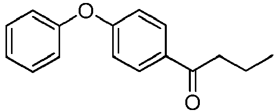

A solution of *n*-butyric acid (10 g, 113.5 mmol) in PCl₃(6.22 g, 45.4 mmol) was heated to 50-60°C for 3h. The supernatant was poured and the residue was washed with CH₂Cl₂. The combined organic layer was placed in three-necked bottle. To the solution diphenyl ether (19.3 g, 113.5 mmol)was added at 0°C, then AlCl₃ (15.2 g, 113.5 mmol) was added in portions. The solution was allowed to return to room temperature and stirred for further 3 h, then poured slowly into HCl-ice mixture. The organic layer was separated and the aqueous phase was extracted with CH₂Cl₂ three times. The combined organic layers were washed with H₂O to neutral, dried over Na₂SO₄, filtered and concentrated, yielding the crude product (24 g) as white solid.
¹HNMR (MERCURY 300 MHz, CDCl₃) δ 7.95 (d, *J* = 9.3 Hz, 2H, 2ArH) 7.39 (t, *J* = 7.5 Hz, 2H, 2ArH) 7.19 (t, *J* = 7.5 Hz, 1H, 1ArH) 7.07 (d, *J* = 7.2 Hz, 2H, 2ArH) 7.00 (d, *J* = 8.7 Hz, 2H, 2ArH) 2.90 (t, *J* = 7.2 Hz, 2H, 1CH₂) 1.83-1.70 (m, 2H, 1CH₂) 1.00 (t, *J* = 7.5 Hz, 3H, 1CH₃)
ESI(m/z) 241 (M+H⁺) 263 (M+Na⁺)

### (6-2) Preparation of 1-butyl-4-phenoxybenzene

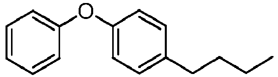

To a solution of 1-(4-phenoxyphenyl)butan-1-one(12.5 g, 52.1 mmol) in dry THF (150 mL) was added AlCl₃(19.5 g, 145.8 mmol) and NaBH₄(10.1 g, 265.6 mmol) at 0°C. The mixture was heated to reflux for 3 h, then was decomposed by ice water. The organic layer was separated and the aqueous phase was extracted with EA three times. The combined organic layers were washed with H₂O to neutral, dried over Na₂SO₄, filtered and concentrated, yielding crude product (11g) as colorless oil.
¹HNMR (MERCURY 300 MHz, CDCl₃) δ 7.53 (t, *J* = 7.8 Hz, 2H, 2ArH) 7.13 (d, *J* = 8.1 Hz, 2H, 2ArH) 7.07 (d, *J* = 7.2 Hz, 1H, 1ArH) 6.98 (d, *J* = 8.1 Hz, 2H, 2ArH) 6.92 (d, *J* = 8.7 Hz, 2H, 2ArH) 2.59 (t, *J* = 7.8 Hz, 2H, 1CH₂) 1.64-1.54 (m, 2H, 1CH₂) 1.42-1.29 (m, 2H, 1CH₂) 0.93 (t, *J* = 7.2 Hz, 3H, 1CH₃)
ESI(m/z) 227 (M+H⁺)

### (6-3) Preparation of 1-(4-(4-butylphenoxy)phenyl)-2-chloroethanone

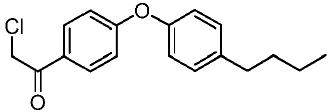

Chloroacetyl chloride (5.8 g, 51.5 mmol) was added dropwise to a cooled solution (0°C) of 1-butyl-4-phenoxybenzene (11.1 g, 49 mmol) in dry CH₂Cl₂ (200mL), then AlCl₃ (7.2 g, 54 mmol) was added in portions. The solution was allowed to return to room temperature and stirred for further 3 h, then poured slowly into HCl-ice mixture. The organic layer was separated and the aqueous phase was extracted with CH₂Cl₂ three times. The combined organic layers were washed with H₂O to neutral, dried over Na₂SO₄, filtered and concentrated. The crude product was used in the next step without purification.
¹HNMR (MERCURY 300 MHz, CDCl₃) δ 7.93 (d, *J* = 8.7 Hz, 2H, 2ArH) 7.21 (d, *J* = 8.1 Hz, 2H, 2ArH) 6.99 (dd, *J* = 8.7 Hz, 2.7 Hz, 4H, 4ArH) 4.65 (s, 2H, CH₂) 2.63 (t, *J* = 7.8 Hz, 2H, 1CH₂) 1.67-1.57 (m, 2H, 1CH₂) 1.42-1.34 (m, 2H, 1CH₂) 0.95 (t, *J* = 7.2 Hz, 3H, 1CH₃)
ESI(m/z) 303 (M+H⁺) 325 (M+Na⁺)

### (6-4) Preparation of diethyl 2-acetamido-2-(2-(4-(4-butylphenoxy) phenyl)-2-oxoethyl)malonate

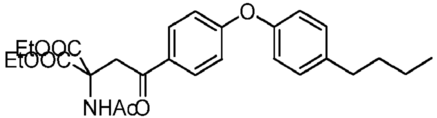

NaH (1.9 g, 56.4 mmol) was added to dry THF (200mL) at room temperature. After 30 min, diethyl acetamidomalonate (12.8 g, 58.8 mmol) was added in portions and stirred for further 5h. Then a solution of 1-(4-(4-butylphenoxy)phenyl)-2-chloroethanone (14.2 g, 47 mmol) in THF was added. The mixture was heated to reflux for further 12h and concentrated. The residue was diluted with EtOAc (20mL), washed with 1NHCl, aq. saturated NaHCO₃, brine, dried over Na₂SO₄, filtered and concentrated. The residue was purified by silica gel column chromatography to afford the title compound (6.9g).
¹HNMR (MERCURY 300 MHz, CDCl₃) δ 7.92 (d, *J* = 8.4 Hz, 2H, 2ArH) 7.20 (d, *J* = 8.1 Hz, 2H, 2ArH) 7.11 (brs, 1H, NH) 6.97 (d, *J* = 8.7 Hz, 4H, 4ArH) 4.27 (dd, *J =* 14.1 Hz, 7.2Hz, 4H, 2CH₂) 4.21 (s, 2H, CH₂) 2.65 (t, *J* = 7.8 Hz, 2H, 1CH₂) 1.97 (s, 3H, CH₃) 1.64-1.57 (m, 2H, 1CH₂) 1.41-1.29 (m, 2H, 1CH₂) 1.24 (t, *J* = 7.2 Hz, 6H, 2CH₃) 0.95 (t, *J* = 7.2 Hz, 3H, 1CH₃)
ESI(m/z) 484 (M+H⁺) 506 (M+Na⁺)

### (6-5) Preparation of N-(4-(4-(4-butylphenoxy)phenyl)-1,4-dihydroxy-2-(hydroxymethyl)butan-2-yl)acetamide

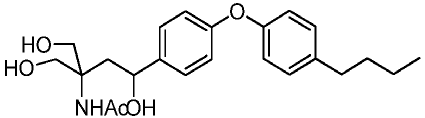

To a solution of diethyl diethyl 2-acetamido-2-(2-(4-(4-butylphenoxy) phenyl)-2-oxoethyl)malonate (1.15 g, 2.4 mmol) in 95% EtOH (17mL) was added K₂HPO₄ (4.3 g, 18.8 mmol) in distilled water (4.3 mL) and NaBH₄ (0.46 g, 12.2 mmol) in aq. 10% NaOH(3.1mL). The mixture was stirred for further 6h at room temperature and concentrated. The residue was extracted with EtOAc, washed with H₂O to neutral, dried over Na₂SO₄, filtered and concentrated. The residue was recrystallized with EtOAc to afford the title compound (0.65 g) as white powder solid.
¹HNMR (MERCURY 300 MHz, CDCl₃) δ 7.28 (d, *J* = 8.4 Hz, 2H, 2ArH) 7.14 (d, *J* = 8.4 Hz, 2H, 2ArH) 6.98-6.88 (m, 5H, 4ArH, NH) 4.89-4.78 (m, 1H, CH) 3.79-3.45 (m, 4H, 2CH₂) 2.59 (t, *J* = 7.5 Hz, 2H, 1CH₂) 2.35 (d, *J* = 15.0 Hz, 1H, CH₂) 2.04 (s, 3H, CH₃) 1.82 (dd, *J* = 15.0 Hz, 10.8 Hz, 1H, CH₂) 1.64-1.54 (m, 2H, CH₂) 1.42-1.29 (m, 2H, CH₂) 0.93 (t, *J =* 7.2 Hz, 3H, CH₃)
ESI(m/z) 384 (M-OH) 424 (M+Na⁺)

### (6-6) Preparation of 3-amino-1-(4-(4-butylphenoxy)phenyl)-3-(hydroxymethyl) butane-1,4-diol

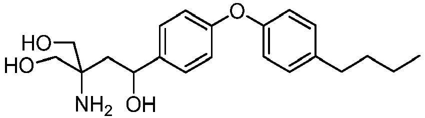

To a solution of *N*-(4-(4-(4-butylphenoxy)phenyl)-1,4-dihydroxy-2-(hydroxymethyl)butan-2-yl)acetamide (0.55 g, 1.4 mmol) in MeOH(10mL) was added NaOH(0.057 g, 1.4 mmol) and heated to reflux for 2 h, then filtered to remove insoluble substance. The solution was added HCl-EtOH solution until pH=3-4 and concentrated. The residue was recrystallized with isopropanol to afford the title compound (0.47 g) as white solid.
¹HNMR (MERCURY 300 MHz, CD₃OD) δ 7.25 (d, *J* = 8.4 Hz, 2H, 2ArH) 7.04 (d, *J* = 8.1 Hz, 2H, 2ArH) 6.78 (dd, *J* = 12.6 Hz, 8.7 Hz, 4H, 4ArH) 4.85 (dd, *J* = 10.2 Hz, 2.7 Hz, 1H, CH) 3.49 (dd, *J* = 15.3 Hz, 12.3 Hz, 2H, CH₂) 3.40 (s, 2H, CH₂) 2.49 (t, *J* = 7.2 Hz, 2H, 1CH₂) 1.72-1.44 (m, 4H, 2CH₂) 1.32-1.20 (m, 2H, CH₂) 0.85 (t, *J* = 7.2 Hz, 3H, CH₃)
¹³C NMR (400 MHz, CD₃OD) δ 158.20, 156.60, 141.97, 139.11, 130.65, 128.22, 119.80, 119.26, 71.27, 67.86, 66.50, 57.23, 44.62, 35.86, 35.07, 23.30, 14.27 ESI(m/z) 360 (M+H⁺) HRMS calcd. for C₂₁H₃₀NO₄(M+H⁺) 360.2174, found 360.2169

### (6-7) Preparation of diethyl 2-acetamido-2-(4-(4-butylphenoxy)phenethyl)malonate

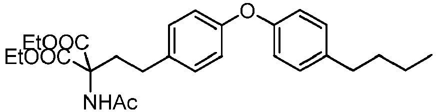

A solution of diethyl 2-acetamido-2-(2-oxo-2-(4-phenoxyphenyl)ethyl) malonate (2.0 g, 4.2 mmol) in CH₂Cl₂ (7 mL) was added dropwise to a solution of Et₃SiH (1.8 g, 15.8 mmol) in CH₂Cl₂ (19 mL) at room temperature under N₂ protection. TiCl₄ (3.0 g, 15.8 mmol) was added to the solution with a syringe and the reaction mixture was stirred overnight, then poured slowly into HCl-ice mixture. The organic layer was separated and the aqueous phase was extracted with CH₂Cl₂ three times. The combined organic layers were washed with H₂O to neutral, dried over Na₂SO₄, filtered and concentrated, yielding the title compound (1.78g) as white solid.
¹HNMR (MERCURY 300 MHz, CDCl₃) δ 7.12 (t, *J* = 8.7 Hz, 4H, 4ArH) 6.90 (dd, *J* = 8.1 Hz, 2.7 Hz, 4H, 4ArH) 6.79 (brs, 1H, NH) 4.26-4.20 (m, 4H, 2CH₂) 2.69 (dd, *J* = 9.9 Hz, 7.2 Hz, 2H, CH₂) 2.59 (t, *J* = 7.5 Hz, 2H, 1CH₂) 2.46 (dd, *J* = 9.0 Hz, 6.6 Hz, 2H, CH₂) 2.02 (s, 3H, CH₃) 1.65-1.55 (m, 2H, 1CH₂) 1.41-1.33 (m, 2H, 1CH₂) 1.27 (t, *J* = 6.9 Hz, 6H, 2CH₃) 0.95 (t, *J* = 7.2 Hz, 3H, 1CH₃)
ESI(m/z) 470 (M+H⁺) 492 (M+Na⁺)

### (6-8) Preparation of N-(4-(4-(4-butylphenoxy)phenyl)-1-hydroxy-2-(hydroxymethyl) butan-2-yl)acetamide

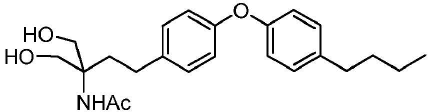

To a solution of diethyl 2-acetamido-2-(4-(4-butylphenoxy)phenethyl)malonate (1.7 g, 3.6 mmol) in 95% EtOH (25mL) was added K₂HPO₄ (6.4 g, 28.3 mmol) in distilled water (6.5 mL) and NaBH₄ (0.7 g, 18.3 mmol) in aq. 10% NaOH(4.7mL). The mixture was stirred for further 6h at room temperature and concentrated. The residue was extracted with EtOAc, washed with H₂O to neutral, dried over Na₂SO₄, filtered and concentrated. The residue was recrystallized with EtOAc to afford the title compound (0.65 g) as white powder solid.
¹HNMR (MERCURY 300 MHz, CDCl₃) δ 7.13 (dd, *J* = 8.1 Hz, 5.7 Hz, 4H, 4ArH) 6.90 (t, *J* = 7.8 Hz, 4H, 4ArH) 5.92 (brs, 1H, NH) 3.86 (d, *J* = 11.7 Hz, 2H, CH₂) 3.79 (brs, 2H, 2OH) 3.63 (d, *J* = 11.1 Hz, 2H, CH₂) 2.65-2.55 (m, 4H, 2CH₂) 2.04-1.93 (m, 5H, 1CH₂, 1CH₃) 1.63-1.53 (m, 2H, CH₂) 1.42-1.29 (m, 2H, CH₂) 0.93 (t, *J* = 7.5 Hz, 3H, CH₃)
ESI(m/z) 386 (M+H⁺)

### (6-9) Preparation of 2-amino-2-(4-(4-butylphenoxy)phenethyl)propane-1,3-diol

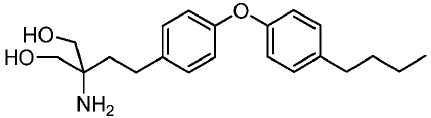

To a solution of *N*-(4-(4-(4-butylphenoxy)phenyl)-1-hydroxy-2-(hydroxymethyl)butan-2-yl)acetamide (0.64 g, 1.67 mmol) in MeOH(10mL) was added NaOH(0.068 g, 1.7 mmol) and heated to reflux for 2 h, then filtered to remove insoluble substance. The solution was added HCl-EtOH solution until pH=3-4 and concentrated. The residue was recrystallized with isopropanol to afford the title compound (0.51 g) as white solid.
¹HNMR (MERCURY 300 MHz, CD₃OD) δ 7.17 (d, *J* = 8.4 Hz, 2H, 2ArH) 7.11 (d, *J* = 8.7 Hz, 2H, 2ArH) 6.83 (dd, *J* = 8.4 Hz, 2.1 Hz, 4H, 4ArH) 3.47 (q, *J* = 10.8 Hz, 4H, 2CH₂) 2.64-2.53 (m, 4H, 2CH₂) 1.66-1.51 (m, 4H, 2CH₂) 1.40-1.30 (m, 2H, CH₂) 0.92 (t, *J* = 7.2 Hz, 3H, CH₃)
¹³C NMR (400 MHz, CD₃OD) δ 180.38, 157.04, 138.90, 130.58, 119.62, 119.54, 66.54, 56.75, 37.82, 35.86, 35.10, 29.68, 23.30, 14.26
ESI(m/z) 344 (M+H⁺) HRMS calcd. for C₂₁H₃₀NO₃(M+H⁺) 344.2220, found 344.2223

### EXAMPLE 7

This example described the preparation of

| | |
|---|---|
| 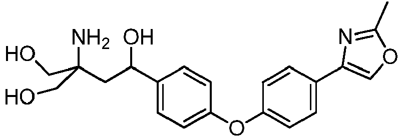 | 3-amino-3-(hydroxymethyl)-1-(4-(4-(2-methyl oxazol-4-yl)phenoxy)phenyl)butane-1,4-diol |
| 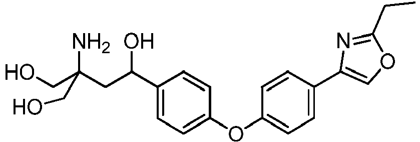 | 3-amino-1-(4-(4-(2-ethyloxazol-4-yl)phenoxy)phe nyl)-3-(hydroxymethyl)butane-1,4-diol |
| 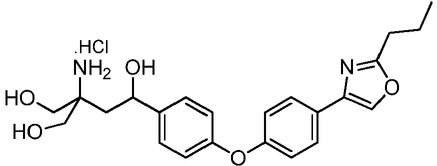 | 3-amino-3-(hydroxymethyl)-1-(4-(4-(2-propylox azol-4-yl)phenoxy)phenyl)butane-1,4-diol hydrochloride |
| 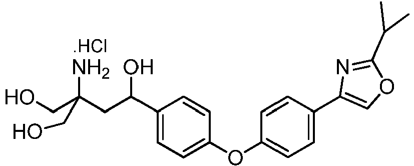 | 3-amino-3-(hydroxymethyl)-1-(4-(4-(2-isopropylox |
| | azol-4-yl)phenoxy)phenyl)butane-1,4-diol hydrochloride |
| 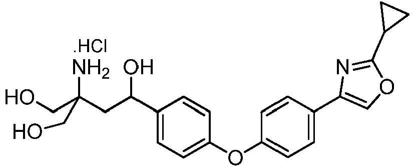 | 3-amino-1-(4-(4-(2-cyclopropyloxazol-4-yl)phenox y)phenyl)-3-(hydroxymethyl)butane-1,4-diol hydrochloride |

### (7-1) Preparation of diethyl 2-acetamido-2-(2-(4-(4-(2-chloroacetyl)phenoxy) phenyl)-2-oxoethyl)malonate

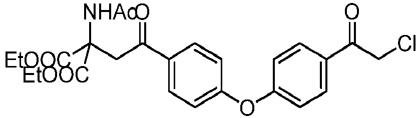

Chloroacetyl chloride (2.1 g, 18.4 mmol) in was added dropwise to a cooled solution (0°C) of diethyl diethyl 2-acetamido-2-(2-oxo-2-(4-phenoxyphenyl)ethyl) malonate (7.5 g, 17.6 mmol) in dry CH₂Cl₂(200mL), then AlCl₃ (12 g, 89.6mmol) was added in portions. The solution was allowed to return to room temperature and stirred for further 5 h, then poured slowly into HCl-ice mixture. The organic layer was separated and the aqueous phase was extracted with CH₂Cl₂ three times. The combined organic layers were washed with H₂O to neutral, dried over Na₂SO₄, filtered and concentrated, yielding the crude product (8.7g) as yellow syrup.
¹HNMR(MERCURY 300 MHz, CDCl₃) δ 8.00 (d, *J* = 8.1 Hz, 4H, 4ArH) 7.10 (d, *J* = 8.1 Hz, 4H, 4ArH) 4.67 (s, 2H, CH₂) 4.31-4.26 (m, 6H, 3CH₂) 1.98 (s, 3H, CH₃) 1.25 (t, *J* = 6.9 Hz, 6H, 2CH₃)
ESI(m/z) 504 (M+H⁺) 526 (M+Na⁺)

### (7-2) Preparation of diethyl 2-acetamido-2-(2-(4-(4-(2-acetoxyacetyl)phenoxy) phenyl)-2-oxoethyl)malonate

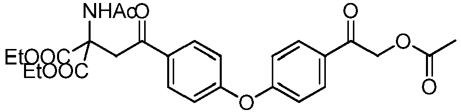

To a solution of diethyl 2-acetamido-2-(2-(4-(4-(2-chloroacetyl)phenoxy) phenyl)-2-oxoethyl)malonate (1.8 g, 3.6 mmol) in CH₃CN (18mL) was added acetic acid (0.49 g, 8.2 mmol) and Et₃N(0.76 g, 7.5 mmol). The mixture was heated to reflux for 2h, then concentrated. The residue was diluted with CH₂Cl₂, washed with H₂O to neutral, dried over Na₂SO₄, filtered and concentrated, yielding the crude product (1.62 g) as yellow syrup.
¹HNMR(MERCURY 300 MHz, CDCl₃) δ 8.00 (d, *J* = 8.7 Hz, 2H, 2ArH) 7.96 (d, *J* = 8.4 Hz, 2H, 2ArH) 7.10 (d, *J* = 8.7 Hz, 2H, 2ArH) 7.09 (d, *J* = 8.4 Hz, 2H, 2ArH) 7.11 (brs, 1H, NH) 5.31 (s, 2H, CH₂) 4.31-4.25 (m, 6H, 3CH₂) 2.24 (s, 3H, CH₃) 1.98 (s, 3H, CH₃) 1.25 (t, *J* = 7.2 Hz, 6H, 2CH₃)
ESI(m/z) 528 (M+H⁺)

### (7-3) Preparation of diethyl 2-acetamido-2-(2-oxo-2-(4-(4-(2-(propionyloxy)acetyl) phenoxy)phenyl)ethyl)malonate

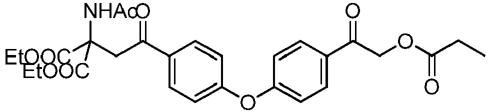

To a solution of diethyl 2-acetamido-2-(2-(4-(4-(2-chloroacetyl)phenoxy) phenyl)-2-oxoethyl)malonate (1.7 g, 3.2 mmol) in CH₃CN (15mL) was added propionic acid (0.55 g, 7.5 mmol) and Et₃N(0.7 g, 6.9 mmol). The mixture was heated to reflux for 2h, then concentrated. The residue was diluted with CH₂Cl₂, washed with H₂O to neutral, dried over Na₂SO₄, filtered and concentrated, yielding the crude product (1.73 g) as yellow syrup.
¹HNMR(MERCURY 300 MHz, CDCl₃) δ 8.00 (d, *J* = 8.7 Hz, 2H, 2ArH) 7.95 (d, *J* = 8.7 Hz, 2H, 2ArH) 7.10 (d, *J* = 8.7 Hz, 2H, 2ArH) 7.08 (d, *J* = 8.4 Hz, 2H, 2ArH) 7.11 (brs, 1H, NH) 5.31 (s, 2H, CH₂) 4.31-4.25 (m, 6H, 3CH₂) 2.53 (q, *J* = 7.5 Hz, 2H, 1CH₂) 1.98 (s, 3H, CH₃) 1.27-1.19 (m, 9H, 3CH₃)
ESI(m/z) 542 (M+H⁺)

### (7-4) Preparation of diethyl 2-acetamido-2-(2-(4-(4-(2-(butyryloxy)acetyl)phenoxy) phenyl)-2-oxoethyl)malonate

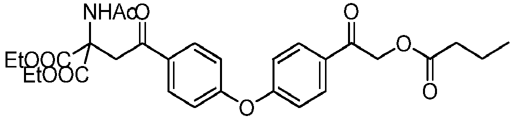

To a solution of diethyl 2-acetamido-2-(2-(4-(4-(2-chloroacetyl)phenoxy) phenyl)-2-oxoethyl)malonate (1.6 g, 3.1 mmol) in CH₃CN (15mL) was added *n*-butyric acid (0.63 g, 7.1 mmol) and Et₃N(0.66 g, 6.6 mmol). The mixture was heated to reflux for 2h, then concentrated. The residue was diluted with CH₂Cl₂, washed with H₂O to neutral, dried over Na₂SO₄, filtered and concentrated, yielding the crude product (1.72 g) as yellow syrup.
¹HNMR(MERCURY 300 MHz, CDCl₃) δ 8.00 (d, *J* = 8.7 Hz, 2H, 2ArH) 7.95 (d, *J* = 9.0 Hz, 2H, 2ArH) 7.10 (d, *J* = 9.0 Hz, 2H, 2ArH) 7.08 (d, *J* = 8.7 Hz, 2H, 2ArH) 7.11 (brs, 1H, NH) 5.31 (s, 2H, CH₂) 4.31-4.24 (m, 6H, 3CH₂) 2.48 (t, *J* = 7.2 Hz, 2H, CH₂) 1.98 (s, 3H, CH₃) 1.78-1.71 (m, 2H, CH₂) 1.25 (t, *J* = 6.9 Hz, 6H, 2CH₃) 1.01 (t, *J =* 7.5 Hz, 3H, 1CH₃)
ESI(m/z) 556 (M+H⁺)

### (7-5) Preparation of diethyl 2-acetamido-2-(2-(4-(4-(2-(isobutyryloxy)acetyl)phenoxy) phenyl)-2-oxoethyl)malonate

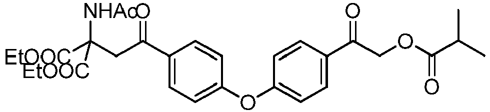

To a solution of diethyl 2-acetamido-2-(2-(4-(4-(2-chloroacetyl)phenoxy) phenyl)-2-oxoethyl)malonate (1.98 g, 3.9 mmol) in CH₃CN (20mL) was added isobutyric acid (0.79 g, 8.9 mmol) and Et₃N(0.84 g, 8.3 mmol). The mixture was heated to reflux for 2h, then concentrated. The residue was diluted with CH₂Cl₂, washed with H₂O to neutral, dried over Na₂SO₄, filtered and concentrated, yielding the crude product (2.2 g) as yellow syrup.
¹HNMR(MERCURY 300 MHz, CDCl₃) δ 8.00 (d, *J* = 8.7 Hz, 2H, 2ArH) 7.95 (d, *J =* 9.0 Hz, 2H, 2ArH) 7.10 (d, *J* = 9.0 Hz, 2H, 2ArH) 7.08 (d, *J* = 8.7 Hz, 2H, 2ArH) 7.11 (brs, 1H, NH) 5.30 (s, 2H, CH₂) 4.31-4.25 (m, 6H, 3CH₂) 2.79-2.70 (m, 1H, 1CH) 1.98 (s, 3H, CH₃) 1.28-1.22 (m, 12H, 4CH₃)
ESI(m/z) 556 (M+H⁺) 578 (M+Na⁺)

### (7-6) Preparation of diethyl 2-acetamido-2-(2-(4-(4-(2-((cyclopropanecarbonyl)oxy) acetyl)phenoxy)phenyl)-2-oxoethyl)malonate

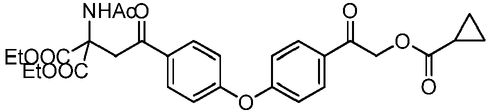

To a solution of diethyl 2-acetamido-2-(2-(4-(4-(2-chloroacetyl)phenoxy) phenyl)-2-oxoethyl)malonate (1.7 g, 3.4 mmol) in CH₃CN (15 mL) was added cyclopropanecarboxylic acid (0.66 g, 7.7 mmol) and Et₃N(0.72 g, 7.1 mmol). The mixture was heated to reflux for 2h, then concentrated. The residue was diluted with CH₂Cl₂, washed with H₂O to neutral, dried over Na₂SO₄, filtered and concentrated, yielding the crude product (1.88 g) as yellow syrup.
¹HNMR(MERCURY 300 MHz, CDCl₃) δ 7.99 (d, *J* = 9.0 Hz, 2H, 2ArH) 7.95 (d, *J* = 8.7 Hz, 2H, 2ArH) 7.10 (d, *J* = 9.0 Hz, 2H, 2ArH) 7.07 (d, *J* = 8.4 Hz, 2H, 2ArH) 7.10 (brs, 1H, NH) 5.30 (s, 2H, CH₂) 4.30-4.24 (m, 6H, 3CH₂) 1.97 (s, 3H, CH₃) 1.83-1.76 (m, 1H, 1CH) 1.24 (t, *J* = 6.9 Hz, 6H, 2CH₃) 1.11-1.00 (m, 2H, CH₂) 0.98-0.95 (m, 2H, CH₂)
ESI(m/z) 554 (M+H⁺)

### (7-7) Preparation of diethyl 2-acetamido-2-(2-(4-(4-(2-methyloxazol-4-yl)phenoxy) phenyl)-2-oxoethyl)malonate

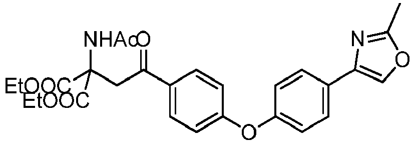

To a solution of diethyl diethyl 2-acetamido-2-(2-(4-(4-(2-acetoxyacetyl)phenoxy) phenyl)-2-oxoethyl)malonate (1.9 g, 3.6 mmol) in xylene(30mL) was added acetamide (1.1 g, 18 mmol) and 47%BF₃·Et₂O(0.34 mL). The mixture was heated to reflux for 40h, then concentrated. The residue was diluted with EtOAc, washed with H₂O (three times) and brine, dried over Na₂SO₄, filtered and concentrated. The residue was purified by silica gel column chromatography to afford the title compound (1.07g) as yellow syrup.
¹HNMR(MERCURY 300 MHz, CDCl₃) δ 7.94 (d, *J* = 9.0 Hz, 2H, 2ArH) 7.80(s, 1H, ArH)7.74 (d, *J* = 8.7 Hz, 2H, 2ArH) 7.11 (brs, 1H, NH) 7.09 (d, *J* = 8.7 Hz, 2H, 2ArH) 7.01 (d, *J* = 9.0 Hz, 2H, 2ArH) 4.30-4.22 (m, 6H, 3CH₂) 2.54 (s, 3H, CH₃) 1.97 (s, 3H, CH₃) 1.25 (t, *J* = 7.5 Hz, 6H, 2CH₃)
ESI(m/z) 509 (M+H⁺)

### (7-8) Preparation of diethyl 2-acetamido-2-(2-(4-(4-(2-ethyloxazol-4-yl)phenoxy) phenyl)-2-oxoethyl)malonate

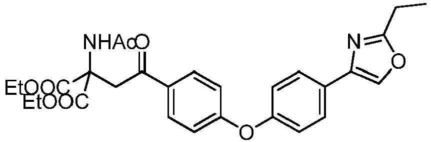

To a solution of diethyl diethyl 2-acetamido-2-(2-oxo-2-(4-(4-(2-(propionyloxy) acetyl)phenoxy)phenyl)ethyl)malonate (1.73 g, 3.2 mmol) in xylene(20mL) was added acetamide (0.95 g, 16 mmol) and 47%BF_{3·}Et₂O(0.31 mL). The mixture was heated to reflux for 40h, then concentrated. The residue was diluted with EtOAc, washed with H₂O (three times) and brine, dried over Na₂SO₄, filtered and concentrated. The residue was purified by silica gel column chromatography to afford the title compound (1.2g) as yellow syrup.
¹HNMR(MERCURY 300 MHz, CDCl₃) δ 7.94 (d, *J* = 8.1 Hz, 2H, 2ArH) 7.80(s, 1H, ArH)7.75 (d, *J* = 7.8 Hz, 2H, 2ArH) 7.11 (brs, 1H, NH) 7.09 (d, *J* = 8.7 Hz, 2H, 2ArH) 7.01 (d, *J* = 8.1 Hz, 2H, 2ArH) 4.30-4.22 (m, 6H, 3CH₂) 2.86 (q, *J* = 7.8Hz, 2H, CH₂) 1.97 (s, 3H, CH₃) 1.38 (t, *J* = 7.2 Hz, 6H, 2CH₃) 1.25 (t, *J* = 7.2 Hz, 6H, 2CH₃) ESI(m/z) 523 (M+H⁺) 545 (M+Na⁺)

### (7-9) Preparation of diethyl 2-acetamido-2-(2-oxo-2-(4-(4-(2-propyloxazol-4-yl) phenoxy)phenyl)ethyl)malonate

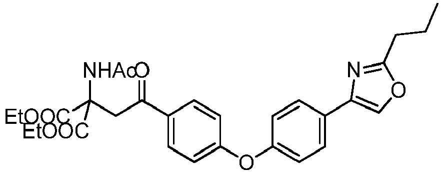

To a solution of diethyl 2-acetamido-2-(2-(4-(4-(2-(butyryloxy)acetyl)phenoxy) phenyl)-2-oxoethyl)malonate (1.72 g, 3.1 mmol) in xylene(30mL) was added acetamide (0.92 g, 15.5 mmol) and 47%BF₃·Et₂O(0.3 mL). The mixture was heated to reflux for 40h, then concentrated. The residue was diluted with EtOAc, washed with H₂O (three times) and brine, dried over Na₂SO₄, filtered and concentrated, yielding the residue (1.0g) as yellow syrup. The crude product was used in the next step without purification.

### (7-10) Preparation of diethyl 2-acetamido-2-(2-(4-(4-(2-isopropyloxazol-4-yl) phenoxy)phenyl)-2-oxoethyl)malonate

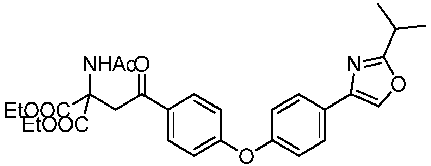

To a solution of diethyl 2-acetamido-2-(2-(4-(4-(2-(isobutyryloxy) acetyl)phenoxy)phenyl)-2-oxoethyl)malonate (2.2 g, 3.9 mmol) in xylene(32mL) was added acetamide (1.15 g, 19.5 mmol) and 47%BF₃·Et₂O(0.37 mL). The mixture was heated to reflux for 40h, then concentrated. The residue was diluted with EtOAc, washed with H₂O (three times) and brine, dried over Na₂SO₄, filtered and concentrated, yielding the residue (1.3g) as yellow syrup. The crude product was used in the next step without purification.

### (7-11) Preparation of diethyl 2-acetamido-2-(2-(4-(4-(2-cyclopropyloxazol-4-yl) phenoxy)phenyl)-2-oxoethyl)malonate

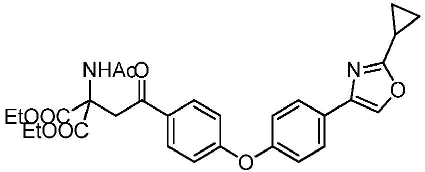

To a solution of diethyl 2-acetamido-2-(2-(4-(4-(2-((cyclopropanecarbonyl)oxy) acetyl)phenoxy)phenyl)-2-oxoethyl)malonate (1.88 g, 3.4 mmol) in xylene(30 mL) was added acetamide (1.0 g, 17 mmol) and 47%BF₃·Et₂O(0.32 mL). The mixture was heated to reflux for 40h, then concentrated. The residue was diluted with EtOAc, washed with H₂O (three times) and brine, dried over Na₂SO₄, filtered and concentrated, yielding the residue (1.3g) as yellow syrup. The crude product was used in the next step without purification.
¹HNMR(MERCURY 300 MHz, CDCl₃) δ 7.94 (d, *J* = 8.7 Hz, 2H, 2ArH) 7.73(s, 1H, ArH)7.72 (d, *J* = 8.4 Hz, 2H, 2ArH) 7.11 (brs, 1H, NH) 7.08 (d, *J* = 8.4 Hz, 2H, 2ArH) 7.01 (d, *J* = 8.7 Hz, 2H, 2ArH) 4.30-4.22 (m, 6H, 3CH₂) 2.16-2.10 (m, 1H, 1CH) 2.01 (s, 3H, 1CH₃) 1.25 (t, *J* = 7.5 Hz, 6H, 2CH₃) 1.13-1.06 (m, 4H, 2CH₂)
ESI(m/z) 535 (M+H⁺)

### (7-12) Preparation of N-(1,4-dihydroxy-2-(hydroxymethyl)-4-(4-(4-(2-methyloxazol -4-yl)phenoxy)phenyl)butan-2-yl)acetamide

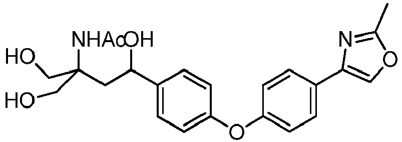

To a solution of diethyl 2-acetamido-2-(2-(4-(4-(2-methyloxazol-4-yl)phenoxy) phenyl)-2-oxoethyl)malonate (1.07 g, 2.5 mmol) in 95% EtOH (18mL) was added K₂HPO₄ (4.5 g, 19.8 mmol) in distilled water (4.5 mL) and NaBH₄ (0.49 g, 12.9 mmol) in aq. 10% NaOH(3.3mL). The mixture was stirred for further 6h at room temperature and concentrated. The residue was extracted with EtOAc, washed with H₂O to neutral, dried over Na₂SO₄, filtered and concentrated. The residue was recrystallized with EtOAc to afford the title compound (0.48 g) as white powder solid.
¹HNMR(MERCURY 300 MHz, CDCl₃) δ 7.77(s, 1H, 1ArH)7.68 (d, *J* = 8.7 Hz, 2H, 2ArH) 7.32 (d, *J* = 8.4 Hz, 2H, 2ArH) 7.01 (d, *J* = 9.0 Hz, 2H, 2ArH) 7.00 (d, *J* = 8.4 Hz, 2H, 2ArH) 4.90 (d, *J* = 10.2 Hz, 1H, CH) 3.79 (d, *J* = 12.3 Hz, 1H, CH₂) 3.75 (d, *J* = 12.3 Hz, 1H, CH₂) 3.61 (d, *J* = 11.7 Hz, 1H, CH₂) 3.48 (d, *J* = 12.0 Hz, 1H, CH₂) 2.53(s, 3H, CH₃) 2.38 (d, *J* = 15.6 Hz, 1H, CH₂) 2.04(s, 3H, CH₃) 1.83 (dd, *J* = 15.0 Hz, 10.8Hz, 1H, CH₂)
ESI(m/z) 409 (M-OH⁺)

### (7-13) Preparation of N-(4-(4-(4-(2-ethyloxazol-4-yl)phenoxy)phenyl)-1,4-dihydroxy -2-(hydroxymethyl)butan-2-yl)acetamide

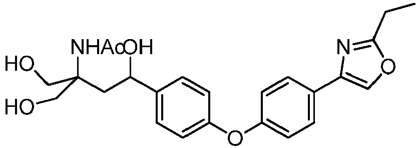

To a solution of diethyl 2-acetamido-2-(2-(4-(4-(2-ethyloxazol-4-yl)phenoxy) phenyl)-2-oxoethyl)malonate (1.2 g, 2.7 mmol) in 95% EtOH (19mL) was added K₂HPO₄ (4.9 g, 21.5 mmol) in distilled water (4.9 mL) and NaBH₄ (0.53 g, 13.9 mmol) in aq. 10% NaOH(3.6mL). The mixture was stirred for further 6h at room temperature and concentrated. The residue was extracted with EtOAc, washed with H₂O to neutral, dried over Na₂SO₄, filtered and concentrated. The residue was recrystallized with EtOAc to afford the title compound (0.61 g) as white powder solid.
¹HNMR(MERCURY 300 MHz, CDCl₃) δ 7.78(s, 1H, 1ArH)7.69 (d, *J* = 8.4 Hz, 2H, 2ArH) 7.32 (d, *J* = 8.4 Hz, 2H, 2ArH) 7.03-6.98 (m, 4H, 4ArH) 4.90 (d, *J* = 10.2 Hz, 1H, CH) 3.79 (d, *J* = 12.3 Hz, 1H, CH₂) 3.75 (d, *J* = 12.3 Hz, 1H, CH₂) 3.61 (d, *J* = 11.7 Hz, 1H, CH₂) 3.48 (d, *J* = 12.0 Hz, 1H, CH₂) 2.87 (q, *J* = 7.5 Hz, 2H, CH₂) 2.38 (d, *J* = 15.6 Hz, 1H, CH₂) 2.04(s, 3H, CH₃) 1.83 (dd, *J* = 15.0 Hz, 10.8Hz, 1H, CH₂) 1.38 (t, *J* = 7.8 Hz, 3H, CH₃)
ESI(m/z) 423 (M-OH⁺)

### (7-14) Preparation of N-(1,4-dihydroxy-2-(hydroxymethyl)-4-(4-(4-(2-propyloxazol -4-yl)phenoxy)phenyl)butan-2-yl)acetamide

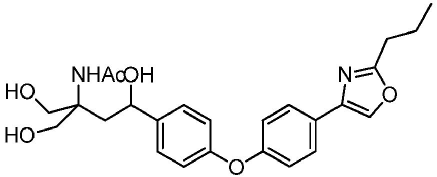

To a solution of diethyl 2-acetamido-2-(2-oxo-2-(4-(4-(2-propyloxazol-4-yl) phenoxy)phenyl)ethyl)malonate (1.0 g, 2.2 mmol) in 95% EtOH (16 mL) was added K₂HPO₄ (4.0 g, 17.4 mmol) in distilled water (4 mL) and NaBH₄ (0.43 g, 11.3 mmol) in aq. 10% NaOH(2.9mL). The mixture was stirred for further 6h at room temperature and concentrated. The residue was extracted with EtOAc, washed with H₂O to neutral, dried over Na₂SO₄, filtered and concentrated. The residue was recrystallized with EtOAc to afford the title compound (0.52 g) as white powder solid.
¹HNMR(MERCURY 300 MHz, CDCl₃) δ 7.77(s, 1H, 1ArH)7.68 (d, *J* = 8.4 Hz, 2H, 2ArH) 7.32 (d, *J* = 8.4 Hz, 2H, 2ArH) 7.01 (d, *J* = 8.7 Hz, 2H, 2ArH) 6.99 (d, *J* = 8.4 Hz, 2H, 2ArH) 4.90 (d, *J* = 10.2 Hz, 1H, CH) 3.75 (d, *J* = 13.2 Hz, 1H, CH₂) 3.75 (d, *J* = 12.3 Hz, 1H, CH₂) 3.63 (d, *J* = 11.4 Hz, 1H, CH₂) 3.49 (d, *J* = 12.3 Hz, 1H, CH₂) 2.78 (t, *J* = 8.1 Hz, 2H, CH₂) 2.38 (d, *J* = 15.6 Hz, 1H, CH₂) 2.05 (s, 3H, CH₃) 1.87-1.79 (m, 3H, 2CH₂) 1.01 (t, *J* = 7.5 Hz, 3H, CH₃)
ESI(m/z) 437 (M-OH⁺)

### (7-15) Preparation of N-(1,4-dihydroxy-2-(hydroxymethyl)-4-(4-(4-(2-isopropyloxazol-4-yl)phenoxy)phenyl)butan-2-yl)acetamide

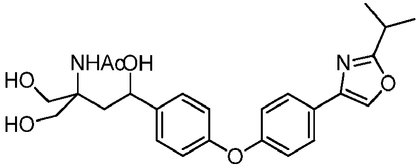

To a solution of diethyl 2-acetamido-2-(2-(4-(4-(2-isopropyloxazol-4-yl) phenoxy)phenyl)-2-oxoethyl)malonate (1.3 g, 2.8 mmol) in 95% EtOH (20 mL) was added K₂HPO₄ (5.2 g, 22.6 mmol) in distilled water (5.2 mL) and NaBH₄ (0.56 g, 14.7 mmol) in aq. 10% NaOH(3.8mL). The mixture was stirred for further 6h at room temperature and concentrated. The residue was extracted with EtOAc, washed with H₂O to neutral, dried over Na₂SO₄, filtered and concentrated. The residue was recrystallized with EtOAc to afford the title compound (0.77 g) as white powder solid. ¹HNMR(MERCURY 300 MHz, CDCl₃) δ 7.77(s, 1H, 1ArH)7.69 (d, *J* = 8.1 Hz, 2H, 2ArH) 7.31 (d, *J* = 8.1 Hz, 2H, 2ArH) 7.03 (brs, 1H, NH) 6.99 (d, *J* = 7.2 Hz, 4H, 4ArH) 4.90 (d, *J* = 10.2 Hz, 1H, CH) 3.79 (d, *J* = 12.0 Hz, 1H, CH₂) 3.75 (d, *J* = 13.2 Hz, 1H, CH₂) 3.61 (d, *J* = 12.3 Hz, 1H, CH₂) 3.49 (d, *J* = 11.4 Hz, 1H, CH₂) 3.20-3.11 (m, 1H, 1CH) 2.38 (d, *J* = 15.0 Hz, 11.1Hz, 1H, CH₂) 2.05(s, 3H, CH₃) 1.84 (d, *J* = 14.4 Hz, 10.8Hz, 1H, CH₂) 1.40 (brs, 3H, CH₃) 1.38 (brs, 3H, CH₃)
ESI(m/z) 437 (M-OH⁺)

### (7-16) Preparation of N-(4-(4-(4-(2-cyclopropyloxazol-4-yl)phenoxy)phenyl) -1,4-dihydroxy-2-(hydroxymethyl)butan-2-yl)acetamide

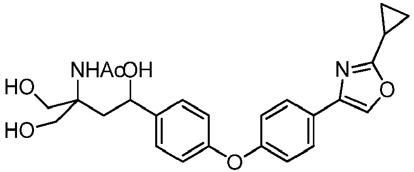

To a solution of diethyl 2-acetamido-2-(2-(4-(4-(2-cyclopropyloxazol-4-yl) phenoxy)phenyl)-2-oxoethyl)malonate (1.3 g, 2.8 mmol) in 95% EtOH (20 mL) was added K₂HPO₄ (5.2 g, 22.7 mmol) in distilled water (5.2 mL) and NaBH₄ (0.56 g, 14.7 mmol) in aq. 10% NaOH(3.8mL). The mixture was stirred for further 6h at room temperature and concentrated. The residue was extracted with EtOAc, washed with H₂O to neutral, dried over Na₂SO₄, filtered and concentrated. The residue was recrystallized with EtOAc to afford the title compound (0.75 g) as white powder solid. ¹HNMR(MERCURY 300 MHz, CDCl₃) δ 7.68(s, 1H, 1ArH)7.64 (d, *J* = 8.1 Hz, 2H, 2ArH) 7.30 (d, *J* = 7.8 Hz, 2H, 2ArH) 7.02 (brs, 1H, NH) 6.99 (d, *J* = 7.8 Hz, 2H, 2ArH) 6.97 (d, *J* = 8.1 Hz, 2H, 2ArH) 4.88 (d, *J* = 10.5 Hz, 1H, CH) 3.77 (d, *J* = 12.3 Hz, 1H, CH₂) 3.73 (d, *J* = 14.7 Hz, 1H, CH₂) 3.58 (d, *J* = 11.7 Hz, 1H, CH₂) 3.48 (d, *J* = 12.0 Hz, 1H, CH₂) 2.33 (d, *J* = 14.7 Hz, 1H, CH₂) 2.12-1.97 (m, 4H, 1CH, 1CH₃) 1.82 (dd, *J =* 15.0 Hz, 11.1Hz, 1H, CH₂) 1.10-1.01 (m, 4H, 2CH₂)
ESI(m/z) 435 (M-OH⁺)

### (7-17) Preparation of 3-amino-3-(hydroxymethyl)-1-(4-(4-(2-methyloxazol-4-yl) phenoxy)phenyl)butane-1,4-diol

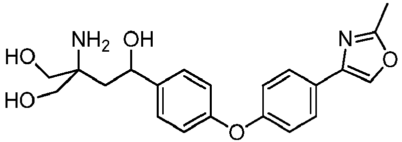

To a solution of N-(1,4-dihydroxy-2-(hydroxymethyl)-4-(4-(4-(2-methyloxazol -4-yl)phenoxy)phenyl)butan-2-yl)acetamide (0.48 g, 1.1 mmol) in MeOH(10mL) was added NaOH(0.046 g, 1.2 mmol) and heated to reflux for 8h, then filtered to remove insoluble substance. The solution was added HCl-EtOH solution until pH=3-4 and concentrated. The residue was purified by silica gel column chromatography to afford the title compound (0.3 g) as light yellow solid.
¹HNMR(MERCURY 300 MHz, CD₃OD) δ 7.94(s, 1H, 1ArH) 7.55 (d, *J* = 8.7 Hz, 2H, 2ArH) 7.28 (d, *J* = 8.4 Hz, 2H, 2ArH) 6.86 (d, *J* = 8.4 Hz, 2H, 2ArH) 6.85 (d, *J* = 8.4 Hz, 2H, 2ArH) 4.90 (dd, *J* = 10.5, 2.4 Hz, 1H, CH) 3.74 (d, *J* = 11.4 Hz, 1H, CH₂) 3.69 (d, *J* = 11.7 Hz, 1H, CH₂) 3.58 (d, *J* = 11.4 Hz, 1H, CH₂) 3.50 (d, *J* = 11.4 Hz, 1H, CH₂) 2.35(s, 3H, CH₃) 1.90-1.72 (m, 2H, CH₂)
ESI(m/z) 388 (M+H⁺) HRMS calcd. for C₂₃H₃₄NO₄ (M+H⁺) 388.2482, found 388.2488

### (7-18) Preparation of 3-amino-1-(4-(4-(2-ethyloxazol-4-yl)phenoxy)phenyl) -3-(hydroxymethyl)butane-1,4-diol

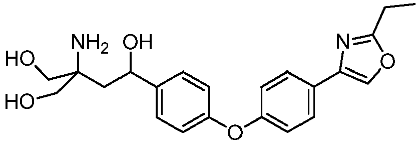

To a solution of *N*-(4-(4-(4-(2-ethyloxazol-4-yl)phenoxy)phenyl)-1,4-dihydroxy -2-(hydroxymethyl)butan-2-yl)acetamide (0.61 g, 1.3 mmol) in MeOH(10mL) was added NaOH(0.057 g, 1.4 mmol) and heated to reflux for 8h, then filtered to remove insoluble substance. The solution was added HCl-EtOH solution until pH=3-4 and concentrated. The residue was purified by silica gel column chromatography to afford the title compound (0.23 g) as light yellow solid.
¹HNMR(MERCURY 300 MHz, CD₃OD) δ 8.01(s, 1H, 1ArH) 7.63 (d, J= 8.4 Hz, 2H, 2ArH) 7.54 (d, *J* = 8.4 Hz, 2H, 2ArH) 6.93 (d, *J* = 8.1 Hz, 4H, 4ArH) 4.93 (d, *J =* 10.2 Hz, 1H, CH) 3.58 (brs, 2H, CH₂) 3.48 (brs, 2H, CH₂) 2.35(s, 3H, CH₃) 1.90-1.72 (m, 2H, CH₂) 2.78 (q, *J* = 7.8Hz, 2H, CH₂) 1.87-1.66 (m, 2H, CH₂) 1.30 (t, *J* = 7.8Hz, 3H, CH₃)
ESI(m/z) 399 (M+H⁺) HRMS calcd. for C₂₂H₂₇N₂O₅ (M+H⁺) 399.1914, found 399.1903

### (7-19) Preparation of 3-amino-3-(hydroxymethyl)-1-(4-(4-(2-propyloxazol-4-yl) phenoxy)phenyl)butane-1,4-diol hydrochloride

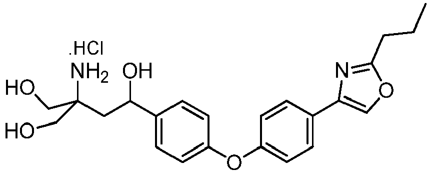

To a solution of N-(1,4-dihydroxy-2-(hydroxymethyl)-4-(4-(4-(2-propyloxazol -4-yl)phenoxy)phenyl)butan-2-yl)acetamide (0.52 g, 1.14 mmol) in MeOH(10mL) was added NaOH(0.047 g, 1.18 mmol) and heated to reflux for 8h, then filtered to remove insoluble substance. The solution was added HCl-EtOH solution until pH=3-4 and concentrated. The residue was purified by silica gel column chromatography to afford the title compound (0.4 g) as light yellow solid.
¹HNMR(MERCURY 300 MHz, CD₃OD) δ 8.17(s, 1H, 1ArH) 7.63 (d, *J* = 8.4 Hz, 2H, 2ArH) 7.35 (d, *J =* 8.4 Hz, 2H, 2ArH) 6.94 (d, *J* = 8.1 Hz, 4H, 4ArH) 4.97 (d, *J* = 9.0 Hz, 1H, CH) 3.83 (d, *J* = 11.7 Hz, 1H, CH₂) 3.77 (d, *J* = 11.4 Hz, 1H, CH₂) 3.65 (d, *J* = 11.7 Hz, 1H, CH₂) 3.56 (d, *J* = 11.7 Hz, 1H, CH₂) 2.54 (t, *J* = 7.8Hz, 2H, CH₂) 1.98-1.74 (m, 4H, 2CH₂) 0.95 (t, *J* = 7.2Hz, 3H, 1CH₃)
ESI(m/z) 413 (M+H⁺) HRMS calcd. for C₂₃H₂₉N₂O₅ (M+H⁺) 413.2071, found 413.2061

### (7-20) Preparation of 3-amino-3-(hydroxymethyl)-1-(4-(4-(2-isopropyloxazol-4-yl) phenoxy)phenyl)butane-1,4-diol hydrochloride

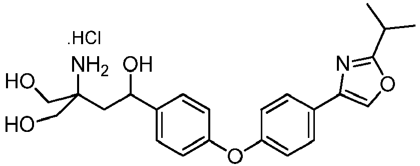

To a solution of N-(1,4-dihydroxy-2-(hydroxymethyl)-4-(4-(4-(2-isopropyloxazol-4-yl)phenoxy)phenyl)butan-2-yl)acetamide (0.77 g, 1.7 mmol) in MeOH(10mL) was added NaOH(0.068 g, 1.76 mmol) and heated to reflux for 8h, then filtered to remove insoluble substance. The solution was added HCl-EtOH solution until pH=3-4 and concentrated. The residue was purified by silica gel column chromatography to afford the title compound (0.4 g) as light yellow solid.
¹HNMR(MERCURY 300 MHz, CD₃OD) δ 8.08(s, 1H, 1ArH) 7.64 (d, *J* = 8.4 Hz, 2H, 2ArH) 7.35 (d, *J* = 8.7 Hz, 2H, 2ArH) 6.94 (d, *J* = 8.4 Hz, 2H, 2ArH) 6.93 (d, *J* = 8.4 Hz, 2H, 2ArH) 4.97 (d, *J* = 8.7 Hz, 1H, 1CH) 3.83 (d, *J* = 11.4 Hz, 1H, CH₂) 3.77 (d, *J* = 11.1 Hz, 1H, CH₂) 3.66 (d, *J* = 11.4 Hz, 1H, CH₂) 3.57 (d, *J* = 11.4 Hz, 1H, CH₂) 3.15-3.10 (m, 1H, 1CH) 1.98-1.81 (m, 2H, CH₂) 1.33 (brs, 3H, CH₃) 1.31 (brs, 3H, CH₃)
ESI(m/z) 413 (M+H⁺) HRMS calcd. for C₂₃H₂₉N₂O₅ (M+H⁺) 413.2071, found 413.2054

### (7-21) Preparation of 3-amino-1-(4-(4-(2-cyclopropyloxazol-4-yl)phenoxy) phenyl)-3-(hydroxymethyl)butane-1,4-diol hydrochloride

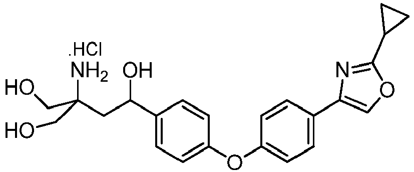

To a solution of N-(4-(4-(4-(2-cyclopropyloxazol-4-yl)phenoxy)phenyl) -1,4-dihydroxy-2-(hydroxymethyl)butan-2-yl)acetamide (0.75 g, 1.6 mmol) in MeOH(10mL) was added NaOH(0.068 g, 1.7 mmol) and heated to reflux for 8h, then filtered to remove insoluble substance. The solution was added HCl-EtOH solution until pH=3-4 and concentrated. The residue was purified by silica gel column chromatography to afford the title compound (0.5 g) as light yellow solid.
¹HNMR(MERCURY 300 MHz, CD₃OD) δ 8.17(s, 1H, 1ArH) 7.61 (d, *J* = 8.7 Hz, 2H, 2ArH) 7.36 (d, *J* = 8.1 Hz, 2H, 2ArH) 6.95 (d, *J* = 6.9 Hz, 4H, 4ArH) 4.98 (d, *J =* 10.2 Hz, 1H, 1CH) 3.82 (d, *J =* 11.4 Hz, 1H, CH₂) 3.77 (d, *J* = 11.7 Hz, 1H, CH₂) 3.66 (d, *J* = 11.4 Hz, 1H, CH₂) 3.57 (d, *J* = 11.4 Hz, 1H, CH₂) 2.30-2.21 (m, 1H, 1CH) 1.98-1.80 (m, 2H, 1CH₂) 1.25-1.22 (m, 4H, 2CH₂)
ESI(m/z) 411 (M+H⁺) HRMS calcd. for C₂₃H₂₇N₂O₅ (M+H⁺) 411.1914, found 411.1916

### EXAMPLE 8

This example described the preparation of

| | |
|---|---|
| 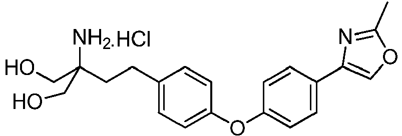 | 2-amino-2-(4-(4-(2-methyloxazol-4-yl)phenoxy)phe nethyl)propane-1,3-diol hydrochloride |
| 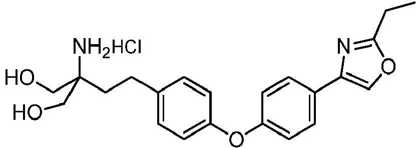 | 2-amino-2-(4-(4-(2-ethyloxazol-4-yl)phenoxy)phe nethyl)propane-1,3-diol hydrochloride |
| 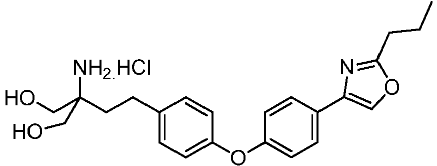 | 2-amino-2-(4-(4-(2-propyloxazol-4-yl)phenoxy)p henethyl)propane-1,3-diol hydrochloride |
| 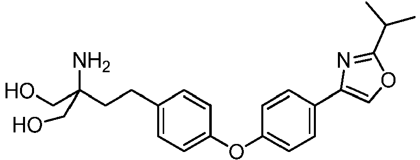 | 2-amino-2-(4-(4-(2-isopropyloxazol-4-yl)phenoxy) phenethyl)propane-1,3-diol |
| 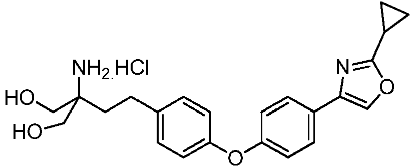 | 2-amino-2-(4-(4-(2-cyclopropyloxazol-4-yl)phenox y)phenethyl)propane-1,3-diol hydrochloride |

### (8-1) Preparation of 2-chloro-1-(4-phenoxyphenyl)ethanone

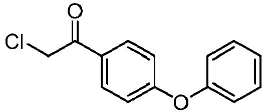

Chloroacetyl chloride (13.3 g, 117.5 mmol) in was added dropwise to a cooled solution (0°C) of diphenyl ether (20 g, 117.5 mmol) in dry CH₂Cl₂(200mL), then AlCl₃ (16.5 g, 123.4 mmol) was added in portions. The solution was allowed to return to room temperature and stirred for further 4 h, then poured slowly into HCl-ice mixture. The organic layer was separated and the aqueous phase was extracted with CH₂Cl₂ three times. The combined organic layers were washed with H₂O to neutral, dried over Na₂SO₄, filtered and concentrated, yielding the crude product (27g) as yellow oil. The crude product was used in the next step without purification.

### (8-2) Preparation of diethyl 2-acetamido-2-(2-oxo-2-(4-phenoxyphenyl)ethyl) malonate

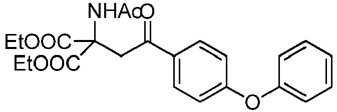

NaH (4.5g, 131mmol) was added to dry THF (300mL) at room temperature. After 30 min, diethyl acetamidomalonate (29.7g, 137mmol) was added in portions and the mixture was stirred for a further 5h. Then a solution of 2-chloro-1-(4-phenoxyphenyl)ethanone (27g, 109mmol) in THF was added. The mixture was heated to reflux for further 12h and concentrated. The residue was diluted with EtOAc, washed with H₂O to neutral, dried over Na₂SO₄, filtered and concentrated. The residue was purified by silica gel column chromatography to afford the title compound (30g) as colorless syrup.
¹HNMR(MERCURY 300MHz, CDCl₃) δ 7.93(d, J=8.7Hz, 2H, 2ArH)7.40(t, *J* =8.0Hz, 2H, 2ArH)7.21(t, J=7.5Hz, 1H, 1ArH)7.10(brs, 1H, NH)7.06(d, *J* =7.8Hz, 2H, 2ArH)6.98(d, J=9.0Hz, 2H, 2ArH)4.27(q, J=7.2Hz, 4H, 2CH₂)4.22(s, 2H, CH₂)1.97(s, 3H, CH₃)1.23(t, J=7.2Hz, 6H, 2CH₃)
ESI(m/z)428(M+H⁺)450(M+Na⁺)

### (8-3) Preparation of diethyl 2-acetamido-2-(4-phenoxyphenethyl)malonate

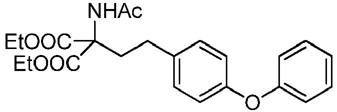

A solution of diethyl 2-acetamido-2-(2-oxo-2-(4-phenoxyphenyl)ethyl) malonate (10.2g, 23.9mmol) in CH₂Cl₂ (38mL) was added dropwise to a solution of Et₃SiH (10.5g, 90.7mmol) in CH₂Cl₂ (100mL) at room temperature under N₂ protection. TiCl₄ (17.2g, 90.7mmol) was added to the solution with a syringe and the reaction mixture was stirred overnight, then poured slowly into HCl-ice mixture. The organic layer was separated and the aqueous phase was extracted with CH₂Cl₂ three times. The combined organic layers were washed with H₂O to neutral, dried over Na₂SO₄, filtered and concentrated. The crude product was used in the next step without purification.

### (8-4) Preparation of diethyl 2-acetamido-2-(4-(4-(2-chloroacetyl)phenoxy)phenethyl) malonate

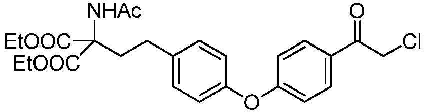

Chloroacetyl chloride (4.7 g, 41.2 mmol 1) in CH₂Cl₂(20mL) was added dropwise to a cooled solution (0°C) of diethyl 2-acetamido-2-(4-phenoxyphenethyl) malonate (15.5 g, 37.5 mmol) in dry CH₂Cl₂(200mL), then AlCl₃ (25 g, 188 mmol) was added in portions. The solution was allowed to return to room temperature and stirred for further 5 h, then poured slowly into HCl-ice mixture. The organic layer was separated and the aqueous phase was extracted with CH₂Cl₂ three times. The combined organic layers were washed with H₂O to neutral, dried over Na₂SO₄, filtered and concentrated. The residue was purified by silica gel column chromatography to afford the title compound (4.1g).
¹HNMR (MERCURY 300 MHz, CDCl₃) δ 7.93 (d, *J* = 8.7 Hz, 2H, 2ArH) 7.19 (d, *J* = 8.7 Hz, 2H, 2ArH) 6.98 (d, *J* = 8.7 Hz, 4H, 4ArH) 6.81 (brs, 1H, NH) 4.65 (s, 2H, 1CH₂) 4.28-4.20 (m, 4H, 2CH₂) 2.70 (dd, *J* = 11.4 Hz, 7.2Hz, 2H, 1CH₂) 2.50 (dd, *J* = 9.3 Hz, 5.1 Hz, 2H, 1CH₂) 2.04 (s, 3H, 1CH₃) 1.25 (t, *J* = 7.2 Hz, 6H, 2CH₃)
ESI(m/z) 490 (M+H⁺) 512 (M+Na⁺)

### (8-5) Preparation of diethyl 2-acetamido-2-(4-(4-(2-acetoxyacetyl)phenoxy) phenethyl)malonate

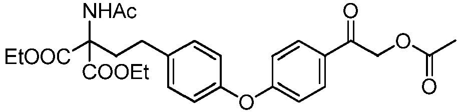

To a solution of diethyl diethyl 2-acetamido-2-(4-(4-(2-chloroacetyl) phenoxy)phenethyl) malonate (1.7 g, 3.4 mmol) in CH₃CN (17mL) was added acetic acid (0.47 g, 7.8 mmol) and Et₃N(0.72 g, 7.2 mmol). The mixture was heated to reflux for 2h, then concentrated. The residue was diluted with CH₂Cl₂, washed with H₂O to neutral, dried over Na₂SO₄, filtered and concentrated, yielding the crude product (1.62g) as yellow syrup.
¹HNMR (MERCURY 300 MHz, CDCl₃) δ 7.88 (d, *J* = 8.7 Hz, 2H, 2ArH) 7.18 (d, *J* = 8.7 Hz, 2H, 2ArH) 6.98 (d, *J* = 8.7 Hz, 4H, 4ArH) 6.80 (brs, 1H, NH) 5.30 (d, *J* = 1.5 Hz, 2H, CH₂) 4.28-4.20 (m, 4H, 2CH₂) 2.70 (dd, *J* = 11.4 Hz, 7.5 Hz, 2H, 1CH₂) 2.50 (dd, *J* = 9.6 Hz, 5.7 Hz, 2H, 1CH₂) 2.23 (s, 3H, 1CH₃) 2.03 (s, 3H, 1CH₃) 1.27 (t, *J* = 7.2 Hz, 6H, 2CH₃)
ESI(m/z) 514 (M+H⁺) 536 (M+Na⁺)

### (8-6) Preparation of diethyl 2-acetamido-2-(4-(4-(2-(propionyloxy)acetyl)phenoxy) phenethyl)malonate

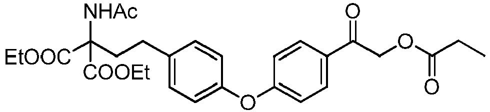

To a solution of diethyl diethyl 2-acetamido-2-(4-(4-(2-chloroacetyl) phenoxy)phenethyl) malonate (1.2 g, 2.4 mmol) in CH₃CN (12mL) was added propionic acid (0.41 g, 5.5 mmol) and Et₃N(0.51 g, 5.1 mmol). The mixture was heated to reflux for 2h, then concentrated. The residue was diluted with CH₂Cl₂, washed with H₂O to neutral, dried over Na₂SO₄, filtered and concentrated, yielding the crude product (1.1g) as yellow syrup.
¹HNMR (MERCURY 300 MHz, CDCl₃) δ 7.88 (d, *J* = 8.7 Hz, 2H, 2ArH) 7.18 (d, *J* = 8.7 Hz, 2H, 2ArH) 6.98 (d, *J* = 8.7 Hz, 4H, 4ArH) 6.80 (brs, 1H, NH) 5.30 (s, 2H, CH₂) 4.27-4.20 (m, 4H, 2CH₂) 2.70 (dd, *J* = 10.8 Hz, 6.9 Hz, 2H, 1CH₂) 2.56-2.46 (m, 4H, 2CH₂) 2.03 (s, 3H, 1CH₃) 1.29-1.19 (m, 9H, 3CH₃)
ESI(m/z) 528 (M+H⁺) 550 (M+Na⁺)

### (8-7) Preparation of diethyl 2-acetamido-2-(4-(4-(2-(butyryloxy)acetyl)phenoxy) phenethyl)malonate

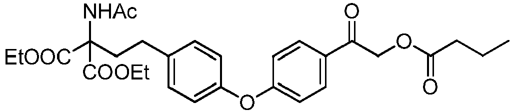

To a solution of diethyl diethyl 2-acetamido-2-(4-(4-(2-chloroacetyl) phenoxy)phenethyl) malonate (2.2 g, 4.1 mmol) in CH₃CN (20mL) was added *n*-butyric acid (0.83 g, 9.4 mmol) and Et₃N(0.88 g, 8.6 mmol). The mixture was heated to reflux for 2h, then concentrated. The residue was diluted with CH₂Cl₂, washed with H₂O to neutral, dried over Na₂SO₄, filtered and concentrated, yielding the crude product (1.4g) as yellow syrup.
¹HNMR (MERCURY 300 MHz, CDCl₃) δ 7.88 (d, *J* = 8.7 Hz, 2H, 2ArH) 7.18 (d, *J* = 8.7 Hz, 2H, 2ArH) 6.97 (d, *J* = 9.0 Hz, 4H, 4ArH) 6.81 (brs, 1H, NH) 5.29 (s, 2H, CH₂) 4.27-4.20 (m, 4H, 2CH₂) 2.70 (dd, *J* = 11.1 Hz, 7.2 Hz, 2H, 1CH₂) 2.52-2.44 (m, 4H, 2CH₂) 2.03 (s, 3H, 1CH₃) 1.77-1.70 (s, 2H, 1CH₂) 1.26 (t, *J* = 7.5 Hz, 6H, 2CH₃) 1.01 (t, *J =* 7.5 Hz, 3H, 1CH₃)
ESI(m/z) 542 (M+H⁺)

### (8-8) Preparation of diethyl 2-acetamido-2-(4-(4-(2-(isobutyryloxy)acetyl)phenoxy) phenethyl)malonate

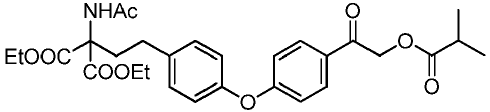

To a solution of diethyl diethyl 2-acetamido-2-(4-(4-(2-chloroacetyl) phenoxy)phenethyl) malonate (1.25 g, 2.5 mmol) in CH₃CN (13mL) was added isobutyric acid (0.51 g, 5.8 mmol) and Et₃N(0.54 g, 5.4 mmol). The mixture was heated to reflux for 2h, then concentrated. The residue was diluted with CH₂Cl₂, washed with H₂O to neutral, dried over Na₂SO₄, filtered and concentrated, yielding the crude product (1.3g) as yellow syrup.
¹HNMR (MERCURY 300 MHz, CDCl₃) δ 7.88 (d, *J* = 8.7 Hz, 2H, 2ArH) 7.18 (d, *J* = 8.7 Hz, 2H, 2ArH) 6.98 (d, *J* = 9.0 Hz, 4H, 4ArH) 6.80 (brs, 1H, NH) 5.28 (s, 2H, CH₂) 4.27-4.20 (m, 4H, 2CH₂) 2.76-2.67 (m, 3H, 1CH, 1CH₂) 2.49 (dd, *J* = 9.6 Hz, 5.1 Hz, 2H, 1CH₂) 2.03 (s, 3H, 1CH₃) 1.29-1.23 (m, 12H, 4CH₃)
ESI(m/z) 542 (M+H⁺) 564 (M+Na⁺)

### (8-9) Preparation of diethyl 2-acetamido-2-(4-(4-(2-((cyclopropanecarbonyl)oxy) acetyl)phenoxy)phenethyl)malonate

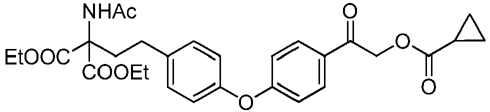

To a solution of diethyl diethyl 2-acetamido-2-(4-(4-(2-chloroacetyl) phenoxy)phenethyl) malonate (1.3 g, 2.7 mmol) in CH₃CN (11mL) was added cyclopropanecarboxylic acid (0.53 g, 6.2 mmol) and Et₃N(0.58 g, 5.7 mmol). The mixture was heated to reflux for 2h, then concentrated. The residue was diluted with CH₂Cl₂, washed with H₂O to neutral, dried over Na₂SO₄, filtered and concentrated, yielding the crude product (1.2g) as yellow syrup.
¹HNMR (MERCURY 300 MHz, CDCl₃) δ 7.88 (d, *J* = 6.9 Hz, 2H, 2ArH) 7.17 (d, *J* = 7.2 Hz, 2H, 2ArH) 6.97 (d, *J* = 7.2 Hz, 4H, 4ArH) 6.79 (brs, 1H, NH) 5.29 (s, 2H, CH₂) 4.23 (q, *J* = 7.2 Hz, 4H, 2CH₂) 2.70 (dd, *J* = 8.7 Hz, 7.2 Hz, 2H, 1CH₂) 2.50 (dd, *J=* 8.1 Hz, 6.9 Hz, 2H, 1CH₂) 2.02 (s, 3H, 1CH₃) 1.79-1.78 (m, 1H, 1CH) 1.26 (t, *J* = 7.2 Hz, 6H, 2CH₃) 1.03 (m, 2H, CH₂) 0.96 (m, 2H, CH₂)
ESI(m/z) 540 (M+H⁺)

### (8-10) Preparation of diethyl 2-acetamido-2-(4-(4-(2-methyloxazol-4-yl)phenoxy) phenethyl)malonate

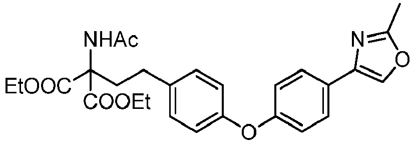

To a solution of diethyl 2-acetamido-2-(4-(4-(2-acetoxyacetyl)phenoxy) phenethyl)malonate (1.62 g, 3.15 mmol) in xylene(32 mL) was added acetamide (0.93 g, 15.8 mmol) and 47%BF₃·Et₂O(0.3mL). The mixture was heated to reflux for 40h, then concentrated. The residue was diluted with EtOAc, washed with H₂O (three times) and brine, dried over Na₂SO₄, filtered and concentrated, yielding the crude product (1.17 g) as light yellow solid.
¹HNMR (MERCURY 300 MHz, CDCl₃) δ 7.75 (s, 1H, ArH) 7.65 (d, *J* = 9.0 Hz, 2H, 2ArH) 7.12 (d, *J* = 8.4 Hz, 2H, 2ArH) 6.99 (d, *J* = 8.4 Hz, 2H, 2ArH) 6.94 (d, *J* = 8.4 Hz, 2H, 2ArH) 6.78 (brs, 1H, NH) 4.27-4.17 (m, 4H, 2CH₂) 2.69 (dd, *J* = 11.1 Hz, 6.9 Hz, 2H, 1CH₂) 2.51-2.44 (m, 5H, 1CH₃, 1CH₂) 2.02 (s, 3H, 1CH₃) 1.26 (t, *J* = 7.2 Hz, 6H, 2CH₃)
ESI(m/z) 495 (M+H⁺) 517 (M+Na⁺)

### (8-11) Preparation of diethyl 2-acetamido-2-(4-(4-(2-ethyloxazol-4-yl)phenoxy) phenethyl)malonate

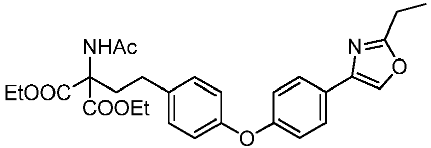

To a solution of diethyl 2-acetamido-2-(4-(4-(2-(propionyloxy)acetyl)phenoxy) phenethyl)malonate (1.1 g, 2.1 mmol) in xylene(32 mL) was added acetamide (0.6 g, 10.2 mmol) and 47%BF₃·Et₂O(0-19mL). The mixture was heated to reflux for 40h, then concentrated. The residue was diluted with EtOAc, washed with H₂O (three times) and brine, dried over Na₂SO₄, filtered and concentrated, yielding the crude product (0.85 g) as light yellow oil.
¹HNMR (MERCURY 300 MHz, CDCl₃) δ 7.76 (s, 1H, ArH) 7.66 (d, *J* = 8.4 Hz, 2H, 2ArH) 7.12 (d, *J* = 8.4 Hz, 2H, 2ArH) 6.99 (d, *J* = 8.7 Hz, 2H, 2ArH) 6.93 (d, *J* = 8.7 Hz, 2H, 2ArH) 6.79 (brs, 1H, NH) 4.25-4.18 (m, 4H, 2CH₂) 2.84 (q, *J* = 7.8 Hz, 2H, CH₂) 2.69 (dd, *J* = 11.1 Hz, 7.2 Hz, 2H, 1CH₂) 2.48 (dd, *J* = 14.1 Hz, 5.4 Hz, 2H, 1CH₂) 2.02 (s, 3H, 1CH₃) 1.37 (t, *J* = 7.5 Hz, 3H, 1CH₃) 1.26 (t, *J* = 7.2 Hz, 6H, 2CH₃)
ESI(m/z) 509 (M+H⁺) 531 (M+Na⁺)

### (8-12) Preparation of diethyl 2-acetamido-2-(4-(4-(2-propyloxazol-4-yl)phenoxy) phenethyl)malonate

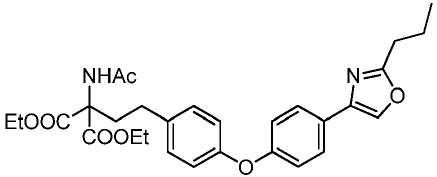

To a solution of diethyl 2-acetamido-2-(4-(4-(2-(butyryloxy)acetyl)phenoxy) phenethyl)malonate (1.4 g, 2.6 mmol) in xylene(15 mL) was added acetamide (0.76 g, 12.8 mmol) and 47%BF₃·Et₂O(0.07mL). The mixture was heated to reflux for 40h, then concentrated. The residue was diluted with EtOAc, washed with H₂O (three times) and brine, dried over Na₂SO₄, filtered and concentrated, yielding the crude product (1.0 g) as light yellow oil.
¹HNMR (MERCURY 300 MHz, CDCl₃) δ 7.76 (s, 1H, ArH) 7.67 (d, *J* = 7.8 Hz, 2H, 2ArH) 7.11 (d, *J* = 7.5 Hz, 2H, 2ArH) 6.98-6.92 (m, 4H, 4ArH) 6.80 (brs, 1H, NH) 4.25-4.20 (m, 4H, 2CH₂) 2.69 (dd, *J* = 11.1 Hz, 7.2 Hz, 2H, 1CH₂) 2.52-2.44 (m, 2H, 1CH₂) 2.03 (s, 3H, 1CH₃) 1.85-1.73 (m, 4H, 2CH₂) 1.26 (t, *J* = 7.2 Hz, 6H, 2CH₃) 1.03 (t, *J* = 7.2 Hz, 3H, 1CH₃)
ESI(m/z) 523 (M+H⁺)

### (8-13) Preparation of diethyl 2-acetamido-2-(4-(4-(2-isopropyloxazol-4-yl)phenoxy) phenethyl)malonate

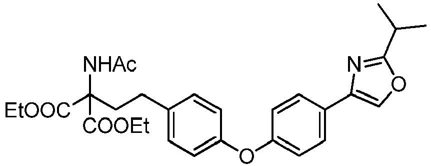

To a solution of diethyl 2-acetamido-2-(4-(4-(2-(isobutyryloxy) acetyl)phenoxy) phenethyl)malonate (1.3 g, 2.5 mmol) in xylene(25 mL) was added acetamide (0.73 g, 12.3 mmol) and 47%BF₃·Et₂O(0.23mL). The mixture was heated to reflux for 40h, then concentrated. The residue was diluted with EtOAc, washed with H₂O (three times) and brine, dried over Na₂SO₄, filtered and concentrated, yielding the crude product (1.0 g) as light yellow oil.
¹HNMR (MERCURY 300 MHz, CDCl₃) δ 7.75 (s, 1H, ArH) 7.67 (d, *J* = 8.4 Hz, 2H, 2ArH) 7.11 (d, *J* = 8.1 Hz, 2H, 2ArH) 6.99 (d, *J* = 8.4 Hz, 2H, 2ArH) 6.93 (d, *J* = 8.7 Hz, 2H, 2ArH) 6.78 (brs, 1H, NH) 4.26-4.17 (m, 4H, 2CH₂) 3.18-3.09 (m, 1H, CH) 2.69 (dd, *J* = 11.4 Hz, 6.9 Hz, 2H, 1CH₂) 2.47 (dd, *J* = 9.0 Hz, 5.1 Hz, 2H, 1CH₂) 2.02 (s, 3H, 1CH₃) 1.39 (s, 3H, 1CH₃) 1.37 (s, 3H, 1CH₃) 1.26 (t, *J* = 7.2 Hz, 6H, 2CH₃)
ESI(m/z) 523 (M+H⁺)

### (8-14) Preparation of diethyl 2-acetamido-2-(4-(4-(2-cyclopropyloxazol-4-yl)phenoxy) phenethyl)malonate

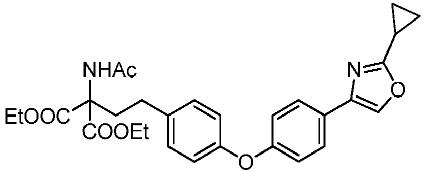

To a solution of diethyl 2-acetamido-2-(4-(4-(2-((cyclopropanecarbonyl)oxy) acetyl)phenoxy)phenethyl)malonate (1.2 g, 2.2 mmol) in xylene(20 mL) was added acetamide (0.65 g, 11 mmol) and 47%BF₃·Et₂O(0.2mL). The mixture was heated to reflux for 40h, then concentrated. The residue was diluted with EtOAc, washed with H₂O (three times) and brine, dried over Na₂SO₄, filtered and concentrated, yielding the crude product (0.4 g) as light yellow oil.
¹HNMR (MERCURY 300 MHz, CDCl₃) δ 7.68 (s, 1H, ArH) 7.66 (d, *J* = 8.4 Hz, 2H, 2ArH) 7.11 (d, *J* = 8.1 Hz, 2H, 2ArH) 6.99 (d, *J* = 8.7 Hz, 2H, 2ArH) 6.93 (d, *J* = 8.7 Hz, 2H, 2ArH) 6.79 (brs, 1H, NH) 4.29-4.19 (m, 4H, 2CH₂) 2.69 (dd, *J* = 10.5 Hz, 6.3 Hz, 2H, 1CH₂) 2.45 (dd, *J* = 8.7 Hz, 5.1 Hz, 2H, 1CH₂) 2.23-2.17 (m, 1H, 1CH) 2.02 (s, 3H, 1CH₃) 1.27 (t, *J* = 7.2 Hz, 6H, 2CH₃) 1.23-0.99 (m, 4H, 2CH₂)
ESI(m/z) 521 (M+H⁺)

### (8-15) Preparation of N-(1-hydroxy-2-(hydroxymethyl)-4-(4-(4-(2-methyloxazol-4-yl) phenoxy)phenyl)butan-2-yl)acetamide

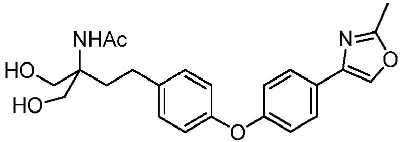

To a solution of diethyl 2-acetamido-2-(4-(4-(2-methyloxazol-4-yl)phenoxy) phenethyl)malonate (1.17 g, 2.4 mmol) in 95% EtOH (17mL) was added K₂HPO₄ (4.3 g, 18.7 mmol) in distilled water (4.3 mL) and NaBH₄ (0.46 g, 12.1 mmol) in aq. 10% NaOH(3.1mL). The mixture was stirred for further 6h at room temperature and concentrated. The residue was extracted with EtOAc, washed with H₂O to neutral, dried over Na₂SO₄, filtered and concentrated. The residue was recrystallized with EtOAc to afford the title compound (0.54 g) as white powder solid.
¹HNMR (MERCURY 300 MHz, CDCl₃) δ 7.75 (s, 1H, ArH) 7.65 (d, *J* = 8.7 Hz, 2H, 2ArH) 7.17 (d, *J* = 8.4 Hz, 2H, 2ArH) 6.99 (d, *J* = 8.4 Hz, 2H, 2ArH) 6.95 (d, *J* = 8.4 Hz, 2H, 2ArH) 5.95 (brs, 1H, NH) 3.86 (d, *J* = 11.4 Hz, 2H, CH₂) 3.64 (d, *J* = 11.7 Hz, 2H, CH₂) 2.63 (dd, *J* = 11.4 Hz, 8.1 Hz, 2H, 1CH₂) 2.51 (s, 3H, 1CH₃) 2.04-1.94 (m, 5H, 1CH₂, 1CH₃)
ESI(m/z) 411 (M+H⁺)

### (8-16) Preparation of N-(4-(4-(4-(2-ethyloxazol-4-yl)phenoxy)phenyl)-1-hydroxy -2-(hydroxymethyl)butan-2-yl)acetamide

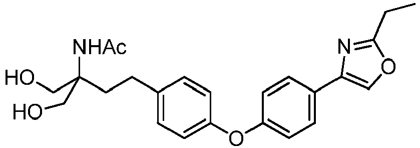

To a solution of diethyl 2-acetamido-2-(4-(4-(2-ethyloxazol-4-yl)phenoxy) phenethyl)malonate(0.85 g, 1.7 mmol) in 95% EtOH (12mL) was added K₂HPO₄ (3 g, 13.2 mmol) in distilled water (3 mL) and NaBH₄ (0.33 g, 8.6 mmol) in aq. 10% NaOH(2.2 mL). The mixture was stirred for further 6h at room temperature and concentrated. The residue was extracted with EtOAc, washed with H₂O to neutral, dried over Na₂SO₄, filtered and concentrated. The residue was purified by silica gel column chromatography to afford the title compound (0.47g) as white powder solid.
¹HNMR (MERCURY 300 MHz, CDCl₃) δ 7.75 (s, 1H, ArH) 7.66 (d, *J* = 8.7 Hz, 2H, 2ArH) 7.17 (d, *J* = 8.4 Hz, 2H, 2ArH) 6.99 (d, *J* = 8.7 Hz, 2H, 2ArH) 6.95 (d, *J* = 8.4 Hz, 2H, 2ArH) 5.95 (brs, 1H, NH) 3.86 (d, *J* = 11.4 Hz, 2H, CH₂) 3.64 (d, *J* = 11.4 Hz, 2H, CH₂) 2.84 (q, *J* = 7.8 Hz, 2H, CH₂) 2.63 (dd, *J* = 11.4 Hz, 8.1 Hz, 2H, 1CH₂) 2.00-1.94 (m, 5H, 1CH₂, 1CH₃) 1.37 (t, *J* = 7.5 Hz, 3H, 1CH₃)
ESI(m/z) 425 (M+H⁺)

### (8-17) Preparation of N-(1-hydroxy-2-(hydroxymethyl)-4-(4-(4-(2-propyloxazol-4-yl) phenoxy)phenyl)butan-2-yl)acetamide

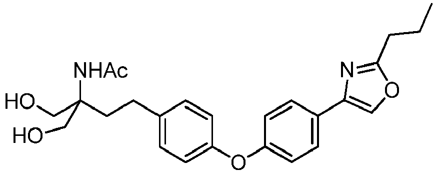

To a solution of diethyl 2-acetamido-2-(4-(4-(2-propyloxazol-4-yl)phenoxy) phenethyl)malonate (1.0 g, 1.9 mmol) in 95% EtOH (14mL) was added K₂HPO₄ (3.5 g, 15.1 mmol) in distilled water (3.5 mL) and NaBH₄ (0.37 g, 9.8 mmol) in aq. 10% NaOH(2.5 mL). The mixture was stirred for further 6h at room temperature and concentrated. The residue was extracted with EtOAc, washed with H₂O to neutral, dried over Na₂SO₄, filtered and concentrated. The residue was purified by silica gel column chromatography to afford the title compound (0.68g) as white powder solid.
¹HNMR (MERCURY 300 MHz, CDCl₃) δ 7.76 (s, 1H, 1ArH) 7.67 (d, *J=* 8.7 Hz, 2H, 2ArH) 7.16 (d, *J* = 8.4 Hz, 2H, 2ArH) 7.01 (d, *J* = 8.4 Hz, 2H, 2ArH) 6.99 (d, *J* = 9.0 Hz, 2H, 2ArH) 6.96 (brs, 1H, NH) 3.87 (d, *J* = 11.4 Hz, 2H, CH₂) 3.64 (d, *J* = 11.4 Hz, 2H, CH₂) 2.80 (t, *J* = 7.2 Hz, 2H, CH₂) 2.63 (dd, *J* = 11.4 Hz, 8.1 Hz, 2H, 1CH₂) 2.00-1.94 (m, 5H, 1CH₂, 1CH₃) 1.85-1.80 (m, 2H, 1CH₂) 1.02 (t, *J* = 7.2 Hz, 3H, 1CH₃)
ESI(m/z) 439 (M+H⁺) 474 (M+Na⁺)

### (8-18) Preparation of N-(1-hydroxy-2-(hydroxymethyl)-4-(4-(4-(2-isopropyloxazol -4-yl)phenoxy)phenyl)butan-2-yl)acetamide

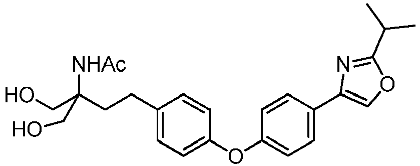

To a solution of diethyl 2-acetamido-2-(4-(4-(2-isopropyloxazol-4-yl)phenoxy) phenethyl)malonate (1.0 g, 1.9 mmol) in 95% EtOH (13.5mL) was added K₂HPO₄ (3.5 g, 15.1 mmol) in distilled water (3.5 mL) and NaBH₄ (0.37 g, 9.8 mmol) in aq. 10% NaOH(2.5 mL). The mixture was stirred for further 6h at room temperature and concentrated. The residue was extracted with EtOAc, washed with H₂O to neutral, dried over Na₂SO₄, filtered and concentrated. The residue was purified by silica gel column chromatography to afford the title compound (0.57g) as yellow oil.
¹HNMR (MERCURY 300 MHz, CDCl₃) δ 7.75 (s, 1H, ArH) 7.67 (d, *J* = 8.7 Hz, 2H, 2ArH) 7.17 (d, *J* = 8.4 Hz, 2H, 2ArH) 6.99 (d, *J* = 8.7 Hz, 2H, 2ArH) 6.95 (d, *J* = 8.4 Hz, 2H, 2ArH) 5.91 (brs, 1H, NH) 3.88 (d, *J* = 11.4 Hz, 2H, CH₂) 3.64 (d, *J=* 11.7 Hz, 2H, CH₂) 3.19-3.09 (m, 1H, CH) 2.63 (dd, *J* = 11.1 Hz, 8.1 Hz, 2H, 1CH₂) 2.01-1.94 (m, 5H, 1CH₂, 1CH₃) 1.40 (s, 3H, 1CH₃) 1.37 (s, 3H, 1CH₃)
ESI(m/z) 439 (M+H⁺)

### (8-19) Preparation of N-(4-(4-(4-(2-cyclopropyloxazol-4-yl)phenoxy)phenyl) -1-hydroxy-2-(hydroxymethyl)butan-2-yl)acetamide

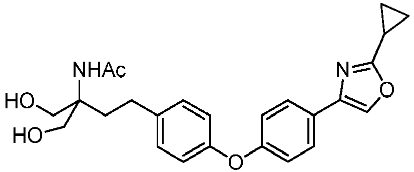

To a solution of diethyl diethyl 2-acetamido-2-(4-(4-(2-cyclopropyloxazol-4-yl)phenoxy) phenethyl)malonate (0.4 g, 0.75 mmol) in 95% EtOH (5.2 mL) was added K₂HPO₄ (1.4 g, 5.9 mmol) in distilled water (1.4 mL) and NaBH₄ (0.15 g, 3.8 mmol) in aq. 10% NaOH(1 mL). The mixture was stirred for further 6h at room temperature and concentrated. The residue was extracted with EtOAc, washed with H₂O to neutral, dried over Na₂SO₄, filtered and concentrated. The residue was purified by silica gel column chromatography to afford the title compound (0.15g) as white powder solid.
¹HNMR (MERCURY 300 MHz, CDCl₃) δ 7.70 (s, 1H, ArH) 7.69 (d, *J* = 8.4 Hz, 2H, 2ArH) 7.18 (d, *J* = 8.1 Hz, 2H, 2ArH) 7.00 (d, *J* = 8.4 Hz, 2H, 2ArH) 6.96 (d, *J* = 7.5 Hz, 2H, 2ArH) 5.95 (brs, 1H, NH) 3.88 (d, *J* = 11.7 Hz, 2H, CH₂) 3.65 (d, *J* = 11.7 Hz, 2H, CH₂) 2.62 (t, *J* = 8.7 Hz, 2H, 1CH₂) 2.23-2.17 (m, 1H, CH) 2.05-1.95 (m, 5H, 1CH₂, 1CH₃) 1.28-1.13 (m, 4H, 2CH₂)
ESI(m/z) 437 (M+H⁺)

### (8-20) Preparation of 2-amino-2-(4-(4-(2-methyloxazol-4-yl)phenoxy)phenethyl) propane-1,3-diol hydrochloride

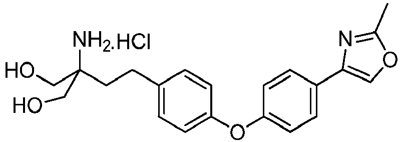

To a solution of *N*-(1-hydroxy-2-(hydroxymethyl)-4-(4-(4-(2-methyloxazol-4-yl) phenoxy)phenyl)butan-2-yl)acetamide (0.53 g, 1.26 mmol) in MeOH(10mL) was added NaOH(0.053 g, 1.3 mmol) and heated to reflux for 8h, then filtered to remove insoluble substance. The solution was added HCl-EtOH solution until pH=3-4 and concentrated. The residue was recrystallized with isopropanol to afford the title compound (0.27 g) as white solid.
¹HNMR (MERCURY 300 MHz, CD₃OD) δ 8.03 (s, 1H, ArH) 7.63 (d, *J=* 8.7 Hz, 2H, 2ArH) 7.22 (d, *J* = 8.1 Hz, 2H, 2ArH) 6.92 (d, *J* = 8.1 Hz, 2H, 2ArH) 6.91 (d, *J* = 8.1 Hz, 2H, 2ArH) 3.65 (s, 4H, 2CH₂) 2.66-2.60 (m, 2H, CH₂) 2.44 (s, 3H, 1CH₃) 1.95-1.89 (m, 2H, CH₂)
¹³CNMR (400MHz, CD₃OD) δ 163.95, 158.99, 156.64, 141.20, 137.93, 134.96, 130.79, 128.00, 127.18, 120.39, 119.58, 62.51, 62.06, 34.76, 29.39, 13.55
ESI(m/z) 369 (M+H⁺) HRMS calcd. for C₂₁H₂₅N₂O₄(M+H⁺) 369.1808, found 369.1814

### (8-21) Preparation of 2-amino-2-(4-(4-(2-ethyloxazol-4-yl)phenoxy)phenethyl) propane-1,3-diol hydrochloride

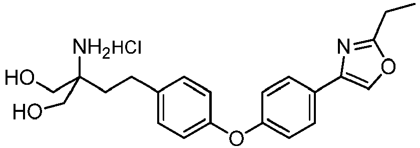

To a solution of *N*-(4-(4-(4-(2-ethyloxazol-4-yl)phenoxy)phenyl)-1-hydroxy -2-(hydroxymethyl)butan-2-yl)acetamide (0.46 g, 1.1 mmol) in MeOH(10mL) was added NaOH(0.045 g, 1.2 mmol) and heated to reflux for 8h, then filtered to remove insoluble substance. The solution was added HCl-EtOH solution until pH=3-4 and concentrated. The residue was recrystallized with isopropanol to afford the title compound (0.4 g) as white solid.
¹HNMR (MERCURY 300 MHz, CD₃OD) δ 8.37 (s, 1H, ArH) 7.68 (d, *J=* 8.4 Hz, 2H, 2ArH) 7.27 (d, *J* = 8.1 Hz, 2H, 2ArH) 7.02 (d, *J* = 8.4 Hz, 2H, 2ArH) 6.96 (d, *J* = 7.8 Hz, 2H, 2ArH) 3.68 (s, 4H, 2CH₂) 3.04 (q, 2H, 1CH₂) 2.69-2.64 (m, 2H, CH₂) 1.98-1.92 (m, 2H, CH₂) 1.41 (t, *J* = 7.8 Hz, 3H, CH₃)
¹³CNMR (400 MHz, CD₃OD) δ 169.75, 160.19, 156.05, 138.41, 137.97, 136.47, 130.93, 128.69, 123.33, 120.76, 119.54, 62.50, 62.03, 34.71, 29.41, 22.26, 10.65 ESI(m/z) 383 (M+H⁺) HRMS calcd. for C₂₂H₂₇N₂O₄(M+H⁺) 383.1965, found 383.1971

### (8-22) Preparation of 2-amino-2-(4-(4-(2-propyloxazol-4-yl)phenoxy)phenethyl) propane-1,3-diol hydrochloride

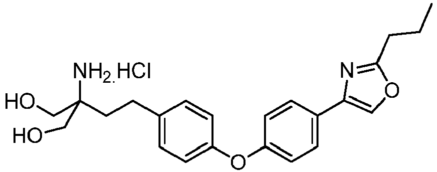

To a solution of *N*-(4-(4-(4-(2-propyloxazol-4-yl)phenoxy)phenyl)-1-hydroxy -2-(hydroxymethyl)butan-2-yl)acetamide (0.68 g, 1.6 mmol) in MeOH(10mL) was added NaOH(0.064 g, 1.6 mmol) and heated to reflux for 8h, then filtered to remove insoluble substance. The solution was added HCl-EtOH solution until pH=3-4 and concentrated. The residue was purified by silica gel column chromatography to afford the title compound (0.32 g) as white solid.
¹HNMR (MERCURY 300 MHz, CD₃OD) δ 8.20 (s, 1H, 1ArH) 7.60 (d, *J* = 8.4 Hz, 2H, 2ArH) 7.19 (d, *J* = 8.1 Hz, 2H, 2ArH) 6.92 (d, *J* = 8.7 Hz, 2H, 2ArH) 6.88 (d, *J* = 8.7 Hz, 2H, 2ArH) 3.61 (s, 4H, 2CH₂) 2.84 (t, *J* = 7.5 Hz, 2H, 1CH₂) 2.62-2.56 (m, 2H, 1CH₂) 1.91-1.81 (m, 2H, 1CH₂) 1.79-1.74 (m, 2H, 1CH₂) 0.95 (t, *J* = 7.2 Hz, 3H, 1CH₃)
¹³CNMR (400 MHz, CD₃OD) δ 168.24, 159.75, 156.27, 139.11, 138.25, 135.88, 130.88, 128.44, 124.78, 120.63, 119.55, 62.49, 62.05, 34.73, 30.45, 29.40, 21.19, 13.85
ESI (m/z) 397 (M+H⁺) HRMS calcd. for C₂₃H₂₉N₂O₄ (M+H⁺) 397.2122, found 397.2119

### (8-23) Preparation of 2-amino-2-(4-(4-(2-isopropyloxazol-4-yl)phenoxy)phenethyl) propane-1,3-diol

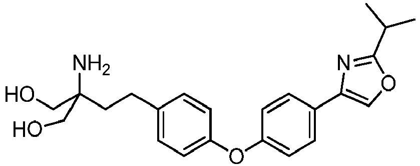

To a solution of *N*-(4-(4-(4-(2-isopropyloxazol-4-yl)phenoxy)phenyl)-1-hydroxy -2-(hydroxymethyl)butan-2-yl)acetamide (0.56 g, 1.2 mmol) in MeOH(10mL) was added NaOH(0.053 g, 1.3 mmol) and heated to reflux for 8h, then filtered to remove insoluble substance. The solution was added HCl-EtOH solution until pH=3-4 and concentrated. The residue was purified by silica gel column chromatography to afford the title compound (0.35 g) as white solid.
¹HNMR (MERCURY 300 MHz, CD₃OD) δ 8.00 (s, 1H, ArH) 7.63 (d, *J=* 9.0 Hz, 2H, 2ArH) 7.18 (d, *J* = 8.4 Hz, 2H, 2ArH) 6.91 (d, *J* = 9.3 Hz, 2H, 2ArH) 6.87 (d, *J* = 8.4 Hz, 2H, 2ArH) 3.48 (d, *J* = 10.8 Hz, 2H, CH₂) 3.42 (d, *J* = 11.1 Hz, 2H, CH₂) 3.11-3.06 (m, 1H, CH) 2.63-2.57 (m, 2H, 1CH₂) 1.67-1.61 (m, 2H, 1CH₂) 1.32 (s, 3H, 1CH₃) 1.30 (s, 3H, 1CH₃)
¹³CNMR (400 MHz, CD₃OD) δ 171.08, 159.15, 156.22, 141.11, 139.52, 134.51, 130.78, 128.07, 127.26, 120.35, 119.39, 66.15, 57.27, 37.51, 29.77, 29.70, 20.74 ESI(m/z) 397 (M+H⁺) HRMS calcd. for C₂₃H₂₉N₂O₄(M+H⁺) 397.2121, found 397.2128

### (8-24) Preparation of 2-amino-2-(4-(4-(2-cyclopropyloxazol-4-yl)phenoxy) phenethyl)propane-1,3-diol hydrochloride

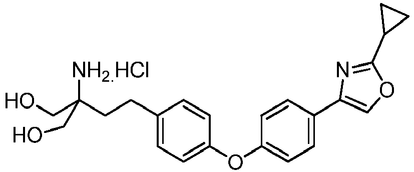

To a solution of *N*-(4-(4-(4-(2-cyclopropyloxazol-4-yl)phenoxy)phenyl)-1-hydroxy -2-(hydroxymethyl)butan-2-yl)acetamide (0.1 g, 0.23 mmol) in MeOH(10mL) was added NaOH(0.01 g, 0.24 mmol) and heated to reflux for 8h, then filtered to remove insoluble substance. The solution was added HCl-EtOH solution until pH=3-4 and concentrated. The residue was purified by silica gel column chromatography to afford the title compound (0.074 g) as white solid.
¹HNMR (MERCURY 300 MHz, CD₃OD) δ 8.19 (s, 1H, ArH) 7.58 (d, *J* = 8.4 Hz, 2H, 2ArH) 7.19 (d, *J* = 8.4Hz, 2H, 2ArH) 6.92 (d, *J* = 8.7 Hz, 2H, 2ArH) 6.87 (d, J = 8.7 Hz, 2H, 2ArH) 3.60 (s, 4H, 2CH₂) 2.62-2.56 (m, 2H, CH₂) 2.29-2.25 (m, 1H, CH) 1.90-1.84 (m, 2H, 1CH₂) 1.27-1.24 (m, 4H, 2CH₂)
¹³CNMR (400 MHz, CD₃OD) δ 170.16, 160.34, 156.01, 138.52, 135.73, 130.97, 128.70, 122.81, 120.81, 119.54, 62.51, 62.07, 34.73, 29.43, 10.58, 9.46
ESI(m/z) 395 (M+H⁺) HRMS calcd. for C₂₃H₂₇N₂O₄ (M+H⁺) 395.1965, found 395.1946

### EXAMPLE 9

This example described the preparation of

| | |
|---|---|
| 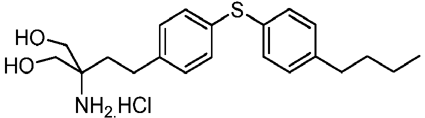 | 2-amino-2-(4-((4-butylphenyl)thio)phenethyl)pro pane-1,3-diol hydrochloride |
| 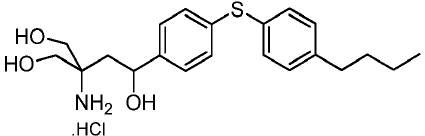 | 3-amino-1-(4-((4-butylphenyl)thio)phenyl)-3-(hy |
| | droxymethyl)butane-1,4-diol hydrochloride |

### (9-1) Preparation of 1-(4-(phenylthio)phenyl)butan-1-one

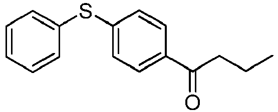

A solution of *n*-butyric acid (10 g, 113.5 mmol) in PCl₃(6.22 g, 45.4 mmol) was heated to 50-60°C for 3h. The supernatant was poured and the residue was washed with CH₂Cl₂. The combined organic layer was placed in three-necked bottle. To the solution diphenyl sulfide (20.1 g, 108.2 mmol)was added at 0°C, then AlCl₃ (15.9 g, 119.1 mmol) was added in portions. The solution was allowed to return to room temperature and stirred for further 3 h, then poured slowly into HCl-ice mixture. The organic layer was separated and the aqueous phase was extracted with CH₂Cl₂ three times. The combined organic layers were washed with H₂O to neutral, dried over Na₂SO₄, filtered and concentrated, yielding the crude product (21.2 g) as colorless oil.
¹HNMR (MERCURY 300 MHz CDCl₃) δ 7.83 (d, *J* = 8.7 Hz, 2H, 2ArH) 7.51-7.47 (m, 2H, 2ArH) 7.41-7.38 (m, 3H, 3ArH) 7.22 (d, *J* = 8.4 Hz, 2H, 2ArH) 2.88 (t, *J* = 7.5 Hz, 2H, 1CH₂) 1.80-1.68 (m, 2H, 1CH₂) 0.98 (t, *J* = 7.2 Hz, 3H, 1CH₃)
ESI(m/z) 257 (M+H⁺) 279(M+Na⁺)

### (9-2) Preparation of (4-butylphenyl)(phenyl)sulfane

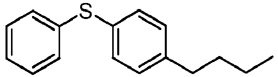

To a solution of 1-(4-(phenylthio)phenyl)butan-1-one (21.2 g, 82.8 mmol) in dry THF (200 mL) was added AlCl₃(30.9 g, 231.9 mmol) and NaBH₄(16.1 g, 422.3 mmol) at 0°C. The mixture was heated to reflux for 3 h, then was decomposed by ice water. The organic layer was separated and the aqueous phase was extracted with EA three times. The combined organic layers were washed with H₂O to neutral, dried over Na₂SO₄, filtered and concentrated, yielding crude product as colorless oil. The product was used in the next step without purification.
ESI(m/z) 243 (M+H⁺)

### (9-3) Preparation of 1-(4-((4-butylphenyl)thio)phenyl)-2-chloroethanone

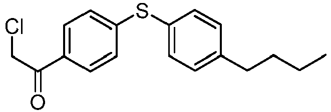

Chloroacetyl chloride (9.7 g, 85.9 mmol) was added dropwise to a cooled solution (0°C) of (4-butylphenyl)(phenyl)sulfane (19.8 g, 81.8 mmol) in dry CH₂Cl₂ (200mL), then AlCl₃ (12.1 g, 89.9 mmol) was added in portions. The solution was allowed to return to room temperature and stirred for further 3 h, then poured slowly into HCl-ice mixture. The organic layer was separated and the aqueous phase was extracted with CH₂Cl₂ three times. The combined organic layers were washed with H₂O to neutral, dried over Na₂SO₄, filtered and concentrated. The crude product was used in the next step without purification.

### (9-4) Preparation of diethyl 2-acetamido-2-(2-(4-((4-butylphenyl)thio)phenyl) -2-oxoethyl)malonate

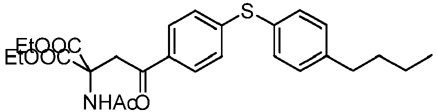

NaH (3.4 g, 98.2 mmol) was added to dry THF (200mL) at room temperature. After 30 min, diethyl acetamidomalonate (22.2 g, 102.3 mmol) was added in portions and stirred for further 5h. Then a solution of 1-(4-((4-butylphenyl)thio)phenyl)-2-chloroethanone (26.0 g, 81.8 mmol) in THF was added. The mixture was heated to reflux for further 12h and concentrated. The residue was diluted with EtOAc (20mL), washed with 1NHCl, aq. saturated NaHCO₃, brine, dried over Na₂SO₄, filtered and concentrated. The residue was purified by silica gel column chromatography to afford the title compound (10.0g) as yellow syrup.
¹HNMR (MERCURY 300 MHz, CDCl₃) δ 7.79 (d, *J* = 8.4 Hz, 2H, 2ArH) 7.42 (d, *J* = 8.1 Hz, 2H, 2ArH) 7.23 (d, *J* = 8.1 Hz, 2H, 2ArH) 7.14 (d, *J* = 8.4 Hz, 2H, 2ArH) 7.08 (brs, 1H, NH) 4.25 (q, *J* = 6.9 Hz, 4H, 2CH₂) 4.18 (s, 2H, CH₂) 2.65 (t, *J* = 8.1 Hz, 2H, 1CH₂) 1.95 (s, 3H, CH₃) 1.67-1.57 (m, 2H, 1CH₂) 1.41-1.28 (m, 2H, 1CH₂) 1.23 (t, *J* = 6.9 Hz, 6H, 2CH₃) 0.94 (t, *J* = 7.2 Hz, 3H, 1CH₃)
ESI(m/z) 500 (M+H⁺)

### (9-5) Preparation of N-(4-(4-((4-butylphenyl)thio)phenyl)-1,4-dihydroxy -2-(hydroxymethyl)butan-2-yl)acetamide

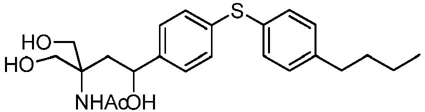

To a solution of diethyl 2-acetamido-2-(2-(4-((4-butylphenyl)thio)phenyl) -2-oxoethyl)malonate (2.0 g, 4.0 mmol) in 95% EtOH (28mL) was added K₂HPO₄ (7.2 g, 18.8 mmol) in distilled water (7.2 mL) and NaBH₄ (0.78 g, 20.5 mmol) in aq. 10% NaOH(5.3 mL). The mixture was stirred for further 6h at room temperature and concentrated. The residue was extracted with EtOAc, washed with H₂O to neutral, dried over Na₂SO₄, filtered and concentrated. The residue was recrystallized with EtOAc to afford the title compound (0.9 g) as white powder solid.
¹HNMR (MERCURY 300 MHz, CDCl₃) δ 7.30-7.23 (m, 6H, 6ArH) 7.14 (d, *J* = 8.1 Hz,
2H, 2ArH) 6.90 (brs, 1H, 1NH) 4.85 (d, *J* = 10.2 Hz, 1H, CH) 3.76 (d, *J* = 12.0 Hz, 1H, CH₂) 3.70 (d, *J* = 12.3 Hz, 1H, CH₂) 3.57 (d, *J* = 12Hz, 1H, CH₂) 3.46 (d, *J* = 12 Hz, 1H, CH₂) 2.60 (t, *J* = 7.5 Hz, 2H, 1CH₂) 2.32 (d, *J* = 15.3 Hz, 1H, CH₂) 2.02 (s, 3H, CH₃) 1.82 (dd, *J* = 15.3 Hz, 10.5Hz, 1H, CH₂) 1.64-1.54 (m, 2H, CH₂) 1.41-1.25 (m, 2H, CH₂) 0.93 (t, *J =* 7.5 Hz, 3H, CH₃)
ESI(m/z) 400 (M+H⁺)

### (9-6) Preparation of 3-amino-1-(4-((4-butylphenyl)thio)phenyl)-3-(hydroxymethyl) butane-1,4-diol hydrochloride

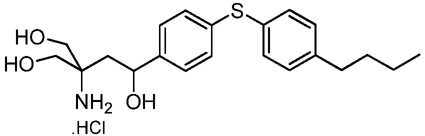

To a solution of *N*-(4-(4-((4-butylphenyl)thio)phenyl)-1,4-dihydroxy -2-(hydroxymethyl)butan-2-yl)acetamide (0.8 g, 1.9 mmol) in MeOH(10mL) was added NaOH(0.08 g, 2.1 mmol) and heated to reflux for 2h, then filtered to remove insoluble substance. The solution was added HCl-EtOH solution until pH=3-4 and concentrated. The residue was recrystallized with isopropanol to afford the title compound (0.55 g) as white solid.
¹HNMR (MERCURY 300 MHz, CD₃OD) δ 7.24 (d, *J* = 8.4 Hz, 2H, 2ArH) 7.15 (d, *J* = 8.1 Hz, 2H, 2ArH) 7.14 (d, *J* = 7.8 Hz, 2H, 2ArH) 7.06 (d, *J* = 8.1 Hz, 2H, 2ArH) 4.91 (d, *J* = 11.1 Hz, 1H, CH) 3.76 (dd, *J* = 15.3 Hz, 12.3 Hz, 2H, CH₂) 3.62 (d, *J* = 11.4 Hz, 1H, CH₂) 3.53 (d, *J* = 11.4Hz, 1H, CH₂) 2.51 (t, *J* = 7.8Hz, 2H, 1CH₂) 1.86-1.79 (m, 2H, 1CH₂) 1.52-1.47 (m, 2H, 1CH₂) 1.30-1.22 (m, 2H, 1CH₂) 0.84 (t, *J* = 7.2 Hz, 3H, 1CH₃)
¹³CNMR (400 MHz, CD₃OD) δ 145.15, 143.93, 137.40, 133.10, 133.06, 131.22, 130.49, 126.61, 70.58, 63.94, 62.29, 61.56, 40.95, 36.15, 34.77, 23.30, 14.23 ESI(m/z) 376 (M+H⁺) HRMS calcd. for C₂₁H₃₀NO₃S (M+H⁺) 376.1941, found 376.1925

### (9-7) Preparation of diethyl 2-acetamido-2-(4-((4-butylphenyl)thio) phenethyl)malonate

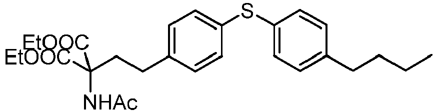

A solution of diethyl diethyl 2-acetamido-2-(2-(4-((4-butylphenyl)thio)phenyl) -2-oxoethyl)malonate (2.9 g, 5.8 mmol) in CH₂Cl₂ (9mL) was added dropwise to a solution of Et₃SiH (2.6 g, 22.2 mmol mmol) in CH₂Cl₂ (27 mL) at room temperature under N₂ protection. TiCl₄ (4.2 g, 22.2 mmol) was added to the solution with a syringe and the reaction mixture was stirred overnight, then poured slowly into HCl-ice mixture. The organic layer was separated and the aqueous phase was extracted with CH₂Cl₂ three times. The combined organic layers were washed with H₂O to neutral, dried over Na₂SO₄, filtered and concentrated, yielding crude product as light yellow oil. The crude product was used in the next step without purification.

### (9-8) Preparation of N-(4-(4-((4-butylphenyl)thio)phenyl)-1-hydroxy-2-(hydroxymethyl)butan-2-yl)acetamide

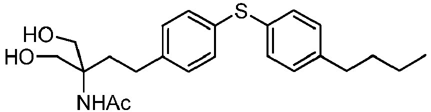

To a solution of diethyl 2-acetamido-2-(4-((4-butylphenyl)thio) phenethyl)malonate (2.8 g, 5.8 mmol) in 95% EtOH (41mL) was added K₂HPO₄ (10.5 g, 46.0 mmol) in distilled water (10.5 mL) and NaBH₄ (1.1 g, 29.8 mmol) in aq. 10% NaOH(7.7 mL). The mixture was stirred for further 6h at room temperature and concentrated. The residue was extracted with EtOAc, washed with H₂O to neutral, dried over Na₂SO₄, filtered and concentrated. The residue was purified by silica gel column chromatography to afford the title compound (1.08 g) as yellow oil.
¹HNMR (MERCURY 300 MHz, CDCl₃) δ 7.23 (d, *J* = 7.2 Hz, 4H, 4ArH) 7.11 (d, *J* = 7.8 Hz, 4H, 4ArH) 5.92 (brs, 1H, NH) 3.84 (d, *J* = 11.4 Hz, 2H, CH₂) 3.62 (d, J = 11.7 Hz, 2H, CH₂) 2.63-2.55 (m, 4H, 2CH₂) 1.97-1.92 (m, 5H, 1CH₂, 1CH₃) 1.62-1.52 (m, 2H, CH₂) 1.38-1.30 (m, 2H, CH₂) 0.91 (t, *J* = 7.5 Hz, 3H, CH₃)
ESI(m/z) 402 (M+H⁺)

### (9-9) Preparation of 2-amino-2-(4-((4-butylphenyl)thio)phenethyl)propane-1,3-diol hydrochloride

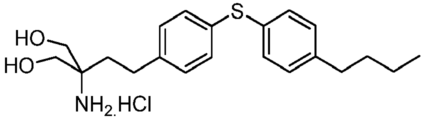

To a solution of *N*-(4-(4-((4-butylphenyl)thio)phenyl)-1-hydroxy-2-(hydroxymethyl)butan-2-yl)acetamide (1.1 g, 2.7 mmol) in MeOH(10mL) was added NaOH(0.1 g, 2.8 mmol) and heated to reflux for 2h, then filtered to remove insoluble substance. The solution was added HCl-EtOH solution until pH=3-4 and concentrated. The residue was recrystallized with isopropanol to afford the title compound (0.9 g) as white solid.
¹HNMR (MERCURY 300 MHz, CD₃OD) δ 7.17-7.11 (m, 6H, 6ArH) 7.08 (d, *J* = 8.4 Hz, 2H, 2ArH) 3.62 (s, 4H, 2CH₂) 2.62-2.51 (m, 4H, 2CH₂) 1.90-1.85 (m, 2H, 1CH₂) 1.55-1.47 (m, 2H, 1CH₂) 1.33-1.25 (m, 2H, CH₂) 0.87 (t, *J* = 6.9 Hz, 3H, CH₃) ¹³CNMR (400 MHz, CD₃OD) δ 143.64, 141.41, 135.55, 133.65, 132.59, 131.89, 130.42, 130.21, 62.48, 62.02, 36.15, 34.80, 34.50, 29.63, 23.31, 14.23
ESI(m/z) 360 (M+H⁺) HRMS calcd. for C₂₁H₃₀NO₂S (M+H⁺) 360.1992, found 360.1988

### EXAMPLE 10

This example described the preparation of

| | |
|---|---|
| 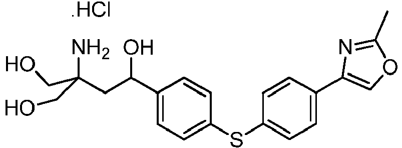 | 3-amino-3-(hydroxymethyl)-1-(4-((4-(2-methy loxazol-4-yl)phenyl)thio)phenyl)butane-1,4-diol hydrochloride |
| 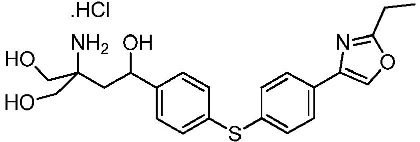 | 3-amino-1-(4-((4-(2-ethyloxazol-4-yl)phenyl )thio)phenyl)-3-(hydroxymethyl)butane-1,4-diol hydrochloride |
| 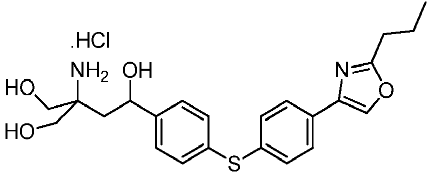 | 3-amino-3-(hydroxymethyl)-1-(4-((4-(2-pr opyloxazol-4-yl)phenyl)thio)phenyl)butane-1,4-diol hydrochloride |
| 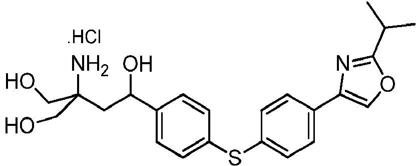 | 3-amino-3-(hydroxymethyl)-1-(4-((4-(2-isop ropyloxazol-4-yl)phenyl)thio)phenyl)butane-1,4-diol hydrochloride |
| 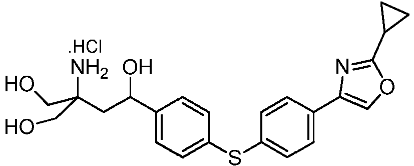 | 3-amino-1-(4-((4-(2-cyclopropyloxazol-4-yl) phenyl)thio)phenyl)-3-(hydroxymethyl)butane-1,4-diol hydrochloride |

### (10-1) Preparation of diethyl 2-acetamido-2-(2-(4-((4-(2-chloroacetyl)phenyl)thio) phenyl)-2-oxoethyl)malonate

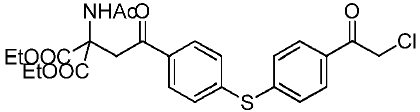

Chloroacetyl chloride (2.2 g, 19.4 mmol) in was added dropwise to a cooled solution (0°C) of diethyl 2-acetamido-2-(2-oxo-2-(4-(phenylthio)phenyl)ethyl) malonate (8.2 g, 18.5 mmol) in dry CH₂Cl₂(200mL), then AlCl₃ (13.6 g, 101.8 mmol) was added in portions. The solution was allowed to return to room temperature and stirred for further 5 h, then poured slowly into HCl-ice mixture. The organic layer was separated and the aqueous phase was extracted with CH₂Cl₂ three times. The combined organic layers were washed with H₂O to neutral, dried over Na₂SO₄, filtered and concentrated, yielding the crude compound (8.9g) as yellow syrup.
¹HNMR (MERCURY 300 MHz, CDCl₃) δ 7.92 (d, *J* = 8.4 Hz, 2H, 2ArH) 7.90 (d, *J* = 8.4 Hz, 2H, 2ArH) 7.43 (d, *J* = 8.7 Hz, 2H, 2ArH) 7.41 (d, *J* = 8.4 Hz, 2H, 2ArH) 7.11 (brs, 1H, 1NH) 5.30 (s, 2H, 1CH₂) 4.66 (s, 2H, 1CH₂) 4.30-4.19 (m, 4H, 2CH₂) 1.98 (s, 3H, CH₃) 1.23 (t, *J* = 7.5 Hz, 6H, 2CH₃)
ESI(m/z) 520 (M+H⁺) 542 (M+Na⁺)

### (10-2) Preparation of diethyl 2-acetamido-2-(2-(4-((4-(2-acetoxyacetyl)phenyl)thio) phenyl)-2-oxoethyl)malonate

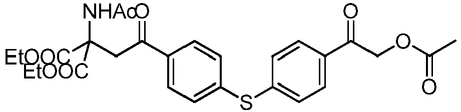

To a solution of diethyl 2-acetamido-2-(2-(4-((4-(2-chloroacetyl)phenyl)thio) phenyl)-2-oxoethyl)malonate (1.7 g, 3.3 mmol) in CH₃CN (18 mL) was added acetic acid (0.45 g, 7.5 mmol) and Et₃N(0.69 g, 6.9 mmol). The mixture was heated to reflux for 2h, then concentrated. The residue was diluted with CH₂Cl₂, washed with H₂O to neutral, dried over Na₂SO₄, filtered and concentrated, yielding the crude product (1.79g) as yellow syrup.
¹HNMR (MERCURY 300 MHz, CDCl₃) δ 7.91 (d, *J* = 8.1 Hz, 2H, 2ArH) 7.86 (d, *J* = 8.7 Hz, 2H, 2ArH) 7.42 (d, *J* = 7.8 Hz, 4H, 4ArH) 7.11 (brs, 1H, NH) 5.30 (s, 2H, CH₂) 4.30-4.19 (m, 6H, 3CH₂) 2.23 (s, 3H, CH₃) 1.98 (s, 3H, CH₃) 1.25 (t, *J* = 7.2 Hz, 6H, 2CH₃)
ESI(m/z) 566 (M+Na⁺)

### (10-3) Preparation of diethyl 2-acetamido-2-(2-oxo-2-(4-((4-(2-(propionyloxy)acetyl) phenyl)thio)phenyl)ethyl)malonate

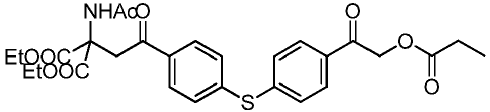

To a solution of diethyl 2-acetamido-2-(2-(4-((4-(2-chloroacetyl)phenyl)thio) phenyl)-2-oxoethyl)malonate (1.5 g, 2.8 mmol) in CH₃CN (15 mL) was added propionic acid (0.47 g, 6.3 mmol) and Et₃N(0.6 g, 5.8 mmol). The mixture was heated to reflux for 2h, then concentrated. The residue was diluted with CH₂Cl₂, washed with H₂O to neutral, dried over Na₂SO₄, filtered and concentrated, yielding the crude product (1.56g) as yellow syrup.
¹HNMR (MERCURY 300 MHz, CDCl₃) δ 7.91 (d, *J* = 8.4 Hz, 2H, 2ArH) 7.86 (d, *J* = 8.7 Hz, 2H, 2ArH) 7.41 (d, *J* = 7.8 Hz, 4H, 4ArH) 7.11 (brs, 1H, NH) 5.30 (s, 2H, CH₂) 4.30-4.25 (m, 6H, 3CH₂) 2.52 (q, *J* = 7.5 Hz, 2H, 1CH₂) 1.98 (s, 3H, CH₃) 1.27-1.19 (m, 9H, 3CH₃)
ESI(m/z) 558 (M+H⁺) 580 (M+Na⁺)

### (10-4) Preparation of diethyl 2-acetamido-2-(2-(4-((4-(2-(butyryloxy)acetyl) phenyl)thio)phenyl)-2-oxoethyl)malonate

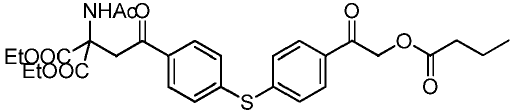

To a solution of diethyl 2-acetamido-2-(2-(4-((4-(2-chloroacetyl)phenyl)thio) phenyl)-2-oxoethyl)malonate (1.6 g, 3.1 mmol) in CH₃CN (15 mL) was added *n*-butyric acid (0.63 g, 7.1 mmol) and Et₃N(0.66 g, 6.6 mmol). The mixture was heated to reflux for 2h, then concentrated. The residue was diluted with CH₂Cl₂, washed with H₂O to neutral, dried over Na₂SO₄, filtered and concentrated, yielding the crude product (1.77g) as yellow syrup.
¹HNMR (MERCURY 300 MHz, CDCl₃) δ 7.92 (d, *J* = 7.8 Hz, 2H, 2ArH) 7.87 (d, *J* = 8.4 Hz, 2H, 2ArH) 7.43 (d, *J* = 7.8 Hz, 4H, 4ArH) 7.12 (brs, 1H, NH) 5.31 (s, 2H, CH₂) 4.32-4.20 (m, 6H, 3CH₂) 2.48 (t, *J* = 7.5 Hz, 2H, CH₂) 1.99 (s, 3H, CH₃) 1.79-1.71 (m, 2H, CH₂) 1.26 (t, *J* = 7.2 Hz, 6H, 2CH₃) 1.02 (t, *J* = 7.2 Hz, 3H, 1CH₃)
ESI(m/z) 572 (M+H⁺) 594 (M+Na⁺)

### (10-5) Preparation of diethyl 2-acetamido-2-(2-(4-((4-(2-(isobutyryloxy)acetyl)phenyl) thio)phenyl)-2-oxoethyl)malonate

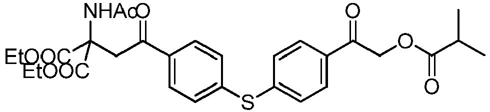

To a solution of diethyl 2-acetamido-2-(2-(4-((4-(2-chloroacetyl)phenyl)thio) phenyl)-2-oxoethyl)malonate (1.76 g, 3.4 mmol) in CH₃CN (18 mL) was added isobutyric acid (0.68 g, 7.7 mmol) and Et₃N(0.72 g, 7.1 mmol). The mixture was heated to reflux for 2h, then concentrated. The residue was diluted with CH₂Cl₂, washed with H₂O to neutral, dried over Na₂SO₄, filtered and concentrated, yielding the crude product (1.94g) as yellow syrup.
¹HNMR (MERCURY 300 MHz, CDCl₃) δ 7.92 (d, *J* = 7.8 Hz, 2H, 2ArH) 7.86 (d, *J* = 8.7 Hz, 2H, 2ArH) 7.42 (d, *J* = 7.8 Hz, 4H, 4ArH) 7.11 (brs, 1H, NH) 5.30 (s, 2H, CH₂) 4.31-4.25 (m, 6H, 3CH₂) 2.79-2.70 (m, 1H, 1CH) 1.98 (s, 3H, CH₃) 1.27-1.23 (m, 12H, 4CH₃)
ESI(m/z) 572 (M+H⁺) 594 (M+Na⁺)

### (10-6) Preparation of diethyl 2-acetamido-2-(2-(4-((4-(2-((cyclopropanecarbonyl)oxy) acetyl)phenyl)thio)phenyl)-2-oxoethyl)malonate

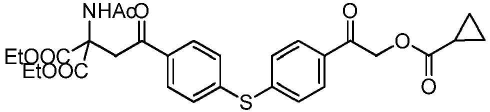

To a solution of diethyl 2-acetamido-2-(2-(4-((4-(2-chloroacetyl)phenyl)thio) phenyl)-2-oxoethyl)malonate (2.4 g, 4.6 mmol) in CH₃CN (25 mL) was added cyclopropanecarboxylic acid (0.91 g, 10.5 mmol) and Et₃N(0.98 g, 9.7 mmol). The mixture was heated to reflux for 2h, then concentrated. The residue was diluted with CH₂Cl₂, washed with H₂O to neutral, dried over Na₂SO₄, filtered and concentrated, yielding the crude product (2.6g) as yellow syrup.
¹HNMR (MERCURY 300 MHz, CDCl₃) δ 7.91 (d, *J* = 8.4 Hz, 2H, 2ArH) 7.86 (d, *J* = 8.4 Hz, 2H, 2ArH) 7.42 (d, *J* = 8.1 Hz, 4H, 4ArH) 7.11 (brs, 1H, NH) 5.30 (s, 2H, 1CH₂) 4.31-4.24 (m, 6H, 3CH₂) 1.98 (s, 3H, CH₃) 1.83-1.77 (m, 1H, 1CH) 1.24 (t, *J* = 6.9 Hz, 6H, 2CH₃) 1.11-1.10 (m, 2H, 1CH₂) 0.98-0.96 (m, 2H, 1CH₂)
ESI(m/z) 570 (M+H⁺) 592 (M+Na⁺)

### (10-7) Preparation of diethyl 2-acetamido-2-(2-(4-((4-(2-methyloxazol-4-yl)phenyl) thio)phenyl)-2-oxoethyl)malonate

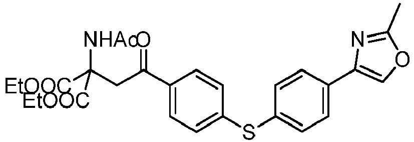

To a solution of diethyl 2-acetamido-2-(2-(4-((4-(2-acetoxyacetyl)phenyl)thio) phenyl)-2-oxoethyl)malonate (1.8 g, 3.3 mmol) in xylene(30 mL) was added acetamide (0.97 g, 16.5 mmol) and 47%BF₃·Et₂O(0.31mL). The mixture was heated to reflux for 40h, then concentrated. The residue was diluted with EtOAc, washed with H₂O (three times) and brine, dried over Na₂SO₄, filtered and concentrated. The residue was purified by silica gel column chromatography to afford the title compound (0.57g) as yellow syrup.
¹HNMR (MERCURY 300 MHz, CDCl₃) δ 7.88 (s, 1H, 1ArH) 7.82 (d, *J=* 8.7 Hz, 2H, 2ArH) 7.76 (d, *J* = 8.1 Hz, 2H, 2ArH) 7.51 (d, *J* = 8.1 Hz, 2H, 2ArH) 7.22 (d, *J* = 8.7 Hz, 2H, 2ArH) 7.08 (brs, 1H, NH) 4.26 (q, J = 7.2 Hz, 4H, 2CH₂) 4.19 (s, 2H, 1CH₂) 2.58 (s, 3H, 1CH₃) 1.96 (s, 3H, CH₃) 1.23 (t, *J* = 7.2 Hz, 6H, 2CH₃)
ESI(m/z) 525 (M+H⁺)

### (10-8) Preparation of diethyl 2-acetamido-2-(2-(4-((4-(2-ethyloxazol-4-yl)phenyl)thio) phenyl)-2-oxoethyl)malonate

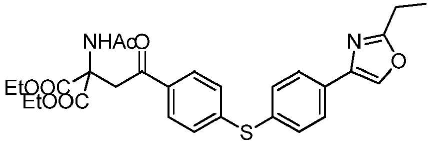

To a solution of diethyl 2-acetamido-2-(2-oxo-2-(4-((4-(2-(propionyloxy)acetyl) phenyl)thio)phenyl)ethyl)malonate (1.56 g, 2.8 mmol) in xylene(20 mL) was added acetamide (0.83 g, 14 mmol) and 47%BF₃·Et₂O(0.27 mL). The mixture was heated to reflux for 40h, then concentrated. The residue was diluted with EtOAc, washed with H₂O (three times) and brine, dried over Na₂SO₄, filtered and concentrated. The residue was purified by silica gel column chromatography to afford the title compound (0.46g) as yellow syrup.
¹HNMR (MERCURY 300 MHz, CDCl₃) δ 7.88 (s, 1H, 1ArH) 7.81 (d, *J* = 8.1 Hz, 2H, 2ArH) 7.77 (d, *J* = 8.7 Hz, 2H, 2ArH) 7.51 (d, *J* = 7.8 Hz, 2H, 2ArH) 7.21 (d, *J* = 8.1 Hz, 2H, 2ArH) 7.09 (brs, 1H, NH) 4.25 (q, J = 7.2 Hz, 4H, 2CH₂) 4.19 (s, 2H, 1CH₂) 2.89 (q, *J* = 7.5 Hz, 2H, 1CH₂) 1.96 (s, 3H, CH₃) 1.40 (t, *J* = 7.5 Hz, 3H, 1CH₃) 1.23 (t, *J* = 6.9 Hz, 6H, 2CH₃)
ESI(m/z) 539 (M+H⁺)

### (10-9) Preparation of diethyl 2-acetamido-2-(2-oxo-2-(4-((4-(2-propyloxazol-4-yl) phenyl)thio)phenyl)ethyl)malonate

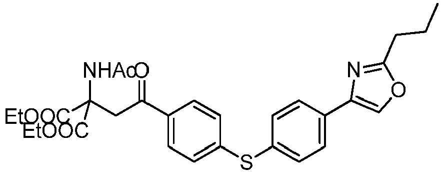

To a solution of diethyl 2-acetamido-2-(2-(4-((4-(2-(butyryloxy)acetyl) phenyl)thio)phenyl)-2-oxoethyl)malonate (1.77 g, 3.1 mmol) in xylene(30 mL) was added acetamide (0.92 g, 15.5 mmol) and 47%BF₃·Et₂O(0.3mL). The mixture was heated to reflux for 40h, then concentrated. The residue was diluted with EtOAc, washed with H₂O (three times) and brine, dried over Na₂SO₄, filtered and concentrated. The residue was purified by silica gel column chromatography to afford the title compound (0.57g) as yellow syrup.
¹HNMR (MERCURY 300 MHz, CDCl₃) δ 7.88 (s, 1H, 1ArH) 7.81 (d, *J=* 8.4 Hz, 2H, 2ArH) 7.77 (d, *J* = 8.4 Hz, 2H, 2ArH) 7.51 (d, *J* = 8.1 Hz, 2H, 2ArH) 7.20 (d, *J* = 8.4 Hz, 2H, 2ArH) 7.08 (brs, 1H, NH) 4.27 (q, J = 8.1 Hz, 4H, 2CH₂) 4.19 (s, 2H, 1CH₂) 2.83 (t, *J* = 7.8 Hz, 2H, 1CH₂) 1.96 (s, 3H, CH₃) 1.89-1.81 (m, 2H, 1CH₂) 1.23 (t, *J* = 6.9 Hz, 6H, 2CH₃) 1.03 (t, *J* = 7.2 Hz, 3H, 1CH₃)
ESI(m/z) 553 (M+H⁺)

### (10-10) Preparation of diethyl 2-acetamido-2-(2-(4-((4-(2-isopropyloxazol-4-yl) phenyl)thio)phenyl)-2-oxoethyl)malonate

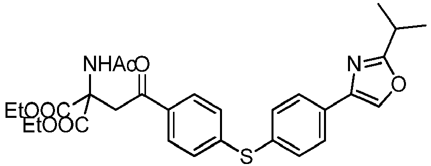

To a solution of diethyl 2-acetamido-2-(2-(4-((4-(2-(isobutyryloxy)acetyl)phenyl) thio)phenyl)-2-oxoethyl)malonate (1.94 g, 3.4 mmol) in xylene(30 mL) was added acetamide (1.0 g, 17 mmol) and 47%BF₃·Et₂O(0.32 mL). The mixture was heated to reflux for 40h, then concentrated. The residue was diluted with EtOAc, washed with H₂O (three times) and brine, dried over Na₂SO₄, filtered and concentrated. The residue was purified by silica gel column chromatography to afford the title compound (0.6g) as yellow syrup.
¹HNMR (MERCURY 300 MHz, CDCl₃) δ 7.87 (s, 1H, 1ArH) 7.81 (d, *J=* 8.4 Hz, 2H, 2ArH) 7.77 (d, *J* = 8.7 Hz, 2H, 2ArH) 7.51 (d, *J* = 8.4 Hz, 2H, 2ArH) 7.19 (d, *J* = 8.4 Hz, 2H, 2ArH) 7.08 (brs, 1H, NH) 4.25 (q, J = 6.9 Hz, 4H, 2CH₂) 4.19 (s, 2H, 1CH₂) 3.20-3.15 (m, 1H, 1CH) 1.96 (s, 3H, CH₃) 1.41 (s, 3H, 1CH₃) 1.39 (s, 3H, 1CH₃) 1.23 (t, *J* = 7.2 Hz, 6H, 2CH₃)
ESI(m/z) 553 (M+H⁺)

### (10-11) Preparation of diethyl 2-acetamido-2-(2-(4-((4-(2-cyclopropyloxazol-4-yl) phenyl)thio)phenyl)-2-oxoethyl)malonate

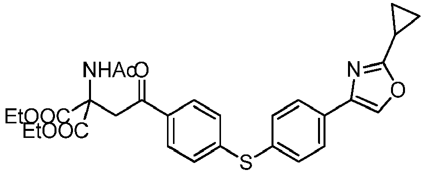

To a solution of diethyl 2-acetamido-2-(2-(4-((4-(2-((cyclopropanecarbonyl)oxy) acetyl)phenyl)thio)phenyl)-2-oxoethyl)malonate (2.6 g, 4.6 mmol) in xylene(30 mL) was added acetamide (1.36 g, 23 mmol) and 47%BF₃·Et₂O(0.43 mL). The mixture was heated to reflux for 40h, then concentrated. The residue was diluted with EtOAc, washed with H₂O (three times) and brine, dried over Na₂SO₄, filtered and concentrated. The residue was purified by silica gel column chromatography to afford the title compound (0.65g) as yellow syrup.
¹HNMR (MERCURY 300 MHz, CDCl₃) δ 7.81 (d, *J* = 8.4 Hz, 2H, 2ArH) 7.79 (s, 1H, 1ArH) 7.74 (d, *J* = 7.8 Hz, 2H, 2ArH) 7.50 (d, *J* = 7.8 Hz, 2H, 2ArH) 7.20 (d, *J* = 7.8 Hz, 2H, 2ArH) 7.08 (brs, 1H, NH) 4.25 (q, J = 7.2 Hz, 4H, 2CH₂) 4.19 (s, 2H, 1CH₂) 2.16-2.10 (m, 1H, 1CH) 1.96 (s, 3H, 1CH₃) 1.23 (t, *J* = 6.9 Hz, 6H, 2CH₃) 1.15-1.05 (m, 4H, 2CH₂)
ESI(m/z) 551 (M+H⁺)

### (10-12) Preparation of N-(1,4-dihydroxy-2-(hydroxymethyl)-4-(4-((4-(2-methyloxazol -4-yl)phenyl)thio)phenyl)butan-2-yl)acetamide

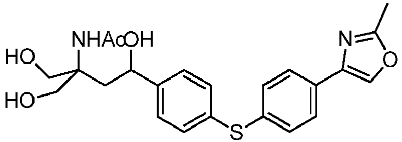

To a solution of diethyl 2-acetamido-2-(2-(4-((4-(2-methyloxazol-4-yl)phenyl) thio)phenyl)-2-oxoethyl)malonate (0.57 g, 1.1 mmol) in 95% EtOH (8mL) was added K₂HPO₄ (2.0 g, 8.6 mmol) in distilled water (2 mL) and NaBH₄ (0.21 g, 5.6 mmol) in aq. 10% NaOH(1.4mL). The mixture was stirred for further 6h at room temperature and concentrated. The residue was extracted with EtOAc, washed with H₂O to neutral, dried over Na₂SO₄, filtered and concentrated, yielding the crude product as light yellow syrup. The product was used in the next step without purification.
¹HNMR (MERCURY 300 MHz, CDCl₃) δ 7.79 (s, 1H, 1ArH) 7.62 (d, *J* = 8.1 Hz, 2H, 2ArH) 7.34-7.28 (m, 6H, 6ArH) 6.99 (brs, 1H, 1NH) 4.86 (d, *J* = 9.9 Hz, 1H, 1CH) 3.76 (d, *J* = 12.3 Hz, 1H, 1CH₂) 3.69 (d, *J* = 12.0 Hz, 1H, 1CH₂) 3.57 (d, *J* = 11.7 Hz, 1H, 1CH₂) 3.47 (d, *J* = 11.7 Hz, 1H, 1CH₂) 2.51(s, 3H, CH₃) 2.33 (d, *J* = 15.3 Hz, 1H, 1CH₂) 1.98(s, 3H, CH₃) 1.80 (dd, *J* = 14.7, 10.8 Hz, 1H, 1CH₂)
ESI(m/z) 443 (M+H⁺)

### (10-13) Preparation of N-(4-(4-((4-(2-ethyloxazol-4-yl)phenyl)thio)phenyl) -1,4-dihydroxy-2-(hydroxymethyl)butan-2-yl)acetamide

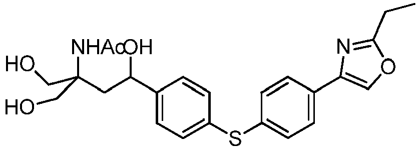

To a solution of diethyl 2-acetamido-2-(2-(4-((4-(2-ethyloxazol-4-yl)phenyl) thio)phenyl)-2-oxoethyl)malonate (0.46 g, 0.86 mmol) in 95% EtOH (6mL) was added K₂HPO₄ (1.5 g, 6.7 mmol) in distilled water (1.5 mL) and NaBH₄ (0.17 g, 4.4 mmol) in aq. 10% NaOH(1.1mL). The mixture was stirred for further 6h at room temperature and concentrated. The residue was extracted with EtOAc, washed with H₂O to neutral, dried over Na₂SO₄, filtered and concentrated. The residue was recrystallized with EtOAc to afford the title compound (0.32 g) as light yellow oil.
¹HNMR (MERCURY 300 MHz, CDCl₃) δ 7.80 (s, 1H, 1ArH) 7.65 (d, *J* = 8.1 Hz, 2H, 2ArH) 7.36-7.28 (m, 6H, 6ArH) 6.93 (brs, 1H, 1NH) 4.88 (d, *J* = 10.2 Hz, 1H, 1CH) 3.77 (d, *J=* 12.3 Hz, 1H, 1CH₂) 3.73 (d, *J=* 12.0 Hz, 1H, 1CH₂) 3.59 (d, *J* = 11.7 Hz, 1H, 1CH₂) 3.48 (d, *J* = 11.7 Hz, 1H, 1CH₂) 2.79 (q, *J* = 6.9 Hz, 2H, 1CH₂) 2.35 (d, *J* = 14.4 Hz, 1H, 1CH₂) 1.99 (s, 3H, 1CH₃) 1.81 (dd, *J=* 14.7, 10.8 Hz, 1H, 1CH₂) 1.25 (t, *J* = 7.2 Hz, 3H, 1CH₃)
ESI(m/z) 457 (M+H⁺)

### (10-14) Preparation of N-(1,4-dihydroxy-2-(hydroxymethyl)-4-(4-((4-(2-propyloxazol -4-yl)phenyl)thio)phenyl)butan-2-yl)acetamide

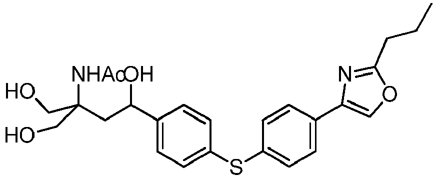

To a solution of diethyl 2-acetamido-2-(2-(4-((4-(2-propyloxazol-4-yl)phenyl) thio)phenyl)-2-oxoethyl)malonate (0.57 g, 1.1 mmol) in 95% EtOH (7.2 mL) was added K₂HPO₄(1.9 g, 8.2 mmol) in distilled water (1.9 mL) and NaBH₄ (0.2 g, 5.3 mmol) in aq. 10% NaOH(1.4mL). The mixture was stirred for further 6h at room temperature and concentrated. The residue was extracted with EtOAc, washed with H₂O to neutral, dried over Na₂SO₄, filtered and concentrated. The residue was recrystallized with EtOAc to afford the title compound (0.43 g) as white powder solid.
¹HNMR (MERCURY 300 MHz, CDCl₃) δ 7.80 (s, 1H, 1ArH) 7.65 (d, *J=* 8.4 Hz, 2H, 2ArH) 7.36-7.28 (m, 6H, 6ArH) 6.95 (brs, 1H, 1NH) 4.87 (d, *J* = 9.6 Hz, 1H, 1CH) 3.77 (d, *J* = 12.0 Hz, 1H, 1CH₂) 3.73 (d, *J* = 12.6 Hz, 1H, 1CH₂) 3.58 (d, *J* = 11.7 Hz, 1H, 1CH₂) 3.48 (d, *J* = 11.7 Hz, 1H, 1CH₂) 2.75 (t, *J* = 7.5 Hz, 2H, 1CH₂) 2.34 (d, *J* = 15.0 Hz, 1H, 1CH₂) 1.99 (s, 3H, 1CH₃) 1.85-1.77 (m, 3H, 2CH₂) 1.00 (t, *J* = 7.5 Hz, 3H, 1CH₃)
ESI(m/z) 471 (M+H⁺)

### (10-15) Preparation of N-(1,4-dihydroxy-2-(hydroxymethyl)-4-(4-((4-(2-isopropyloxazol-4-yl)phenyl)thio)phenyl)butan-2-yl)acetamide

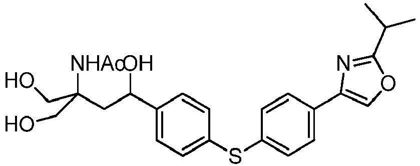

To a solution of diethyl 2-acetamido-2-(2-(4-((4-(2-isopropyloxazol-4-yl)phenyl) thio)phenyl)-2-oxoethyl)malonate (0.6 g, 1.1 mmol) in 95% EtOH (7.6 mL) was added K₂HPO₄ (2.0 g, 8.6 mmol) in distilled water (2 mL) and NaBH₄ (0.21 g, 5.6 mmol) in aq. 10% NaOH(1.4 mL). The mixture was stirred for further 6h at room temperature and concentrated. The residue was extracted with EtOAc, washed with H₂O to neutral, dried over Na₂SO₄, filtered and concentrated. The residue was recrystallized with EtOAc to afford the title compound (0.47 g) as white powder solid.
¹HNMR (MERCURY 300 MHz, CDCl₃) δ 7.81 (s, 1H, 1ArH) 7.66 (d, *J* = 8.1 Hz, 2H, 2ArH) 7.36-7.26 (m, 6H, 6ArH) 6.95 (brs, 1H, 1NH) 4.88 (d, *J* = 9.6 Hz, 1H, 1CH) 3.77 (d, *J* = 12.0 Hz, 1H, 1CH₂) 3.73 (d, *J* = 12.6 Hz, 1H, 1CH₂) 3.58 (d, *J* = 11.7 Hz, 1H, 1CH₂) 3.48 (d, *J* = 11.7 Hz, 1H, 1CH₂) 3.16-3.11 (m, 1H, 1CH) 2.36 (d, *J* = 15.3 Hz, 1H, 1CH₂) 1.98 (s, 3H, 1CH₃) 1.80 (dd, *J* = 14.7 Hz, 10.8Hz, 1H, 1CH₂) 1.39 (s, 3H, 1CH₃) 1.36 (s, 3H, 1CH₃)
ESI(m/z) 471 (M+H⁺)

### (10-16) Preparation of N-(4-(4-((4-(2-cyclopropyloxazol-4-yl)phenyl)thio) phenyl)-1,4-dihydroxy-2-(hydroxymethyl)butan-2-yl)acetamide

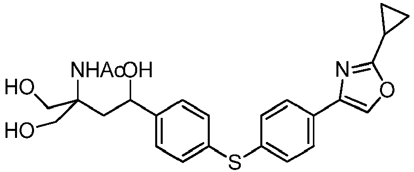

To a solution of diethyl 2-acetamido-2-(2-(4-((4-(2-cyclopropyloxazol-4-yl)phenyl) thio)phenyl)-2-oxoethyl)malonate (0.65 g, 1.2 mmol) in 95% EtOH (8.3 mL) was added K₂HPO₄(2.1 g, 9.3 mmol) in distilled water (2.1 mL) and NaBH₄ (0.23 g, 6.1 mmol) in aq. 10% NaOH(1.6 mL). The mixture was stirred for further 6h at room temperature and concentrated. The residue was extracted with EtOAc, washed with H₂O to neutral, dried over Na₂SO₄, filtered and concentrated. The residue was recrystallized with EtOAc to afford the title compound (0.5 g) as white powder solid.
¹HNMR (MERCURY 300 MHz, CDCl₃) δ 7.72 (s, 1H, 1ArH) 7.61 (d, *J=* 8.4 Hz, 2H, 2ArH) 7.34-7.26 (m, 6H, 6ArH) 7.02 (brs, 1H, 1NH) 4.85 (d, *J* = 10.5 Hz, 1H, 1CH) 3.75 (d, *J* = 11.4 Hz, 1H, 1CH₂) 3.71 (d, *J* = 11.7 Hz, 1H, 1CH₂) 3.56 (d, *J* = 11.4 Hz, 1H, 1CH₂) 3.46 (d, *J* = 12.0 Hz, 1H, 1CH₂) 2.30 (d, *J* = 15.3 Hz, 1H, 1CH₂) 1.98 (s, 3H, 1CH₃) 2.04-1.96 (m, 1H, 1CH) 1.80 (dd, *J* = 14.4 Hz, 10.5Hz, 1H, 1CH₂) 1.09-1.02 (m, 4H, 2CH₂)
ESI(m/z) 469 (M+H⁺)

### (10-17) Preparation of 3-amino-3-(hydroxymethyl)-1-(4-((4-(2-methyloxazol-4-yl) phenyl)thio)phenyl)butane-1,4-diol hydrochloride

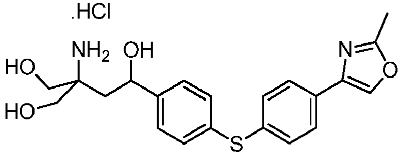

To a solution of N-(1,4-dihydroxy-2-(hydroxymethyl)-4-(4-((4-(2-methyloxazol -4-yl)phenyl)thio)phenyl)butan-2-yl)acetamide (0.57 g, 1.3 mmol) in MeOH(10mL) was added NaOH(0.053 g, 1.32 mmol) and heated to reflux for 8h, then filtered to remove insoluble substance. The solution was added HCl-EtOH solution until pH=3-4 and concentrated. The residue was purified by silica gel column chromatography to afford the title compound (0.3 g) as light yellow solid.
¹HNMR (MERCURY 300 MHz, CD₃OD) δ 8.17 (s, 1H, 1ArH) 7.57 (d, *J* = 7.8 Hz, 2H, 2ArH) 7.34 (d, *J* = 8.4 Hz, 2H, 2ArH) 7.30 (d, *J* = 7.8 Hz, 2H, 2ArH) 7.23 (d, *J =* 7.5 Hz, 2H, 2ArH) 4.97 (d, *J* = 9.3 Hz, 1H, 1CH) 3.82 (d, *J* = 12.3 Hz, 1H, 1CH₂) 3.77 (d, *J=* 12.3 Hz, 1H, 1CH₂) 3.65 (d, *J* = 11.7 Hz, 1H, 1CH₂) 3.57 (d, *J* = 11.4 Hz, 1H, 1CH₂) 2.48 (s, 3H, 1CH₃) 1.91-1.80 (m, 2H, 1CH₂)
¹³CNMR (400 MHz, CD₃OD) δ 164.78, 146.36, 139.93, 138.18, 136.30, 135.14, 133.07, 131.59, 127.85, 127.27, 70.51, 63.76, 62.11, 61.71, 40.82, 13.52
ESI(m/z) 401 (M+H⁺) HRMS calcd. for C₂₁H₂₄N₂O₄S (M+H⁺) 401.1530, found 401.1511

### (10-18) Preparation of 3-amino-1-(4-((4-(2-ethyloxazol-4-yl)phenyl)thio) phenyl)-3-(hydroxymethyl)butane-1,4-diol hydrochloride

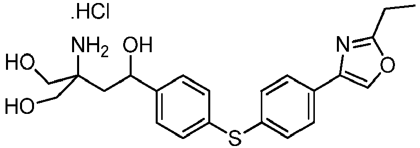

To a solution of *N*-(1,4-dihydroxy-2-(hydroxymethyl)-4-(4-((4-(2-ethyloxazol -4-yl)phenyl)thio)phenyl)butan-2-yl)acetamide (0.32 g, 0.7 mmol) in MeOH(10mL) was added NaOH(0.029 g, 0.72 mmol) and heated to reflux for 8h, then filtered to remove insoluble substance. The solution was added HCl-EtOH solution until pH=3-4 and concentrated. The residue was purified by silica gel column chromatography to afford the title compound (0.27 g) as light yellow solid.
¹HNMR (MERCURY 300 MHz, CD₃OD) δ 8.18 (s, 1H, 1ArH) 7.60 (d, *J* = 7.2 Hz, 2H, 2ArH) 7.35 (d, *J* = 8.7 Hz, 2H, 2ArH) 7.31 (d, *J* = 7.5 Hz, 2H, 2ArH) 7.24 (d, *J* = 6.9 Hz, 2H, 2ArH) 4.98 (d, *J=* 8.7 Hz, 1H, CH) 3.80 (brs, 2H, 1CH₂) 3.66 (d, *J* = 11.4 Hz, 1H, 1CH₂) 3.58 (d, *J* = 11.7 Hz, 1H, 1CH₂) 2.84 (q, *J* = 7.2 Hz, 2H, 1CH₂) 1.96-1.81 (m, 2H, CH₂) 1.31 (t, *J* = 7.5 Hz, 3H, CH₃)
¹³CNMR (400 MHz, CD₃OD) δ 168.76, 146.34, 139.84, 138.13, 136.16, 135.13, 133.05, 131.57, 127.84, 127.35, 70.49, 63.75, 62.10, 61.70, 40.80, 22.34, 11.24 ESI(m/z) 415 (M+H⁺) HRMS calcd. for C₂₂H₂₆N₂O₄S (M+H⁺) 415.1686, found 415.1682

### (10-19) Preparation of 3-amino-3-(hydroxymethyl)-1-(4-((4-(2-propyloxazol-4-yl) phenyl)thio)phenyl)butane-1,4-diol hydrochloride

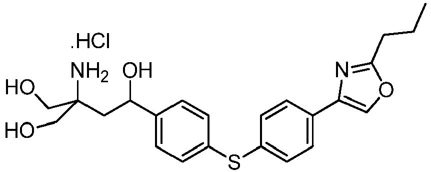

To a solution of N-(1,4-dihydroxy-2-(hydroxymethyl)-4-(4-((4-(2-propyloxazol -4-yl)phenyl)thio)phenyl)butan-2-yl)acetamide (0.43 g, 0.9 mmol) in MeOH(10mL) was added NaOH(0.038 g, 0.94 mmol) and heated to reflux for 8h, then filtered to remove insoluble substance. The solution was added HCl-EtOH solution until pH=3-4 and concentrated. The residue was purified by silica gel column chromatography to afford the title compound (0.36g) as light yellow solid.
¹HNMR (MERCURY 300 MHz, CD₃OD) δ 8.17 (s, 1H, 1ArH) 7.58 (d, *J* = 8.1 Hz, 2H, 2ArH) 7.33 (d, *J* = 7.8 Hz, 2H, 2ArH) 7.29 (d, *J* = 8.4 Hz, 2H, 2ArH) 7.22 (d, *J =* 8.4 Hz, 2H, 2ArH) 4.96 (d, *J* = 9.9 Hz, 1H, 1CH) 3.81 (d, *J* = 11.7 Hz, 1H, 1CH₂) 3.76 (d, *J* = 11.7 Hz, 1H, 1CH₂) 3.64 (d, *J* = 11.4 Hz, 1H, 1CH₂) 3.56 (d, *J* = 11.4 Hz, 1H, 1CH₂) 2.78 (t, *J* = 7.2Hz, 2H, 1CH₂) 1.94-1.72 (m, 4H, 2CH₂) 0.93 (t, *J* = 7.2Hz, 3H, 1CH₃)
¹³CNMR (400 MHz, CD₃OD) δ 167.85, 146.34, 139.79, 138.15, 136.23, 135.11, 133.05, 131.56, 127.4, 127.34, 70.49, 63.75, 62.10, 61.70, 40.80, 30.58, 21.36, 13.88 ESI(m/z) 429 (M+H⁺) HRMS calcd. for C₂₃H₂₉N₂O₄S (M+H⁺) 429.1843, found 429.1838

### (10-20) Preparation of 3-amino-3-(hydroxymethyl)-1-(4-((4-(2-isopropyloxazol-4-yl) phenyl)thio)phenyl)butane-1,4-diol hydrochloride

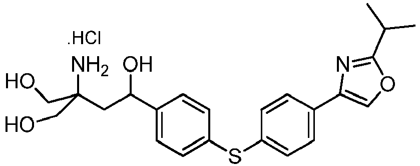

To a solution of *N*-(1,4-dihydroxy-2-(hydroxymethyl)-4-(4-((4-(2-isopropyloxazol -4-yl)phenyl)thio)phenyl)butan-2-yl)acetamide (0.47g, 1.0 mmol) in MeOH(10mL) was added NaOH(0.041 g, 1.03 mmol) and heated to reflux for 8h, then filtered to remove insoluble substance. The solution was added HCl-EtOH solution until pH=3-4 and concentrated. The residue was purified by silica gel column chromatography to afford the title compound (0.36g) as light yellow solid.
¹HNMR (MERCURY 300 MHz, CD₃OD) δ 8.21 (s, 1H, 1ArH) 7.59 (d, *J* = 8.1 Hz, 2H, 2ArH) 7.33 (d, *J* = 8.7 Hz, 2H, 2ArH) 7.31 (d, *J* = 8.4 Hz, 2H, 2ArH) 7.22 (d, *J =* 8.1 Hz, 2H, 2ArH) 4.97 (d, *J* = 13.2 Hz, 1H, 1CH) 3.81 (d, *J* = 11.4 Hz, 1H, 1CH₂) 3.76 (d, *J* = 11.4 Hz, 1H, CH₂) 3.64 (d, *J* = 11.1 Hz, 1H, 1CH₂) 3.56 (d, *J* = 11.4 Hz, 1H, 1CH₂) 3.22-3.15 (m, 1H, 1CH) 1.98-1.81 (m, 2H, 1CH₂) 1.34 (brs, 3H, 1CH₃) 1.31 (brs, 3H, 1CH₃)
¹³CNMR (400 MHz, CD₃OD) δ 171.97, 146.46, 139.24, 138.60, 136.30, 134.92, 133.21, 131.41, 127.88, 127.53, 70.49, 63.75, 62.10, 61.70, 40.81, 29.73, 20.34 ESI(m/z) 429 (M+H⁺) HRMS calcd. for C₂₃H₂₈N₂O₄S (M+H⁺) 429.1843, found 429.1843

### (10-21) Preparation of 3-amino-1-(4-((4-(2-cyclopropyloxazol-4-yl)phenyl) thio)phenyl)-3-(hydroxymethyl)butane-1,4-diol hydrochloride

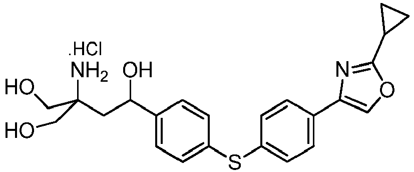

To a solution of *N*-(1,4-dihydroxy-2-(hydroxymethyl)-4-(4-((4-(2-cyclopropyloxazol-4-yl)phenyl)thio)phenyl)butan-2-yl)acetamide (0.5 g, 1.0 mmol) in MeOH(10mL) was added NaOH(0.044 g, 1.1 mmol) and heated to reflux for 8h, then filtered to remove insoluble substance. The solution was added HCl-EtOH solution until pH=3-4 and concentrated. The residue was purified by silica gel column chromatography to afford the title compound (0.43g) as light yellow solid.
¹HNMR (MERCURY 300 MHz, CD₃OD) δ 8.19 (s, 1H, 1ArH) 7.54 (d, *J* = 8.1 Hz, 2H, 2ArH) 7.34 (d, *J* = 8.4 Hz, 2H, 2ArH) 7.30 (d, *J* = 8.4 Hz, 2H, 2ArH) 7.20 (d, *J =* 8.4 Hz, 2H, 2ArH) 4.96 (dd , *J* = 10.2 Hz, 2.4 Hz, 1H, 1CH) 3.80 (d, *J* = 11.4 Hz, 1H, 1CH₂) 3.75 (d, *J* = 11.7 Hz, 1H, 1CH₂) 3.63 (d, *J* = 11.4 Hz, 1H, 1CH₂) 3.54 (d, *J* = 11.4 Hz, 1H, 1CH₂) 2.26-2.17 (m, 1H, 1CH) 1.93-1.77 (m, 2H, 1CH₂) 1.24-1.18 (m, 4H, 2CH₂)
¹³CNMR (400 MHz, CD₃OD) δ 169.85, 146.64, 139.46, 138.18, 136.04, 134.52, 133.49, 131.19, 127.93, 127.49, 70.49, 63.78, 62.13, 61.75, 40.80, 10.09, 9.47 ESI(m/z) 427 (M+H⁺) HRMS calcd. for C₂₃H₂₆N₂O₄S (M+H⁺) 427.1686, found 427.1671

### EXAMPLE 11

This example described the preparation of

| | |
|---|---|
| 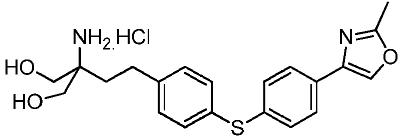 | 2-amino-2-(4-((4-(2-methyloxazol-4-yl)pheny l)thio)phenethyl)propane-1,3-diol hydrochloride |
| 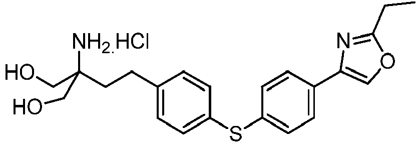 | 2-amino-2-(4-((4-(2-ethyloxazol-4-yl)pheny l)thio)phenethyl)propane-1,3-diol hydrochloride |
| 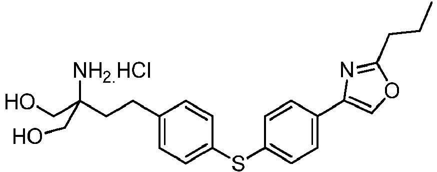 | 2-amino-2-(4-((4-(2-propyloxazol-4-yl)phe nyl)thio)phenethyl)propane-1,3-diol hydrochloride |
| 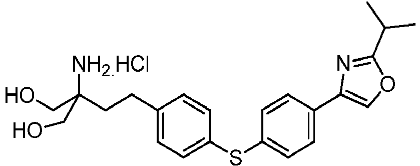 | 2-amino-2-(4-((4-(2-isopropyloxazol-4-yl)p henyl)thio)phenethyl)propane-1,3-diol hydrochloride |
| 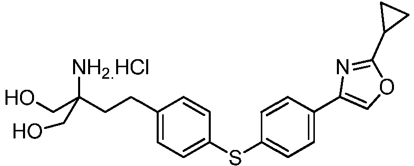 | 2-amino-2-(4-((4-(2-cyclopropyloxazol-4-yl) phenyl)thio)phenethyl)propane-1,3-diol hydrochloride |

### (11-1) Preparation of 2-chloro-1-(4-(phenylthio)phenyl)ethanone

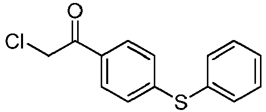

Chloroacetyl chloride (12.2 g, 107.5 mmol) was added dropwise to a cooled solution (0°C) of diphenyl sulfide (20 g, 107.5 mmol) in dry CH₂Cl₂ (200mL), then AlCl₃ (15.0 g, 112.4 mmol) was added in portions. The solution was allowed to return to room temperature and stirred for further 4 h, then poured slowly into HCl-ice mixture. The organic layer was separated and the aqueous phase was extracted with CH₂Cl₂ three times. The combined organic layers were washed with H₂O to neutral, dried over Na₂SO₄, filtered and concentrated, yielding the crude product (25g) as light yellow oil. The product was used in the next step without purification.

### (11-2) Preparation of diethyl 2-acetamido-2-(2-oxo-2-(4-(phenylthio)phenyl) ethyl)malonate

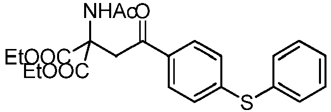

NaH (2.5 g, 104.5 mmol) was added to dry THF (300mL) at room temperature. After 30 min, diethyl acetamidomalonate (24.7 g, 114 mmol) was added in portions and stirred for further 5h. Then a solution of 2-chloro-1-(4-(phenylthio)phenyl)ethanone (25 g, 95 mmol) in THF was added. The mixture was heated to reflux for further 12h and concentrated. The residue was diluted with EtOAc, washed with H₂O to neutral, dried over Na₂SO₄, filtered and concentrated. The residue was purified by silica gel column chromatography to afford the title compound (27g) as colorless syrup.

### (11-3) Preparation of diethyl 2-acetamido-2-(4-(phenylthio)phenethyl)malonate

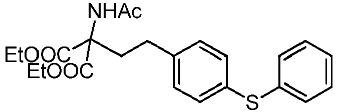

A solution of diethyl 2-acetamido-2-(2-oxo-2-(4-(phenylthio)phenyl) ethyl)malonate (10.6 g, 23.9 mmol) in CH₂Cl₂ (38mL) was added dropwise to a solution of Et₃SiH (10.5 g, 90.7 mmol) in CH₂Cl₂ (100mL) at room temperature under N₂ protection. TiCl₄ (17.2g, 90.7mmol) was added to the solution with a syringe and the reaction mixture was stirred overnight, then poured slowly into HCl-ice mixture. The organic layer was separated and the aqueous phase was extracted with CH₂Cl₂ three times. The combined organic layers were washed with H₂O to neutral, dried over Na₂SO₄, filtered and concentrated. The residue was purified by silica gel column chromatography to afford the title compound (8.0g) as yellow syrup.
¹HNMR (MERCURY 300 MHz, CDCl₃) δ 7.32-7.21 (m, 7H, 7ArH) 7.10 (d, *J* = 8.4 Hz, 2H, 2ArH) 6.77 (brs, 1H, NH) 4.25-4.18 (m, 4H, 2CH₂) 2.68 (dd, *J* = 9.6 Hz, 6.9 Hz, 2H, 1CH₂) 2.46 (dd, *J* = 8.7 Hz, 6.9 Hz, 2H, 1CH₂) 1.99 (s, 3H, CH₃) 1.25 (t, *J* = 6.9 Hz, 6H, 2CH₃)

### (11-4) Preparation of diethyl 2-acetamido-2-(4-((4-(2-chloroacetyl)phenyl)thio) phenethyl)malonate

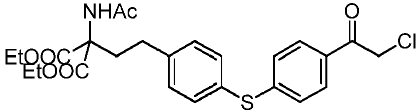

Chloroacetyl chloride (4.7 g, 41.2 mmol) in was added dropwise to a cooled solution (0°C) of diethyl 2-acetamido-2-(4-(phenylthio)phenethyl)malonate (15.9 g, 37.5 mmol) in dry CH₂Cl₂(200mL), then AlCl₃ (25 g, 188 mmol) was added in portions. The solution was allowed to return to room temperature and stirred for further 5 h, then poured slowly into HCl-ice mixture. The organic layer was separated and the aqueous phase was extracted with CH₂Cl₂ three times. The combined organic layers were washed with H₂O to neutral, dried over Na₂SO₄, filtered and concentrated. The residue was purified by silica gel column chromatography to afford the title compound (4.2g).
¹HNMR(MERCURY 300 MHz, CDCl₃) δ 7.79 (d, J = 8.4 Hz, 2H, 2ArH) 7.43 (d, J = 7.8 Hz, 2H, 2ArH) 7.21 (d, J = 7.5 Hz, 2H, 2ArH) 7.15 (d, J = 7.5 Hz, 2H, 2ArH) 6.81 (brs, 1H, 1NH) 4.62 (s, 2H, 1CH₂) 4.29-4.22 (m, 4H, 2CH₂) 2.70 (dd, J = 11.4 Hz, 7.5 Hz, 2H, 1CH₂) 2.52 (dd, J = 10.2 Hz, 6.3 Hz, 2H, 1CH₂) 2.03 (s, 3H, 1CH₃) 1.24 (t, J = 7.2 Hz, 6H, 2CH₃)
ESI(m/z) 506 (M+H⁺) 528 (M+Na⁺)

### (11-5) Preparation of diethyl 2-acetamido-2-(4-((4-(2-acetoxyacetyl)phenyl)thio) phenethyl)malonate

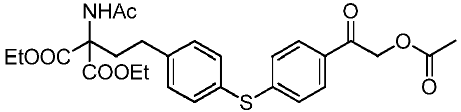

To a solution of diethyl 2-acetamido-2-(4-((4-(2-chloroacetyl)phenyl)thio) phenethyl)malonate (1.0 g, 2.0 mmol) in CH₃CN (10 mL) was added acetic acid (0.27 g, 4.5 mmol) and Et₃N(0.42 g, 4.2 mmol). The mixture was heated to reflux for 2h, then concentrated. The residue was diluted with CH₂Cl₂, washed with H₂O to neutral, dried over Na₂SO₄, filtered and concentrated, yielding the crude product (1.05g) as yellow syrup.
¹HNMR(MERCURY 300MHz, CDCl₃) δ 7.76 (d, *J* = 7.8 Hz, 2H, 2ArH) 7.43 (d, *J* = 7.5 Hz, 2H, 2ArH) 7.21 (d, *J* = 7.8 Hz, 2H, 2ArH) 7.16 (d, *J* = 8.4 Hz, 2H, 2ArH) 6.80 (brs, 1H, NH) 5.27 (s, 2H, 1CH₂) 4.28-4.20 (m, 4H, 2CH₂) 2.72 (dd, *J* = 8.4 Hz, 7.8 Hz, 2H, 1CH₂) 2.51 (dd, *J* = 9.3 Hz, 7.8 Hz, 2H, 1CH₂) 2.22 (s, 3H, 1CH₃) 2.03 (s, 3H, 1CH₃) 1.27 (t, *J* = 7.2 Hz, 6H, 2CH₃)
ESI(m/z) 530 (M+H⁺)

### (11-6) Preparation of diethyl 2-acetamido-2-(4-((4-(2-(propionyloxy)acetyl)phenyl) thio)phenethyl)malonate

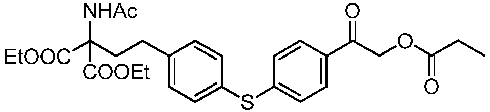

To a solution of diethyl 2-acetamido-2-(4-((4-(2-chloroacetyl)phenyl)thio) phenethyl)malonate (1.05 g, 2.1 mmol) in CH₃CN (10 mL) was added propionic acid (0.35 g, 4.7 mmol) and Et₃N(0.44 g, 4.4 mmol). The mixture was heated to reflux for 2h, then concentrated. The residue was diluted with CH₂Cl₂, washed with H₂O to neutral, dried over Na₂SO₄, filtered and concentrated, yielding the crude product (1.1g) as yellow syrup.
¹HNMR(MERCURY 300MHz, CDCl₃) δ 7.74 (d, *J* = 8.7 Hz, 2H, 2ArH) 7.41 (d, *J* = 7.8 Hz, 2H, 2ArH) 7.20 (d, *J* = 7.2 Hz, 2H, 2ArH) 7.15 (d, *J* = 8.1 Hz, 2H, 2ArH) 6.79 (brs, 1H, NH) 5.26 (s, 2H, 1CH₂) 4.28-4.20 (m, 4H, 2CH₂) 2.71 (dd, *J* = 9.0 Hz, 7.2 Hz, 2H, 1CH₂) 2.53-2.49 (m, 4H, 2CH₂) 2.02 (s, 3H, 1CH₃) 1.39-1.17 (m, 9H, 3CH₃)
ESI(m/z) 544 (M+H⁺)

### (11-7) Preparation of diethyl 2-acetamido-2-(4-((4-(2-(butyryloxy)acetyl)phenyl)thio) phenethyl)malonate

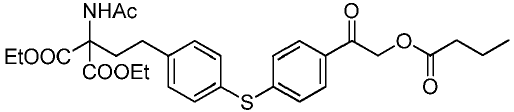

To a solution of diethyl 2-acetamido-2-(4-((4-(2-chloroacetyl)phenyl)thio) phenethyl)malonate (1.1 g, 2.0 mmol) in CH₃CN (10 mL) was added *n*-butyric acid (0.40 g, 4.6 mmol) and Et₃N(0.43 g, 4.2 mmol). The mixture was heated to reflux for 2h, then concentrated. The residue was diluted with CH₂Cl₂, washed with H₂O to neutral, dried over Na₂SO₄, filtered and concentrated. The residue was purified by silica gel column chromatography to afford the title compound (0.56g) as white solid.

### (11-8) Preparation of diethyl 2-acetamido-2-(4-((4-(2-(isobutyryloxy)acetyl)phenyl) thio)phenethyl)malonate

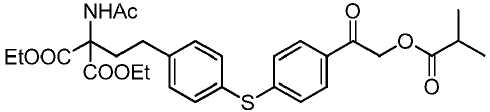

To a solution of diethyl 2-acetamido-2-(4-((4-(2-chloroacetyl)phenyl)thio) phenethyl)malonate (5.2 g, 9.5 mmol) in CH₃CN (20 mL) was added isobutyric acid (1.9 g, 21.7 mmol) and Et₃N(2.0 g, 20 mmol). The mixture was heated to reflux for 2h, then concentrated. The residue was diluted with CH₂Cl₂, washed with H₂O to neutral, dried over Na₂SO₄, filtered and concentrated. The residue was purified by silica gel column chromatography to afford the title compound (1.0g) as light yellow syrup.
¹HNMR(MERCURY 300 MHz, CDCl₃) δ 7.75 (d, *J* = 8.4 Hz, 2H, 2ArH) 7.42 (d, *J* = 8.1 Hz, 2H, 2ArH) 7.18 (d, *J* = 7.2 Hz, 2H, 2ArH) 7.13 (d, *J* = 9.3 Hz, 2H, 2ArH) 6.82 (brs, 1H, NH) 5.17 (s, 2H, 1CH₂) 4.30-4.20 (m, 4H, 2CH₂) 2.75-2.69 (m, 3H, 1CH₂, 1CH) 2.51 (dd, *J* = 9.6 Hz, 5.4 Hz, 2H, 1CH₂) 2.02 (s, 3H, 1CH₃) 1.32-1.18 (m, 1H, 4CH₃)

### (11-9) Preparation of diethyl 2-acetamido-2-(4-((4-(2-((cyclopropanecarbonyl)oxy) acetyl)phenyl)thio)phenethyl)malonate

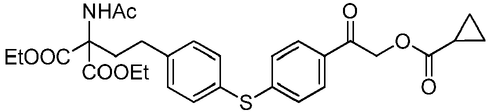

To a solution of diethyl 2-acetamido-2-(4-((4-(2-chloroacetyl)phenyl)thio) phenethyl)malonate (5.2 g, 9.5 mmol) in CH₃CN (20 mL) was added cyclopropanecarboxylic acid (1.9 g, 21.7 mmol) and Et₃N(2.0 g, 20 mmol). The mixture was heated to reflux for 2h, then concentrated. The residue was diluted with CH₂Cl₂, washed with H₂O to neutral, dried over Na₂SO₄, filtered and concentrated. The residue was purified by silica gel column chromatography to afford the title compound (1.0g) as light yellow syrup.

### (11-10) Preparation of diethyl 2-acetamido-2-(4-((4-(2-methyloxazol-4-yl)phenyl) thio)phenethyl)malonate

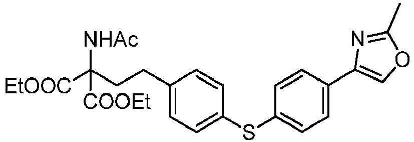

To a solution of diethyl 2-acetamido-2-(4-((4-(2-acetoxyacetyl)phenyl)thio) phenethyl)malonate (1.05 g, 2.0 mmol) in xylene(18 mL) was added acetamide (0.59 g, 9.9 mmol) and 47%BF₃·Et₂O(0.19mL). The mixture was heated to reflux for 40h, then concentrated. The residue was diluted with EtOAc, washed with H₂O (three times) and brine, dried over Na₂SO₄, filtered and concentrated. The residue was purified by silica gel column chromatography to afford the title compound (0.7g) as light yellow syrup.
¹HNMR(MERCURY300MHz, CDCl₃) δ 7.80 (s, 1H, 1ArH) 7.63 (d, *J* = 8.1 Hz, 2H, 2ArH) 7.30 (d, *J* = 7.8 Hz, 4H, 4ArH) 7.11 (d, *J* = 8.1 Hz, 2H, 2ArH) 6.76 (brs, 1H, NH) 4.25-4.17 (m, 4H, 2CH₂) 2.68 (dd, *J* = 10.5 Hz, 6.6 Hz, 2H, 1CH₂) 2.55 (s, 3H, 1CH₃) 2.47 (dd, *J* = 8.7 Hz, 4.8 Hz, 2H, 1CH₂) 2.03 (s, 3H, 1CH₃) 1.25 (t, *J* = 7.2 Hz, 6H, 2CH₃)
ESI(m/z) 511 (M+H⁺)

### (11-11) Preparation of diethyl 2-acetamido-2-(4-((4-(2-ethyloxazol-4-yl)phenyl)thio) phenethyl)malonate

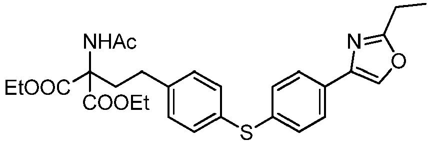

To a solution of diethyl 2-acetamido-2-(4-((4-(2-(propionyloxy)acetyl)phenyl) thio)phenethyl)malonate (1.1 g, 2.1 mmol) in xylene(20 mL) was added acetamide (0.6 g, 10.4 mmol) and 47%BF₃·Et₂O(0.2mL). The mixture was heated to reflux for 40h, then concentrated. The residue was diluted with EtOAc, washed with H₂O (three times) and brine, dried over Na₂SO₄, filtered and concentrated. The residue was purified by silica gel column chromatography to afford the title compound (0.74g) as light yellow oil.
¹HNMR(MERCURY300MHz, CDCl₃) δ 7.82 (s, 1H, 1ArH) 7.65 (d, *J* = 8.7 Hz, 2H, 2ArH) 7.29 (d, *J* = 8.1 Hz, 4H, 4ArH) 7.11 (d, *J* = 7.8 Hz, 2H, 2ArH) 6.76 (brs, 1H, NH) 4.24-4.19 (m, 4H, 2CH₂) 2.92 (q, *J* = 7.2 Hz, 2H) 2.69 (dd, *J* = 8.7 Hz, 7.5 Hz, 2H, 1CH₂) 2.47 (dd, *J* = 8.1 Hz, 7.8 Hz, 2H, 1CH₂) 2.03 (s, 3H, 1CH₃) 1.39 (t, *J* = 7.8 Hz, 3H, 1CH₃) 1.25 (t, *J* = 6.9 Hz, 6H, 2CH₃)
ESI(m/z) 525 (M+H⁺)

### (11-12) Preparation of diethyl 2-acetamido-2-(4-((4-(2-propyloxazol-4-yl)phenyl)thio) phenethyl)malonate

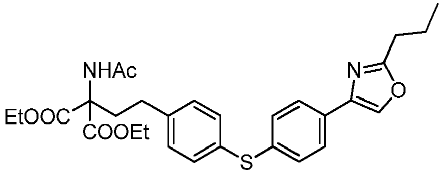

To a solution of diethyl 2-acetamido-2-(4-((4-(2-(butyryloxy)acetyl)phenyl)thio) phenethyl)malonate (0.56 g, 1 mmol) in xylene(10 mL) was added acetamide (0.30 g, 5 mmol) and 47%BF₃·Et₂O(0.1mL). The mixture was heated to reflux for 40h, then concentrated. The residue was diluted with EtOAc, washed with H₂O (three times) and brine, dried over Na₂SO₄, filtered and concentrated. The residue was purified by silica gel column chromatography to afford the title compound (0.44g) as light yellow oil.
¹HNMR(MERCURY300MHz, CDCl₃) δ 7.82 (s, 1H, 1ArH) 7.65 (d, *J* = 8.7 Hz, 2H, 2ArH) 7.29 (d, *J* = 8.1 Hz, 4H, 4ArH) 7.11 (d, *J* = 7.8 Hz, 2H, 2ArH) 6.76 (brs, 1H, NH) 4.24-4.19 (m, 4H, 2CH₂) 2.85 (t, *J* = 7.5 Hz, 2H, 1CH₂) 2.70 (t, *J* = 6.9 Hz, 2H, 1CH₂) 2.55-2.47 (m, 2H, 1CH₂) 1.89-1.70 (m, 2H, 1CH₂) 1.26 (t, *J* = 7.2 Hz, 6H, 2CH₃) 1.01 (t, *J* = 7.2 Hz, 6H, 2CH₃)
ESI(m/z) 539 (M+H⁺)

### (11-13) Preparation of diethyl 2-acetamido-2-(4-((4-(2-isopropyloxazol-4-yl)phenyl) thio)phenethyl)malonate

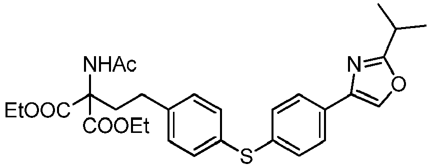

To a solution of diethyl 2-acetamido-2-(4-((4-(2-(isobutyryloxy)acetyl)phenyl) thio)phenethyl)malonate (1.0 g, 1.8 mmol) in xylene(10 mL) was added acetamide (0.53 g, 8.9 mmol) and 47%BF₃·Et₂O(0.18mL). The mixture was heated to reflux for 40h, then concentrated. The residue was diluted with EtOAc, washed with H₂O (three times) and brine, dried over Na₂SO₄, filtered and concentrated. The residue was purified by silica gel column chromatography to afford the title compound (1.0g) as light yellow oil.
¹HNMR(MERCURY 300 MHz, CDCl₃) δ 7.80 (s, 1H, 1ArH) 7.65 (d, *J* = 8.4 Hz, 2H, 2ArH) 7.29 (d, *J* = 8.1 Hz, 4H, 4ArH) 7.09 (d, *J* = 8.1 Hz, 2H, 2ArH) 6.76 (brs, 1H, NH) 4.25-4.13 (m, 4H, 2CH₂) 3.22-3.18 (m, 1H, 1CH) 2.69 (dd, *J* = 11.4 Hz, 6.9 Hz, 2H, 1CH₂) 2.48 (dd, *J* = 9.0 Hz, 5.1 Hz, 2H, 1CH₂) 2.03 (s, 3H, 1CH₃) 1.40 (s, 3H, 1CH₃) 1.38 (s, 3H, 1CH₃) 1.26 (t, *J* = 7.2 Hz, 6H, 2CH₃)
ESI(m/z) 539 (M+H⁺)

### (11-14) Preparation of diethyl 2-acetamido-2-(4-((4-(2-cyclopropyloxazol-4-yl)phenyl) thio)phenethyl)malonate

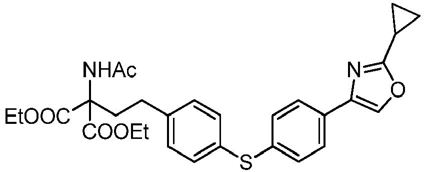

To a solution of diethyl 2-acetamido-2-(4-((4-(2-((cyclopropanecarbonyl)oxy) acetyl)phenyl)thio)phenethyl)malonate (1.0 g, 1.8 mmol) in xylene(20 mL) was added acetamide (0.53 g, 8.9 mmol) and 47%BF₃·Et₂O(0.18mL). The mixture was heated to reflux for 40h, then concentrated. The residue was diluted with EtOAc, washed with H₂O (three times) and brine, dried over Na₂SO₄, filtered and concentrated. The residue was purified by silica gel column chromatography to afford the title compound (0.74g) as light yellow oil.
¹HNMR(MERCURY 300 MHz, CDCl₃) δ 7.77 (s, 1H, 1ArH) 7.73 (d, *J* = 6.3 Hz, 2H, 2ArH) 7.42 (d, *J* = 5.7 Hz, 4H, 4ArH) 7.22-7.14 (m, 4H, 4ArH) 6.81 (brs, 1H, NH) 4.25-4.13 (m, 4H, 2CH₂) 2.69 (dd, *J* = 11.4 Hz, 6.9 Hz, 2H, 1CH₂) 2.48 (dd, *J* = 9.0 Hz, 5.1 Hz, 2H, 1CH₂) 2.03 (s, 3H, 1CH₃) 1.85-1.75 (m, 1H, 1CH)1.27 (t, *J* = 7.2 Hz, 6H, 2CH₃) 1.11-0.94 (m, 4H, 2CH₂)
ESI(m/z) 537 (M+H⁺)

### (11-15) Preparation of N-(1-hydroxy-2-(hydroxymethyl)-4-(4-((4-(2-methyloxazol -4-yl)phenyl)thio)phenyl)butan-2-yl)acetamide

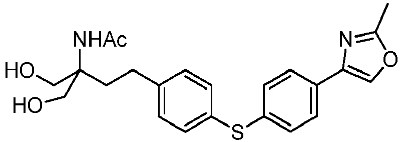

To a solution of diethyl 2-acetamido-2-(4-((4-(2-methyloxazol-4-yl)phenyl) thio)phenethyl)malonate (0.7 g, 1.4 mmol) in 95% EtOH (10 mL) was added K₂HPO₄ (2.5 g, 10.8 mmol) in distilled water (2.5 mL) and NaBH₄ (0.27 g, 7.1 mmol) in aq. 10% NaOH(1.8mL). The mixture was stirred for further 6h at room temperature and concentrated. The residue was extracted with EtOAc, washed with H₂O to neutral, dried over Na₂SO₄, filtered and concentrated. The residue was recrystallized with EtOAc to afford the title compound (0.36 g) as white powder solid.
¹HNMR(MERCURY 300 MHz, CDCl₃) δ 7.80 (s, 1H, 1ArH) 7.63 (d, *J* = 7.5 Hz, 2H, 2ArH) 7.32-7.28 (m, 4H, 4ArH) 7.16 (d, *J* = 7.2 Hz, 2H, 2ArH) 5.95 (brs, 1H, NH) 3.85 (d, *J* = 11.4 Hz, 2H, 1CH₂) 3.63 (d, *J* = 11.4 Hz, 2H, 1CH₂) 2.65 (dd, *J* = 11.4 Hz, 8.1 Hz, 2H, 1CH₂) 2.55 (s, 3H, 1CH₃) 2.04-1.94 (m, 5H, 1CH₂, 1CH₃)
ESI(m/z) 427 (M+H⁺)

### (11-16) Preparation of N-(4-(4-((4-(2-ethyloxazol-4-yl)phenyl)thio)phenyl) -1-hydroxy-2-(hydroxymethyl)butan-2-yl)acetamide

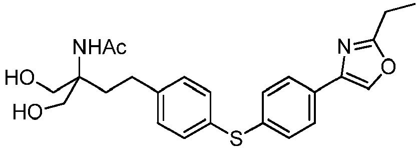

To a solution of diethyl 2-acetamido-2-(4-((4-(2-ethyloxazol-4-yl)phenyl) thio)phenethyl)malonate (0.74 g, 1.4 mmol) in 95% EtOH (10 mL) was added K₂HPO₄ (2.5 g, 11.1 mmol) in distilled water (2.5 mL) and NaBH₄ (0.27 g, 7.2 mmol) in aq. 10% NaOH(1.9mL). The mixture was stirred for further 6h at room temperature and concentrated. The residue was extracted with EtOAc, washed with H₂O to neutral, dried over Na₂SO₄, filtered and concentrated. The residue was recrystallized with EtOAc to afford the title compound (0.33 g) as white powder solid.
¹HNMR(MERCURY 300 MHz, CDCl₃) δ 7.80 (s, 1H, 1ArH) 7.64 (d, *J* = 7.8 Hz, 2H, 2ArH) 7.30 (d, *J* = 8.4 Hz, 4H, 4ArH) 7.15 (d, *J* = 7.5 Hz, 2H, 2ArH) 5.95 (brs, 1H, NH) 3.84 (d, *J* = 11.7 Hz, 2H, 1CH₂) 3.63 (d, *J* = 11.4 Hz, 2H, 1CH₂) 2.86 (q, J = 7.5 Hz, 2H, 1CH₂) 2.63 (dd, *J* = 11.4 Hz, 8.1 Hz, 2H, 1CH₂) 2.04-1.94 (m, 5H, 1CH₂, 1CH₃) 1.38 (t, J = 7.5 Hz, 3H, 1CH₃)
ESI(m/z) 441 (M+H⁺)

### (11-17) Preparation of N-(1-hydroxy-2-(hydroxymethyl)-4-(4-((4-(2-propyloxazol -4-yl)phenyl)thio)phenyl)butan-2-yl)acetamide

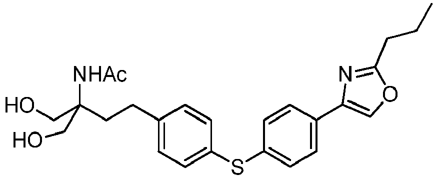

To a solution of diethyl 2-acetamido-2-(4-((4-(2-propyloxazol-4-yl)phenyl) thio)phenethyl)malonate (0.44 g, 0.82 mmol) in 95% EtOH (6 mL) was added K₂HPO₄ (1.5 g, 6.5 mmol) in distilled water (1.5 mL) and NaBH₄ (0.16 g, 4.2 mmol) in aq. 10% NaOH(1.1mL). The mixture was stirred for further 6h at room temperature and concentrated. The residue was extracted with EtOAc, washed with H₂O to neutral, dried over Na₂SO₄, filtered and concentrated, yielding the crude product as yellow oil.

The product was used in the next step without purification.

### (11-18) Preparation of N-(1-hydroxy-2-(hydroxymethyl)-4-(4-((4-(2-isopropyloxazol-4-yl)phenyl)thio)phenyl)butan-2-yl)acetamide

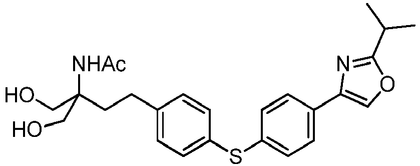

To a solution of diethyl 2-acetamido-2-(4-((4-(2-isoprooxazol-4-yl)phenyl) thio)phenethyl)malonate (0.6 g, 1.1 mmol) in 95% EtOH (8 mL) was added K₂HPO₄ (2 g, 8.8 mmol) in distilled water (2 mL) and NaBH₄ (0.22 g, 5.7 mmol) in aq. 10% NaOH(1.5mL). The mixture was stirred for further 6h at room temperature and concentrated. The residue was extracted with EtOAc, washed with H₂O to neutral, dried over Na₂SO₄, filtered and concentrated, yielding the crude product (0.44g) as yellow oil.

### (11-19) Preparation of N-(4-(4-((4-(2-cyclopropyloxazol-4-yl)phenyl)thio) phenyl)-1-hydroxy-2-(hydroxymethyl)butan-2-yl)acetamide

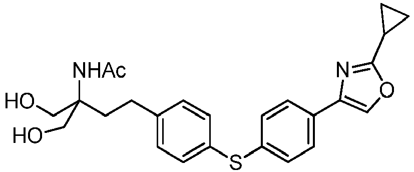

To a solution of diethyl 2-acetamido-2-(4-((4-(2-cycloprooxazol-4-yl)phenyl) thio)phenethyl)malonate (0.74 g, 1.38 mmol) in 95% EtOH (10 mL) was added K₂HPO₄ (2.5 g, 10.9 mmol) in distilled water (2.5 mL) and NaBH₄ (0.27 g, 7.1 mmol) in aq. 10% NaOH(1.8mL). The mixture was stirred for further 6h at room temperature and concentrated. The residue was extracted with EtOAc, washed with H₂O to neutral, dried over Na₂SO₄, filtered and concentrated, yielding the crude product (0.5g) as light yellow syrup.

### (11-20) Preparation of 2-amino-2-(4-((4-(2-methyloxazol-4-yl)phenyl)thio) phenethyl)propane-1,3-diol hydrochloride

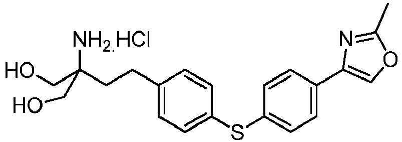

To a solution of *N*-(1-hydroxy-2-(hydroxymethyl)-4-(4-((4-(2-methyloxazol -4-yl)phenyl)thio)phenyl)butan-2-yl)acetamide (0.36 g, 0.85 mmol) in MeOH(10mL) was added NaOH(0.035 g, 0.88 mmol) and heated to reflux for 2h, then filtered to remove insoluble substance. The solution was added HCl-EtOH solution until pH=3-4 and concentrated. The residue was purified by silica gel column chromatography to afford the title compound (0.32g) as light yellow solid.
¹HNMR(MERCURY 300 MHz, CD₃OD) δ 8.22 (s, 1H, 1ArH) 7.54 (d, *J* = 8.1 Hz, 2H, 2ArH) 7.28 (d, *J* = 8.1 Hz, 2H, 2ArH) 7.19 (dd, *J* = 8.1 Hz, 2.4 Hz, 4H, 4ArH) 3.61 (s, 4H, 2CH₂) 2.64-2.58 (m, 2H, 1CH₂) 2.53 (s, 3H, 1CH₃) 1.91-1.85 (m, 2H, 1CH₂) ¹³CNMR (400 MHz, CD₃OD) δ 165.29, 142.84, 139.44, 139.19, 136.56, 133.90, 132.98, 130.93, 130.64, 128.36, 127.31, 62.48, 62.04, 34.42, 29.72, 13.47
ESI(m/z) 385 (M+H⁺) HRMS calcd. for C₂₁H₂₅N₂O₃S (M+H⁺) 385.1580, found 385.1579.

### (11-21) 2-amino-2-(4-((4-(2-ethyloxazol-4-yl)phenyl)thio)phenethyl)propane-1,3-diol hydrochloride

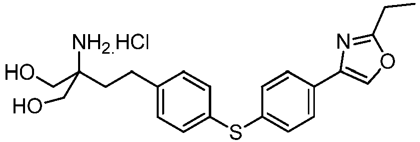

To a solution of *N*-(1-hydroxy-2-(hydroxymethyl)-4-(4-((4-(2-ethyloxazol -4-yl)phenyl)thio)phenyl)butan-2-yl)acetamide (0.33 g, 0.75 mmol) in MeOH(10mL) was added NaOH(0.03 g, 0.77 mmol) and heated to reflux for 2h, then filtered to remove insoluble substance. The solution was added HCl-EtOH solution until pH=3-4 and concentrated. The residue was purified by silica gel column chromatography to afford the title compound (0.19 g) as light yellow solid.
¹HNMR(MERCURY 300 MHz, CD₃OD) δ 8.34 (s, 1H, 1ArH) 7.53 (d, *J* = 7.5 Hz, 2H, 2ArH) 7.27 (d, *J* = 8.1 Hz, 2H, 2ArH) 7.19 (dd, *J* = 8.1 Hz, 2.4 Hz, 4H, 4ArH) 3.59 (s, 4H, 2CH₂) 2.95-2.89 (m, 2H, 1CH₂) 2.60-2.54 (m, 2H, 1CH₂) 1.87-1.83 (m, 2H, 1CH₂) 1.31 (t, *J =* 7.2 Hz, 3H, 1CH₃)
¹³CNMR (400 MHz, CD₃OD) δ 169.94, 143.15, 140.61, 137.69, 137.12, 134.28, 132.36, 130.74, 130.54, 127.57, 126.53, 62.46, 62.04, 34.38, 29.73, 22.18, 10.55 ESI(m/z) 399 (M+H⁺) HRMS calcd. for C₂₂H₂₇N₂O₃S (M+H⁺) 399.1737, found 399.1751.

### (11-22) 2-amino-2-(4-((4-(2-propyloxazol-4-yl)phenyl)thio)phenethyl)propane-1,3-diol hydrochloride

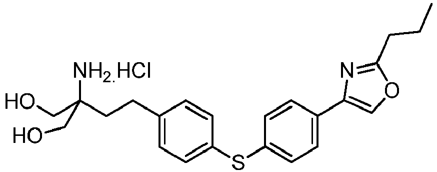

To a solution of *N*-(1-hydroxy-2-(hydroxymethyl)-4-(4-((4-(2-propyloxazol -4-yl)phenyl)thio)phenyl)butan-2-yl)acetamide (0.37 g, 0.82 mmol) in MeOH(10mL) was added NaOH(0.036 g, 0.9 mmol) and heated to reflux for 2h, then filtered to remove insoluble substance. The solution was added HCl-EtOH solution until pH=3-4 and concentrated. The residue was purified by silica gel column chromatography to afford the title compound (0.11 g) as light yellow solid.
¹HNMR(MERCURY 300 MHz, CD₃OD) δ 8.25 (s, 1H, 1ArH) 7.46 (d, *J* = 8.1 Hz, 2H, 2ArH) 7.19 (d, *J* = 7.8 Hz, 2H, 2ArH) 7.11 (d, *J* = 8.4 Hz, 2H, 2ArH) 7.08 (d, *J* = 8.4 Hz, 2H, 2ArH) 3.52 (s, 4H, 2CH₂) 2.82 (t, *J* = 7.5 Hz, 2H, 1CH₂) 2.55-2.50 (m, 2H, 1CH₂) 1.82-1.66 (m, 4H, 2CH₂) 0.89 (t, *J* = 7.2 Hz, 3H, 1CH₃)
¹³CNMR (400 MHz, CD₃OD) δ 169.02, 143.16, 140.64, 137.61, 137.23, 134.28, 132.28, 130.75, 130.50, 127.56, 126.43, 62.45, 62.03, 34.36, 30.23, 29.72, 20.91, 13.83
ESI(m/z) 413 (M+H⁺) HRMS calcd. for C₂₃H₂₉N₂O₃S (M+H⁺) 413.1893, found 413.1895

### (11-23) 2-amino-2-(4-((4-(2-isopropyloxazol-4-yl)phenyl)thio)phenethyl)propane-1,3-diol hydrochloride

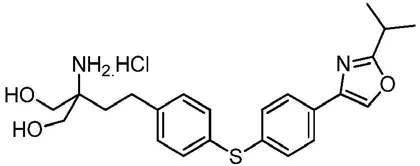

To a solution of *N*-(1-hydroxy-2-(hydroxymethyl)-4-(4-((4-(2-isopropyloxazol -4-yl)phenyl)thio)phenyl)butan-2-yl)acetamide (0.44 g, 0.97 mmol) in MeOH(10mL) was added NaOH(0.04 g, 1.0 mmol) and heated to reflux for 4h, then filtered to remove insoluble substance. The solution was added HCl-EtOH solution until pH=3-4 and concentrated. The residue was purified by silica gel column chromatography to afford the title compound (0.2 g) as light yellow solid.
¹HNMR(MERCURY 300 MHz, CD₃OD) δ 8.20 (s, 1H, 1ArH) 7.57 (d, *J* = 8.1 Hz, 2H, 2ArH) 7.27 (d, *J* = 8.1 Hz, 2H, 2ArH) 7.20 (d, *J* = 8.1 Hz, 4H, 4ArH) 3.61 (s, 4H, 2CH₂) 3.22-3.18 (m, 1H, 1CH) 2.64-2.58 (m, 2H, 1CH₂) 1.91-1.85 (m, 2H, 1CH₂) 1.33 (s, 3H, 1CH₃) 1.31 (s, 3H, 1CH₃)
¹³CNMR (400 MHz, CD₃OD) δ 171.93, 142.74, 139.42, 139.08, 136.14, 133.79, 133.18, 131.00, 130.60, 128.97, 127.48, 62.48, 62.04, 34.43, 29.74, 20.35 ESI(m/z) 413 (M+H⁺) HRMS calcd. for C₂₃H₂₉N₂O₃S (M+H⁺) 413.1893, found 413.1879

### (11-24) 2-amino-2-(4-((4-(2-cyclopropyloxazol-4-yl)phenyl)thio)phenethyl)propane-1,3-diol hydrochloride

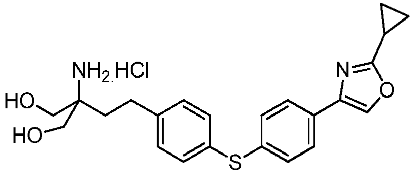

To a solution of *N*-(1-hydroxy-2-(hydroxymethyl)-4-(4-((4-(2-cyclopropyloxazol -4-yl)phenyl)thio)phenyl)butan-2-yl)acetamide (0.5 g, 1.1 mmol) in MeOH(10mL) was added NaOH(0.05 g, 1.2 mmol) and heated to reflux for 2h, then filtered to remove insoluble substance. The solution was added HCl-EtOH solution until pH=3-4 and concentrated. The residue was purified by silica gel column chromatography to afford the title compound (0.35 g) as light yellow solid.
¹HNMR(MERCURY 300 MHz, CD₃OD) δ 8.17 (s, 1H, 1ArH) 7.55 (d, *J* = 8.1 Hz, 2H, 2ArH) 7.29 (d, *J* = 7.8 Hz, 2H, 2ArH) 7.20 (dd, *J* = 8.4 Hz, 2.4 Hz, 4H, 4ArH) 3.62 (s, 4H, 2CH₂) 2.65-2.59 (m, 2H, 1CH₂) 2.28-2.18 (m, 1H, 1CH) 1.91-1.86 (m, 2H, 1CH₂) 1.21-1.18 (m, 4H, 2CH₂)
¹³CNMR (400 MHz, CD₃OD) δ 169.63, 142.89, 139.70, 138.67, 135.82, 133.99, 132.90, 130.84, 130.65, 127.90, 127.40, 62.48, 62.04, 34.43, 29.73, 9.83, 9.48 ESI(m/z) 411 (M+H⁺) HRMS calcd. for C₂₃H₂₇N₂O₃S (M+H⁺) 411.1737, found 411.1736

### Pharmacology Experiments

### I 1.1 Effect of S1P₁ receptor agonists on rat vein blood lymphocyte counts

### 1.2 Materials

**Preparation of drugs:** 10 mg of drugs were measured and placed in a mortar. After 4mL of 5‰ CMC-Na were added, homogenous suspension was prepared in the mortar (one drop of twain-80 was added to help dissolving). Dosage: 10mg/Kg, dose volume: 0.4mL/100g, intragastric administration.

**Experimental animals:** SD rats, male, clean grade, provided by Beijing Weitonglihua experimental animals technology limited company, Certification number: SCXK (Jing) 2006-0009; Wista rats, male, clean grade, provided by breeding ground of institute of experimental animals, Chinese Academy of Medical Sciences, Certification number: SCXK (Jing) 2005-0013. Every medicated group was assigned three rats.

**Apparatus:** Japan Guangdianwufenlei automatic hematology analyzer, type: 7222K, provided by Beijing Xiehejianhao medical technology development limited company (paid service). Diluent: DH-640, provided by Shanghai Donghu biology medical limited company, batch number: 081225.

### 1.3 Methods

After the experimental animals stable in the clean environment for 24h, 10 µL of blood was withdrawn via tail vein and diluted into 2mL of diluents quickly. The peripheral blood lymphocyte counts were assessed using MEK-7222K hematology analyzer. Then the prepared drugs were administered intragastrically to the animals. The blood was withdrawn at the time 1h, 2h, 4h, 8h, 12h and 24h after administration and the peripheral blood lymphocyte counts were assessed using MEK-7222K hematology analyzer. The animals were killed after 24h. See results in the table below.

### II Effect of S1P₁ receptor agonists on rat heart rate

2.1 Experimental animals: SD rats, purchased from Weitonglihua. Weight: 200-240g, male. Every test group was assigned three rats. The normal rats were set as control group and determined as the medicated group parallelly for three times. The positive drug FTY720 was repeated three times.

2.2 Apparatus: Intelligent non-invasive blood pressure measurement meter (Softron, Japan).

### 2.3 methods:

(1)Preparation: The drug was dissolved to reach a concentration of 2.5mg/mL.
(2) Experimental procedure:
   ① Determination of rat heart rate before administration. Repeat for three times.
   ②Administration: intragastric administration after SD rats weighed.
   ③Determination of rat heart rate at the time 0.5h, 1h, 3h, 6h, 8h and 24h after administration. Repeat for three times

### 2.4 results

Every drug was tested with three SD rats parallelly. Average value of heart rate was obtained (Table 1). Delta% of heart rate = (the lowest heart rate postdose - the heart rate pre-administration)/the heart rate pre-administration, reflects the influence of drugs on rat heart rate. See results in the table below.

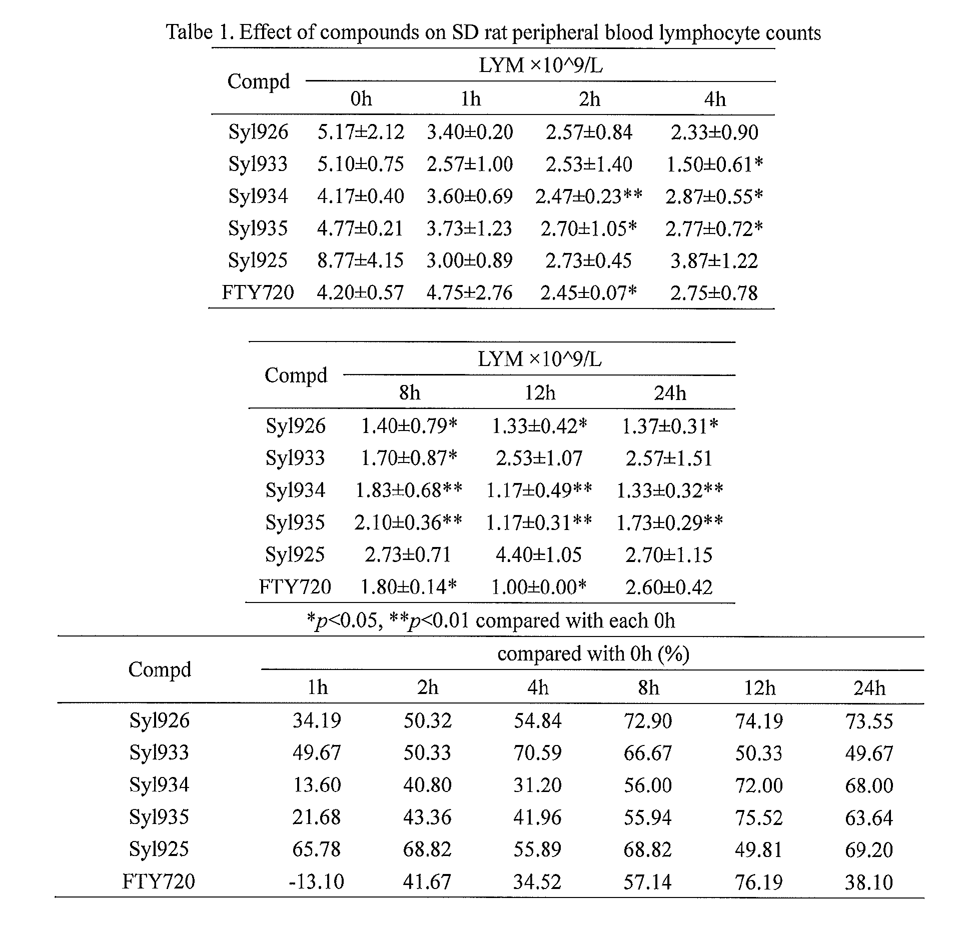

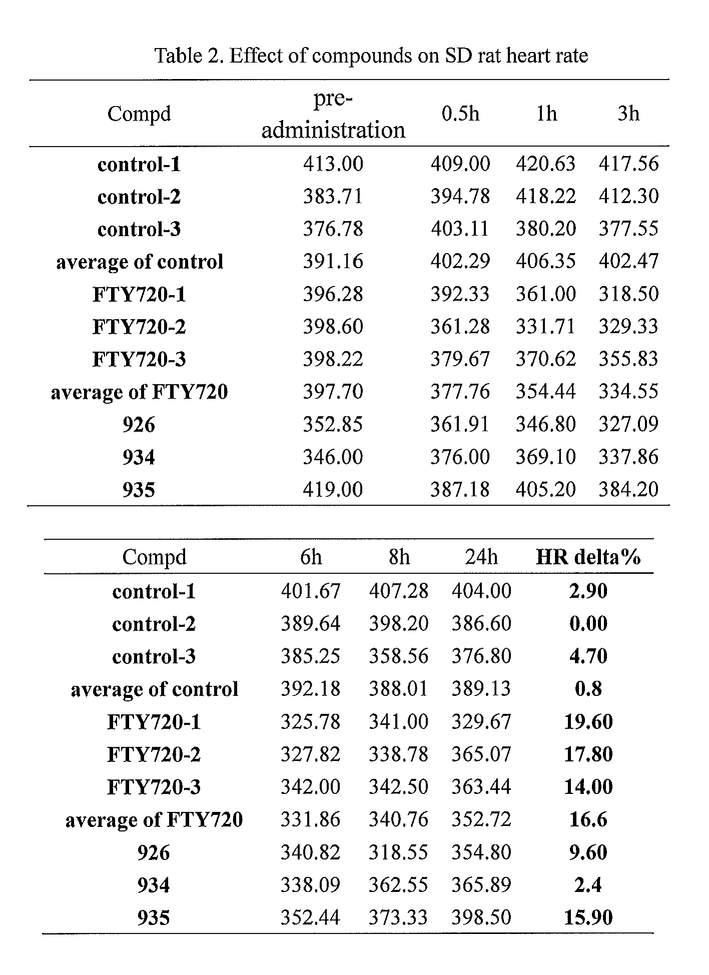

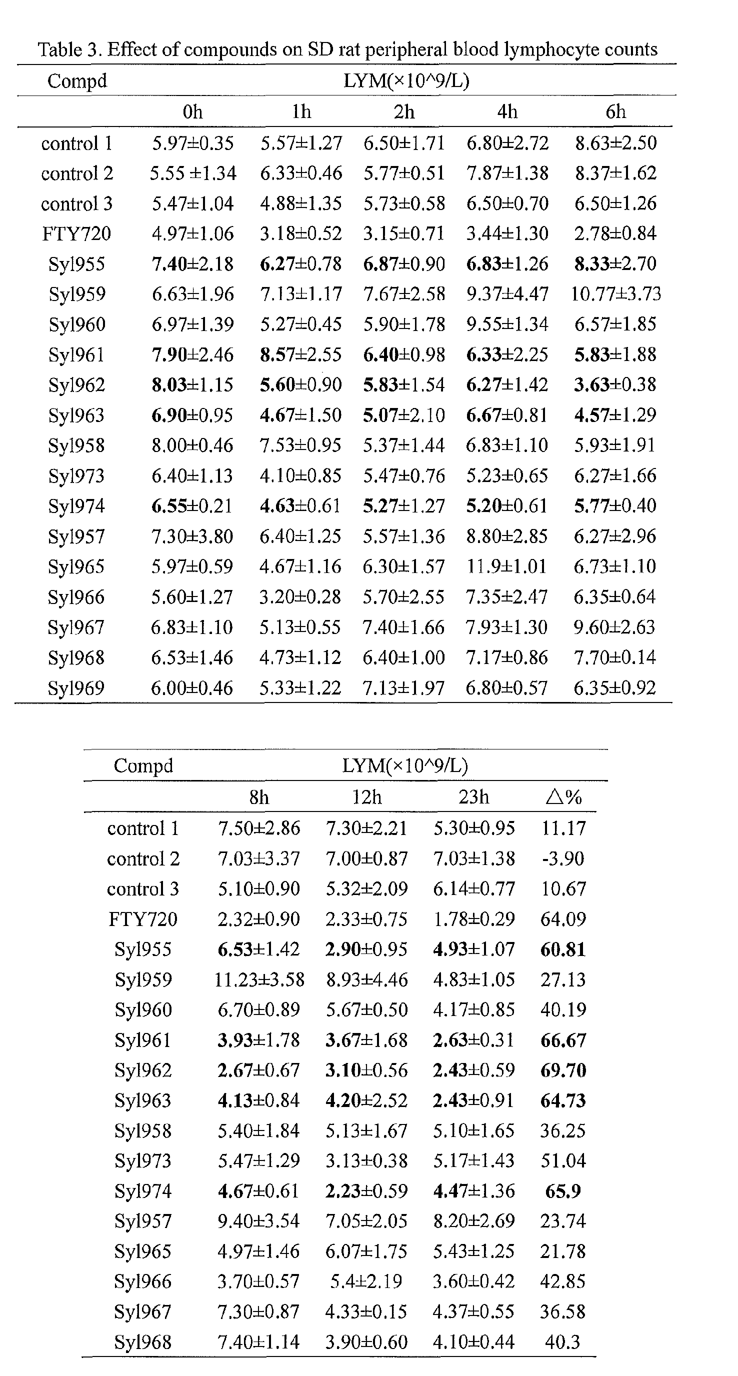

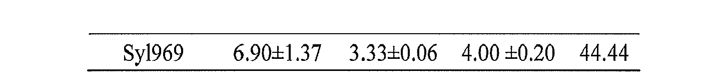

**Table 4. Effect of compounds on SD rat heart rate**

| Compd | **0** | **0**.**5h** | **1h** | **3h** | **6h** | **8h** | **24h** | **Δ%** |
|---|---|---|---|---|---|---|---|---|
| **control1** | 364.75 | 376.23 | 394.63 | 379.75 | 345.33 | 378.09 | 340.83 | 6.12 |
| **control 2** | 369.50 | 388.54 | 390.60 | 350.50 | 367.45 | 378.67 | 348.00 | 5.81 |
| **control 3** | 393.33 | 414.67 | 417.38 | 357.88 | 369.25 | 397.78 | 360.91 | 6.12 |
| **FTY720** | 379.42 | 356.33 | 319.22 | 288.58 | 280.13 | 308.08 | 323.25 | 26.16 |
| **955** | 428.63 | 373.00 | 384.75 | 338.25 | 335.50 | 351.50 | 353.90 | 21.72 |
| **961** | 389.75 | 382.56 | 364.33 | 379.45 | 344.36 | 363.08 | 363.27 | 11.64 |
| **962** | 390.00 | 397.13 | 374.88 | 358.58 | 343.63 | 337.00 | 331.89 | 13.58 |
| **963** | 395.50 | 388.38 | 378.36 | 377.36 | 366.88 | 362.50 | 369.05 | 4.58 |
| **974** | 381.50 | 382.63 | 388.00 | 396.19 | 368.56 | 364.11 | 363.13 | 4.81 |

| | | | | | | | | |
|---|---|---|---|---|---|---|---|---|
| Δ% = the percentage of change of rat heart rate before and after administration | | | | | | | | |

**Table 5. Effect of compound 932 on SD rat peripheral blood lymphocyte counts**

| Compd | LYM × 10^9/L | | | | | | Δ% |
|---|---|---|---|---|---|---|---|
| | 0 h | 1 h | 4 h | 8 h | 12 h | 24 h | |
| Control | 4.87±1.35 | 4.55±1.48 | 5.20±1.68 | 4.90±1.22 | 5.10±1.41 | 6.10±0.28 | 6.51 |
| FTY720 | 3.80±0.14 | 2.30±0.85 | 1.20±0.36 | 1.07±0.50 | 1.27±0.23 | 1.60±0.57 | 71.93 |
| SYL932 | 4.30±0.42 | 4.40±0.42 | 1.80±0.85 | 2.00±0.14 | 2.70±0.99 | 1.40±0.42 | 67.44 |

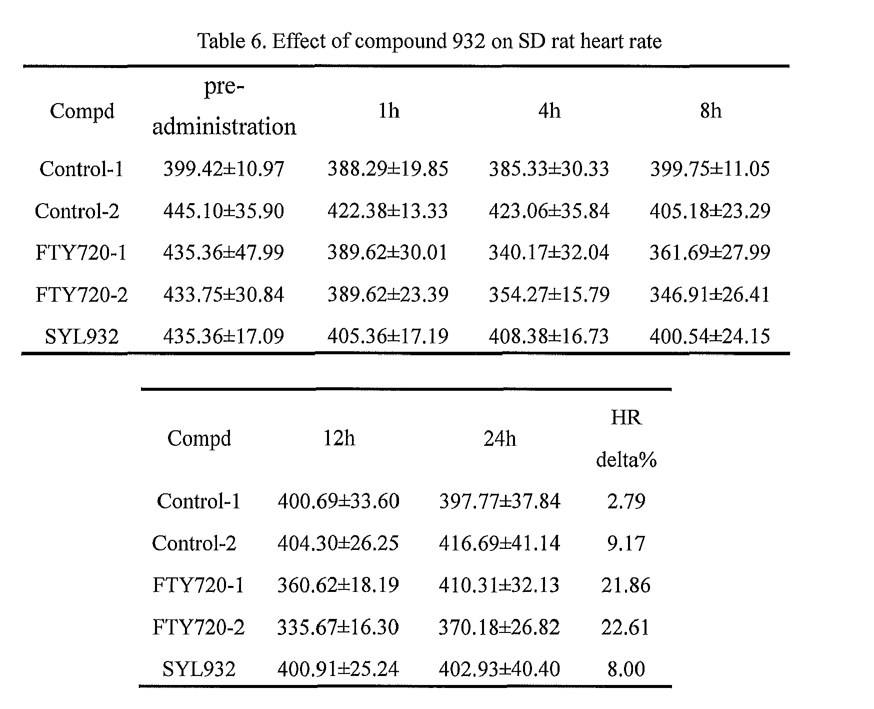

**Table 7. Effect of compound 927 on SD rat peripheral blood lymphocyte counts**

| Compd | LYM × 10^9/L | | | | | | Δ% |
|---|---|---|---|---|---|---|---|
| | 0 h | 1 h | 4h | 8h | 12 h | 24 h | |
| Control | 4.87±1.35 | 4.55±1.48 | 5.20±1.68 | 4.90±1.22 | 5.10±1.41 | 6.10±0.28 | 6.51 |
| FTY720 | 3.80±0.14 | 2.30±0.85 | 1.20±0.36 | 1.07±0.50 | 1.27±0.23 | 1.60±0.57 | 71.93 |
| SYL927 | 2.80±1.27 | 1.70±0.28 | 2.55±0.92 | 1.80±0.99 | 1.40±0.46 | 2.00±0.66 | 50.00 |

**Table 8. Effect of compound 930 on SD rat peripheral blood lymphocyte counts**

| Compd | LYM × 10^9/L | | | | | | Δ% |
|---|---|---|---|---|---|---|---|
| | 0 h | 1 h | 4 h | 8 h | 12 h | 24 h | |
| Control | 5.98±1.89 | 5.00±2.38 | 5.02±1.84 | 5.25±3.32 | 4.90±2.06 | 5.00±2.50 | 18.1 |
| FTY720 | 4.20±0.57 | 4.75±2.76 | 2.75±0.78 | 1.80±0.14 | 1.00±0.00 | 2.60±0.42 | 76.19 |
| SYL930 | 4.97±0.85 | 2.67±0.29 | 2.60±0.95 | 1.20±0.10 | 1.27±0.67 | 1.67±0.31 | 75.84 |

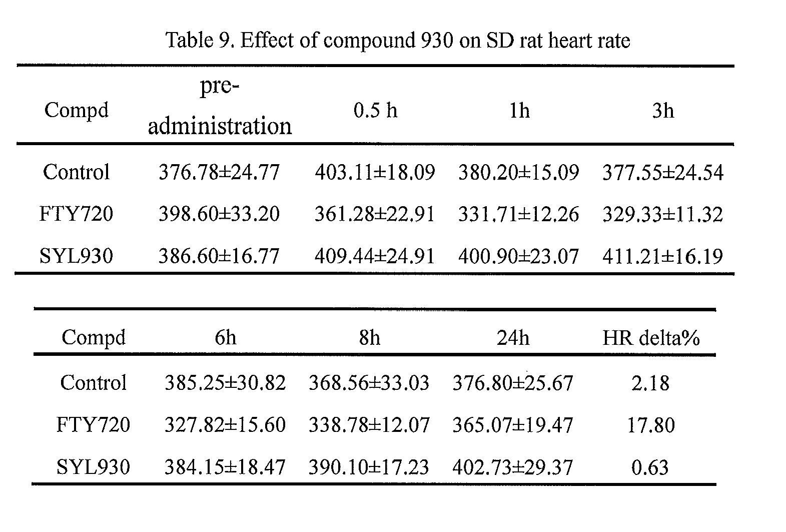

## Claims

1. A compound represented by the general formula (I), or pharmaceutically acceptable salts or esters thereof, 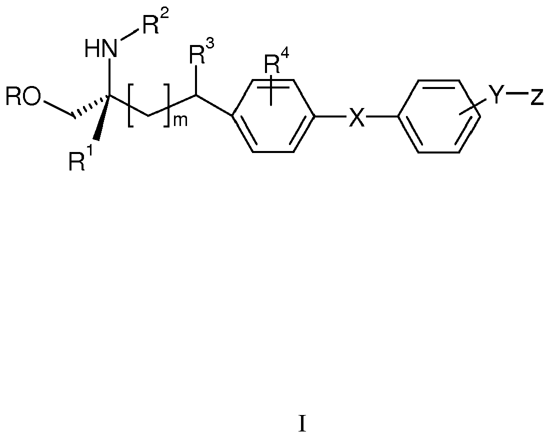 wherein R is selected from the group consisting of hydrogen, C1-6 alkyl, C1-6 acyl group, sulfonate group, -P(=O)(OR')(OR"), wherein OR' and OR" may be the same or different, and R' and R" are independently selected from hydrogen, C1-6 alkyl, C1-6 acyl group;
R¹ is selected from the group consisting of hydrogen, substituted or unsubstituted C1-6 alkyl group, and the substituents are selected from the group consisting of halogen, carbonyl group, hydroxyl group, mercapto group, cyano group, amino group and sulfonate group;
R² is selected from the group consisting of hydrogen, substituted or unsubstituted C1-8 alkoxy group, and the substituents are selected from the group consisting of halogen, carbonyl, hydroxy, mercapto, cyano, amino and phenyl;
R³ is selected from the group consisting of hydrogen or hydroxy;
M is an integer selected from 0 to 4;
R⁴ is selected from the group consisting of hydrogen, hydroxy, mercapto, amino, aldehyde, carboxyl, carbamoyl, halogen, nitro, cyano, C1-6 alkyl, C1-6 alkoxy, C1-6 alkylamino and C1-6 alkylamino group, wherein the alkylamino group includes mono- and bis-alkylamino, C1-6 alkoxy C1-6 alkyl group, C1-6 acyl, C1-6 acyloxy, C1-6 acylamino group, C1-6 haloalkyl and C2-6 olefins;
X is selected from the group consisting of oxygen, sulfur or a single bond, such that when X comprises a single bond the first phenyl group is directly linked to the second phenyl group;
when X comprises oxygen or sulfur; Y is selected from the group consisting of C0-8 alkyl group, a C1-8 alkoxy, C2-8 olefin, a five-or six-membered aryl, five-or six-membered heterocycle ring containing 1, 2 or 3 heteroatoms, the heteroatoms can be the same or different and selected from the group consisting of N, O, and S; such that when Y comprises the C0 alkyl group, Z is directly connected to the benzene ring;
when X comprises a single bond; Y is selected from the group consisting of a five-or six-membered aryl, five-or six-membered heterocyclic ring containing 1, 2 or 3 heteroatoms, the heteroatoms can be the same or different and selected from the group consisting of N, O, and S; such that when Y comprises the C0 alkyl group, Z is directly connected to the benzene ring; and Z is selected from the group consisting of hydrogen, hydroxy, mercapto, amino, aldehyde, carboxyl, carbamoyl, halogen, nitro, cyano, C1-6 alkyl, C1-6 alkoxy, C1-6 alkylthio, C1-6 alkylamino group wherein the alkylamino group includes mono-and bis-alkylamino, C1-6 alkoxy, C1-6 alkyl group, C1-6 acyl, C1-6 acyloxy, C1-6 acylamino group, C1-6 haloalkyl and C2-6 olefins.

2. The compound, or pharmaceutically acceptable salts or esters thereof according to claim 1, **characterized in that** the compound is represented by the general formula IA 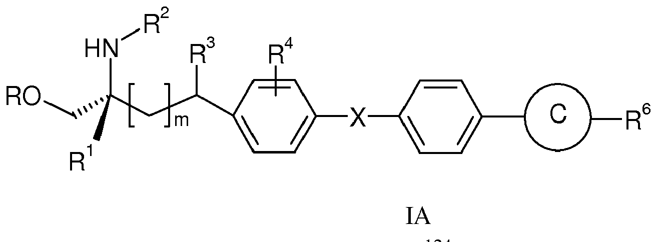 wherein R is selected from hydrogen, C1-4 alkyl, C1-4 acyl group, sulfonate group, -P(=O)(OR')(OR"), wherein the OR' and OR" may be the same or different, and R' and R" are independently selected from hydrogen, C1-4 alkyl, C1-4 acyl group;
R¹ is selected from the group consisting of hydrogen, substituted or unsubstituted C1-4 alkyl group, and the substituents are selected from the group consisting of halogen, carbonyl group, hydroxyl group, mercapto group, cyano group, amino group and sulfonate group;
R² is selected from the group consisting of hydrogen, substituted or unsubstituted C1-6 alkoxy group, and the substituents are selected from the group consisting of halogen, carbonyl, hydroxy, mercapto, cyano, amino and phenyl;
R³ is selected from the group consisting of hydrogen or hydroxy;
M is an integer selected from 1 to 3;
R⁴ is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino and C1-4 alkylamino group, wherein the alkylamino group includes mono- and bis-alkylamino, C1-4 alkoxy C1-4 alkyl group, C1-4 acyl, C1-4 acyloxy, C1-4 acylamino group, C1-4 haloalkyl and C2-4 olefins;
X is selected from the group consisting of oxygen and sulfur;
C ring is selected from 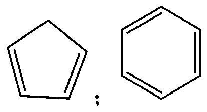 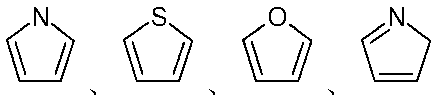 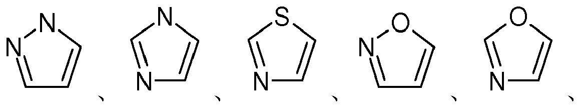 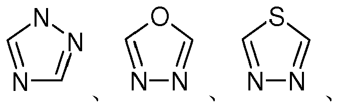 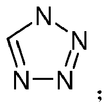 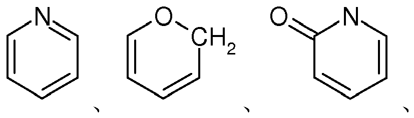 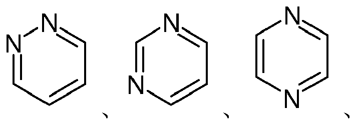 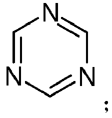 and
R⁶ is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino, C1-4 alkylamino group, wherein the alkylamino group includes mono-and bis-alkylamino, C1-4 alkoxy, C1-4 alkyl group, C1-4 acyl, C1-4 acyloxy, C1-4 acylamino group, C1-4 haloalkyl and C2-4 olefins.

3. The compound, or pharmaceutically acceptable salts or esters thereof according to claim 1, **characterized in that** the compound is represented by the general formula IB 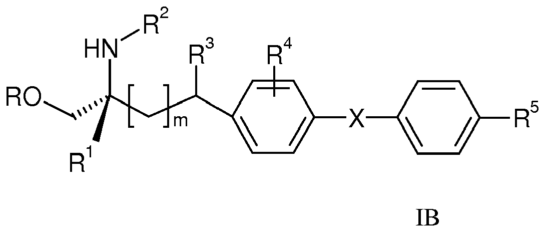 wherein R is selected from hydrogen, C1-4 alkyl, C1-4 acyl group, sulfonate group, -P(=O)(OR')(OR"), wherein the OR' and OR" may be the same or different, and R' and R" are independently selected from hydrogen, C1-4 alkyl, C1-4 acyl group;
R¹ is selected from the group consisting of hydrogen, substituted or unsubstituted C1-4 alkyl group, and the substituents are selected from the group consisting of halogen, carbonyl group, hydroxyl group, mercapto group, cyano group, amino group and sulfonate group;
R² is selected from the group consisting of hydrogen, substituted or unsubstituted C1-6 alkoxy group, and the substituents are selected from the group consisting of halogen, carbonyl, hydroxy, mercapto, cyano, amino and phenyl;
R³ is selected from the group consisting of hydrogen or hydroxy;
M is an integer selected from 1 to 3;
R⁴ is selected from the group consisting of hydrogen, halogen, hydroxy, C1-4 alkyl, C1-4 alkoxy, C1-4 acyl, C1-4 acyloxy, C1-4 alkylthio, amino, C1-4 alkoxy group and alkylamino group, wherein the alkylamino group includes mono- and bis-alkylamino, C1-4 acylamino group, C1-4 haloalkyl, mercapto, C1-4 alkylthio and C2-4 olefins;
X is selected from the group consisting of oxygen and sulfur; and
R⁵ is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino and C1-4 alkylamino group, wherein the alkylamino group includes mono- and bis-alkylamino, C1-4 alkoxy, C1-4 alkyl group, C1-4 acyl, C1-4 acyloxy, C1-4 acylamino group, C1-4 haloalkyl or C2-4 olefins;

4. The compound, or pharmaceutically acceptable salts and esters thereof according to claim 1, **characterized in that** the compound is represented by the general formula IC 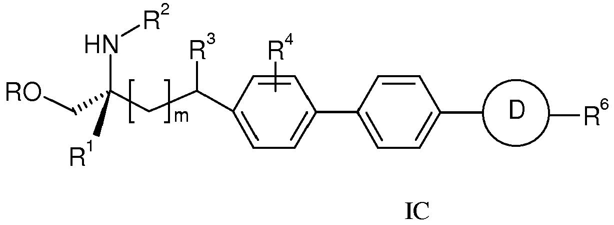 wherein R is selected from hydrogen, C1-4 alkyl, C1-4 acyl group, sulfonate group, -P(=O)(OR')(OR"), wherein the OR' and OR" may be the same or different, and R' and R" are independently selected from hydrogen, C1-4 alkyl, C1-4 acyl group;
R¹ is selected from the group consisting of hydrogen and substituted or unsubstituted C1-4 alkyl group, and the substituents are selected from the group consisting of halogen, carbonyl group, hydroxyl group, mercapto group, cyano group, amino group and sulfonate group;
R² is selected from the group consisting of hydrogen, substituted or unsubstituted C1-6 alkoxy group, and the substituents are selected from the group consisting of halogen, carbonyl, hydroxy, mercapto, cyano, amino and phenyl;
R³ is selected from the group consisting of hydrogen or hydroxy;
m is an integer selected from 1 to 3;
R⁴ is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino and C1-4 alkylamino group, wherein the alkylamino group includes mono- and bis-alkylamino, C1-4 alkoxy, C1-4 alkyl group, C1-4 acyl, C1-4 acyloxy, C1-4 acylamino group, C1-4 haloalkyl or C2-4 olefins;
D ring is selected from 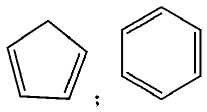 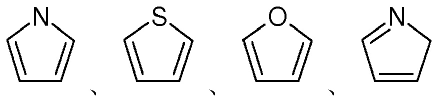 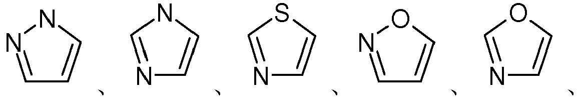 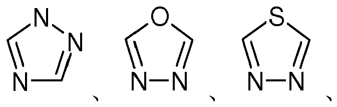 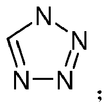 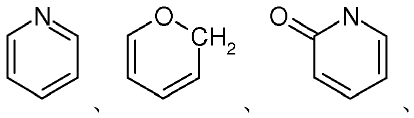 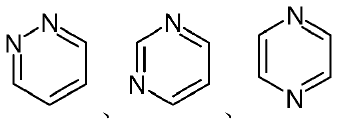 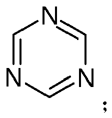 and
R⁶ is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino and C1-4 alkylamino group, wherein the alkylamino group includes mono- and bis-alkylamino, C1-4 alkoxy C1-4 alkyl group, C1-4 acyl, C1-4 acyloxy, C1-4 acylamino group, C1-4 haloalkyl or C2-4 olefins.

5. The compound, or pharmaceutically acceptable salts or esters thereof according to claim 1, **characterized in that** the compound is represented by one of the following general formulae: 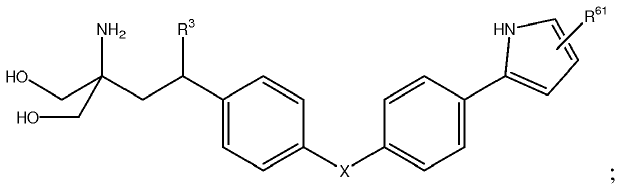 wherein R³ is selected from the group consisting of hydrogen or hydroxy;
X is selected from the group consisting of oxygen and sulfur; and
R⁶¹ is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino and C1-4 alkylamino group, wherein the alkylamino group includes mono- and bis-alkylamino, C1-4 alkoxy, C1-4 alkyl group, C1-4 acyl, C1-4 acyloxy, C1-4 acylamino group, C1-4 haloalkyl and C2-4 olefins; 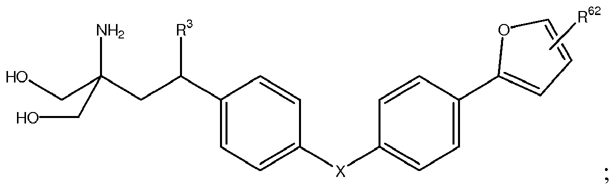 wherein R³ is selected from the group consisting of hydrogen or hydroxy;
X is selected from the group consisting of oxygen and sulfur; and
R⁶² is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino and C1-4 alkylamino group, wherein the alkylamino group includes mono- and bis-alkylamino, C1-4 alkoxy C1-4 alkyl group, C1-4 acyl, C1-4 acyloxy, C1-4 acylamino group, C1-4 haloalkyl and C2-4 olefins; 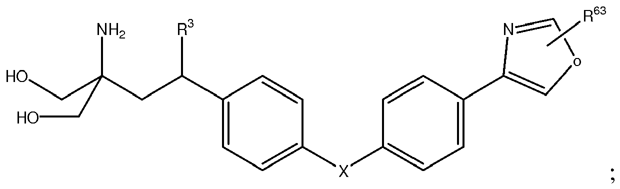 wherein R³ is selected from the group consisting of hydrogen or hydroxy;
X is selected from the group consisting of oxygen and sulfur; and
R⁶³ is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino and C1-4 alkylamino group, wherein the alkylamino group includes mono- and bis-alkylamino, C1-4 alkoxy, C1-4 alkyl group, C1-4 acyl, C1-4 acyloxy, C1-4 acylamino group, C1-4 haloalkyl and C2-4 olefins; 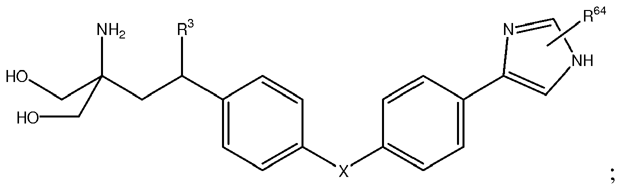 wherein R³ is selected from the group consisting of hydrogen or hydroxy;
X is selected from the group consisting of oxygen and sulfur; and
R⁶⁴ is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino and C1-4 alkylamino group, wherein the alkylamino group includes mono- and bis-alkylamino, C1-4 alkoxy, C1-4 alkyl group, C1-4 acyl, C1-4 acyloxy, C1-4 acylamino group, C1-4 haloalkyl and C2-4 olefins; 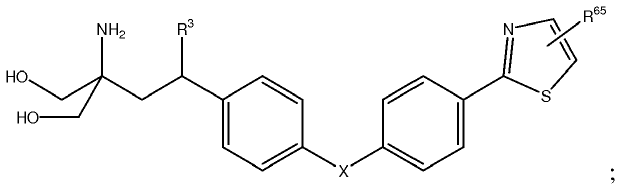 wherein R³ is selected from the group consisting of hydrogen or hydroxy;
X is selected from the group consisting of oxygen and sulfur; and
R⁶⁵ is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino and C1-4 alkylamino group, wherein the alkylamino group includes mono- and bis-alkylamino, C1-4 alkoxy C1-4 alkyl group, C1-4 acyl, C1-4 acyloxy, C1-4 acylamino group, C1-4 haloalkyl and C2-4 olefins; or 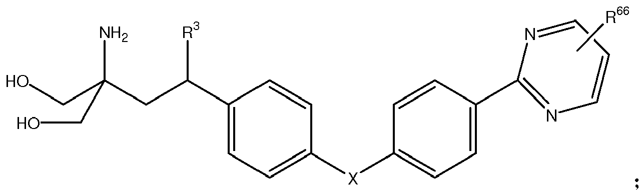 wherein R³ is selected from the group consisting of hydrogen or hydroxy;
X is selected from the group consisting of oxygen and sulfur; and
R⁶⁶ is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino and C1-4 alkylamino group, wherein the alkylamino group includes mono- and bis-alkylamino, C1-4 alkoxy, C1-4 alkyl group, C1-4 acyl, C1-4 acyloxy, C1-4 acylamino group, C1-4 haloalkyl and C2-4 olefins.

6. The compound, or pharmaceutically acceptable salts or esters thereof according to claim 5, **characterized in that** the compound is represented by one of the following general formulae: 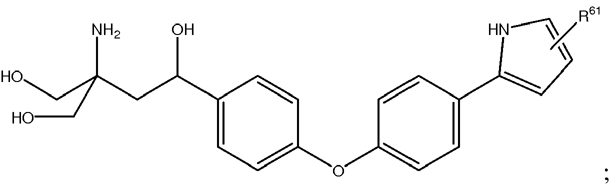 wherein R⁶¹ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 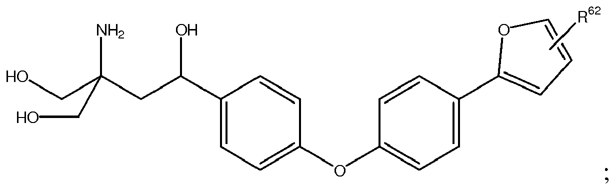 wherein R⁶² is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 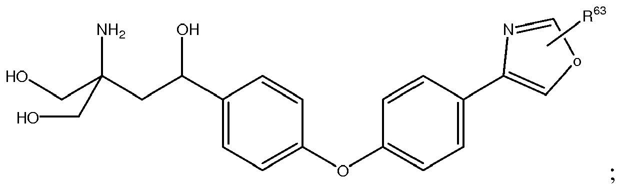 wherein R⁶³ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 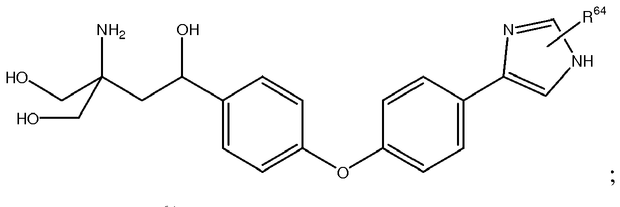 wherein R⁶⁴ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 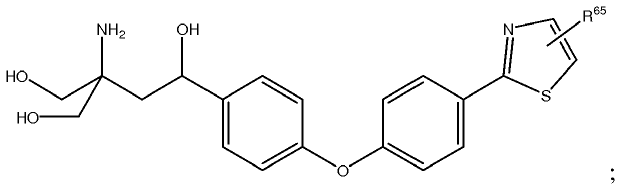 wherein R⁶⁵ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; or 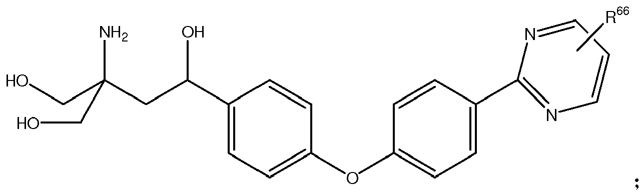 wherein R⁶⁶ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group amino group, tert-butyl amino group, vinyl group, propenyl, allyl, butenyl.

7. The compound, or pharmaceutically acceptable salts or esters thereof according to claim 5, **characterized in that** the compound is represented by one of the following general formulae: 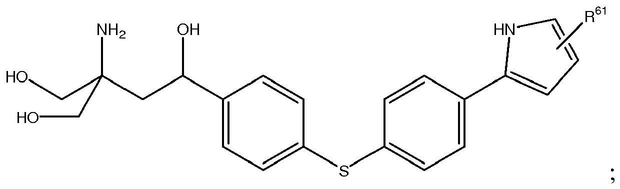 wherein R⁶¹ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 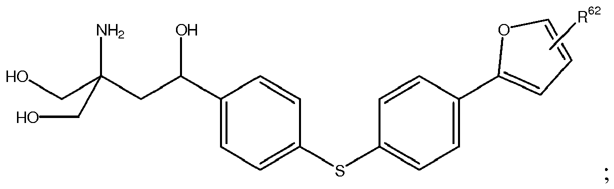 wherein R⁶² is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 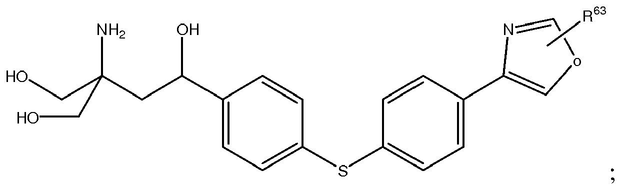 wherein R⁶³ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 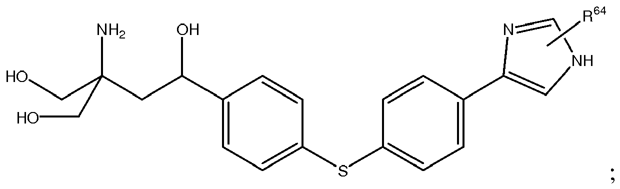 wherein R⁶⁴ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 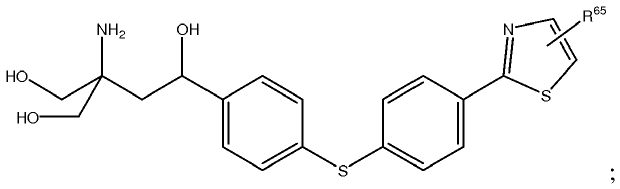 wherein R⁶⁵ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; or 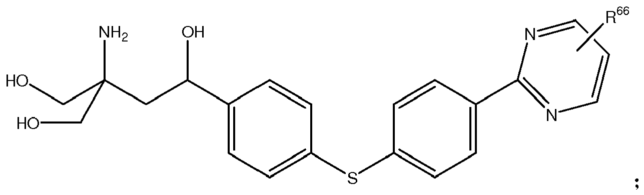 wherein R⁶⁶ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl.

8. The compound, or pharmaceutically acceptable salts and esters thereof according to claim 5, **characterized in that** the compound is represented by one of the following general formulae: 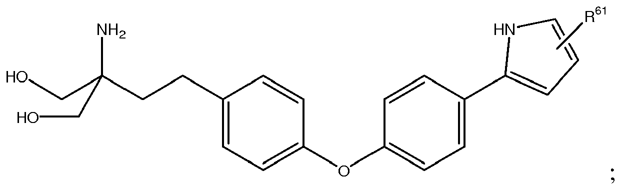 wherein R⁶¹ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 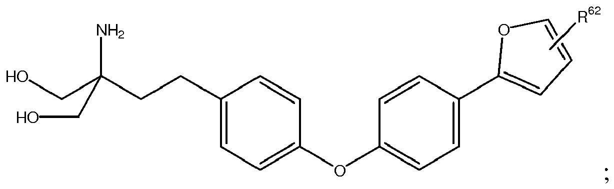 wherein R⁶² is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 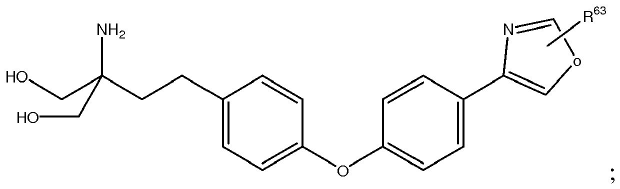 wherein R⁶³ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 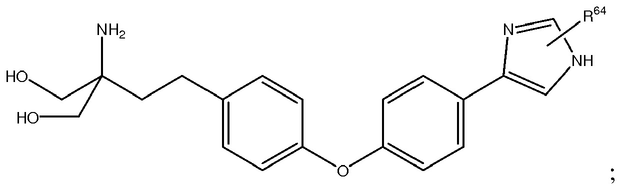 wherein R⁶⁴ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 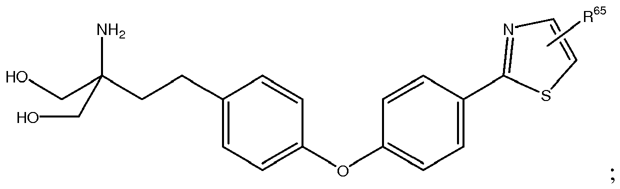 wherein R⁶⁵ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; or 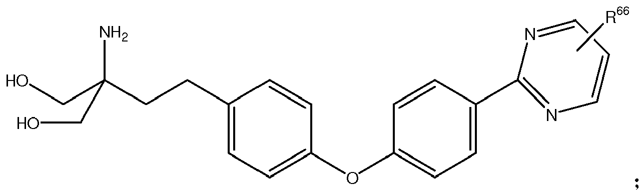 wherein R⁶⁶ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl.

9. The compound, or pharmaceutically acceptable salts or esters thereof according to claim 5, **characterized in that** the compound is represented by one of the follow general formulae: 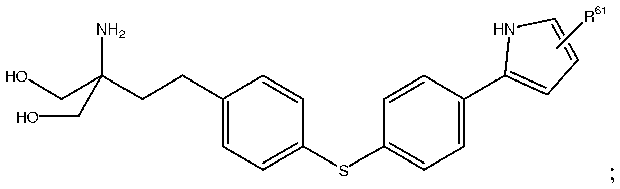 wherein R⁶¹ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 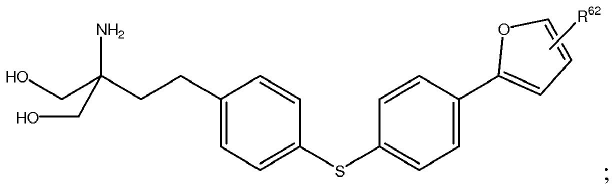 wherein R⁶² is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 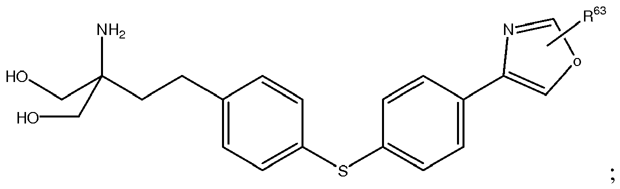 wherein R⁶³ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 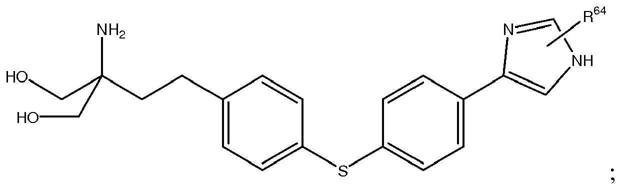 wherein R⁶⁴ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 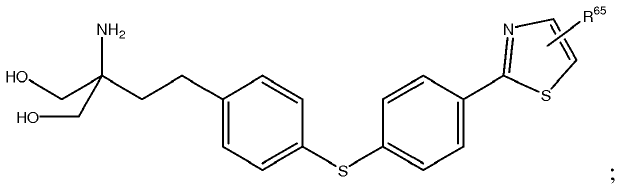 R⁶⁵ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; or 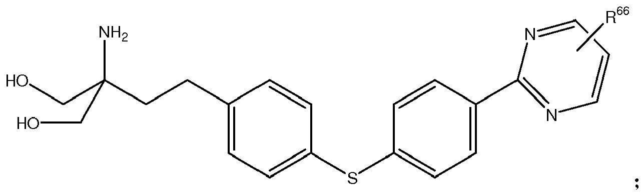 wherein R⁶⁶ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl.

10. The compound, or pharmaceutically acceptable salts or esters thereof according to claim 3, **characterized in that** the compound is represented by the following general formula: 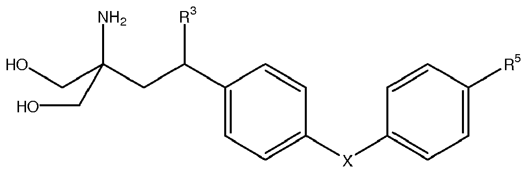 wherein R3 is selected from the group consisting of hydrogen or hydroxy; and
R⁵ is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino and C1-4 alkylamino group, wherein the alkylamino group includes mono- and bis-alkylamino, C1-4 alkoxy C1-4 alkyl group, C1-4 acyl, C1-4 acyloxy, C1-4 acylamino group, C1-4 haloalkyl or C2-4 olefins.

11. The compound, or pharmaceutically acceptable salts or esters thereof according to claim 10, **characterized in that** the compound is represented by one of the following general formulae: 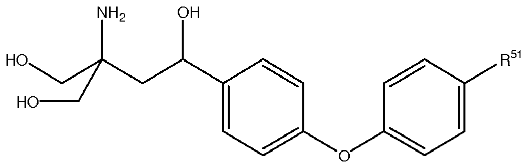 wherein R⁵¹ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 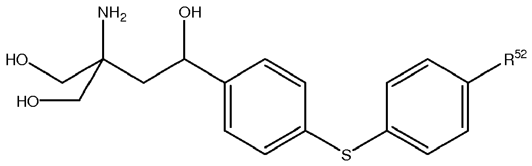 wherein R⁵² is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 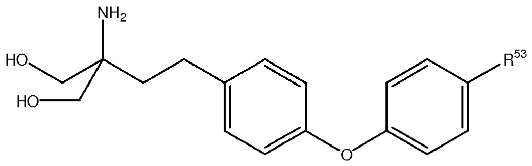 wherein R⁵³ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; or 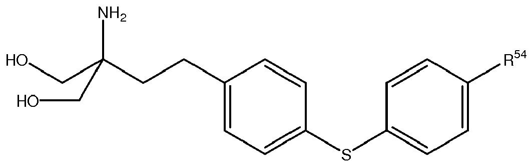 wherein R⁵⁴ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl.

12. The compound, or pharmaceutically acceptable salts or esters thereof according to claim 4, **characterized in that** the compound is represented by one of the following general formulae: 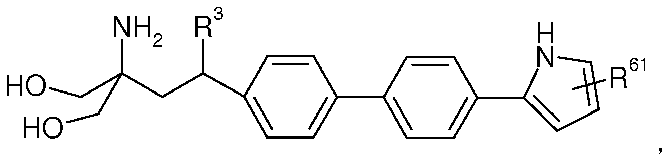 wherein R³ is selected from the group consisting of hydrogen or hydroxy; and
R⁶¹ is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino and C1-4 alkylamino group, wherein the alkylamino group includes mono- and bis-alkylamino, C1-4 alkoxy C1-4 alkyl group, C1-4 acyl, C1-4 acyloxy, C1-4 acylamino group, C1-4 haloalkyl or C2-4 olefins; 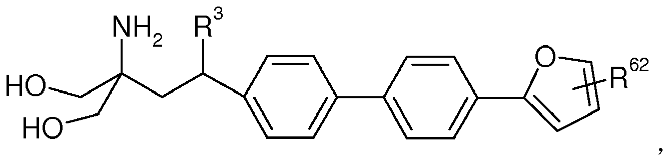 wherein R³ is selected from the group consisting of hydrogen or hydroxy; and
R⁶² is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino and C1-4 alkylamino group, wherein the alkylamino group includes mono- and bis-alkylamino, C1-4 alkoxy C1-4 alkyl group, C1-4 acyl, C1-4 acyloxy, C1-4 acylamino group, C1-4 haloalkyl or C2-4 olefins; 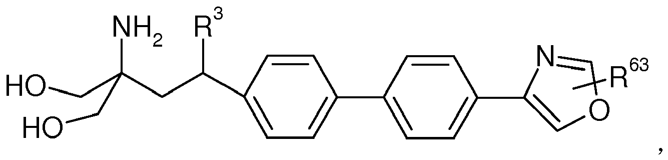 wherein R³ is selected from the group consisting of hydrogen or hydroxy; and
R⁶³ is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino and C1-4 alkylamino group, wherein the alkylamino group includes mono- and bis-alkylamino, C1-4 alkoxy C1-4 alkyl group, C1-4 acyl, C1-4 acyloxy, C1-4 acylamino group, C1-4 haloalkyl or C2-4 olefins; 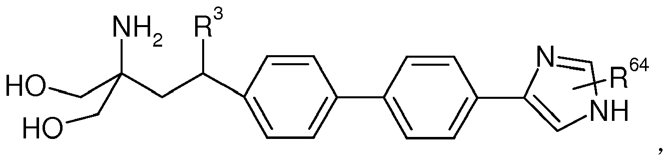 wherein R³ is selected from the group consisting of hydrogen or hydroxy; and
R⁶⁴ is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino and C1-4 alkylamino group, wherein the alkylamino group includes mono- and bis-alkylamino, C1-4 alkoxy C1-4 alkyl group, C1-4 acyl, C1-4 acyloxy, C1-4 acylamino group, C1-4 haloalkyl or C2-4 olefins; 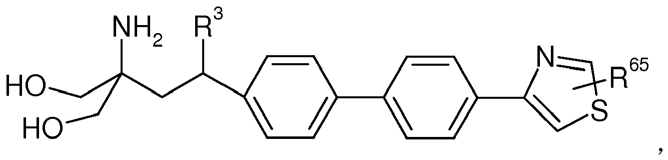 wherein R³ is selected from the group consisting of hydrogen or hydroxy; and
R⁶⁵ is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino and C1-4 alkylamino group, wherein the alkylamino group includes mono- and bis-alkylamino, C1-4 alkoxy C1-4 alkyl group, C1-4 acyl, C1-4 acyloxy, C1-4 acylamino group, C1-4 haloalkyl or C2-4 olefins; or 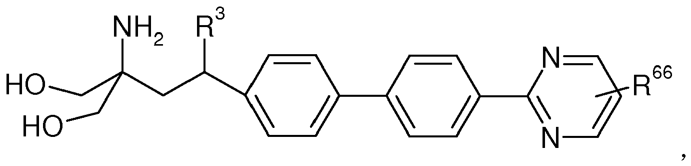 wherein R³ is selected from the group consisting of hydrogen or hydroxy; and
R⁶⁶ is selected from the group consisting of hydrogen, hydroxy, mercapto, amino, aldehyde, carboxyl, carbamoyl, halogen, nitro, cyano, C1-4 alkyl, C1-4 alkoxy, C1-4 alkylamino and C1-4 alkylamino group, wherein the alkylamino group includes mono- and bis-alkylamino, C1-4 alkoxy C1-4 alkyl group, C1-4 acyl, C1-4 acyloxy, C1-4 acylamino group, C1-4 haloalkyl or C2-4 olefins.

13. The compound, or pharmaceutically acceptable salts or esters thereof according to claim 12, **characterized in that** the compound is represented by one of the following general formulae: 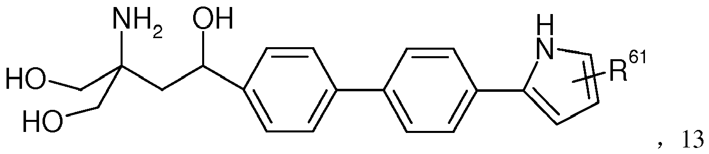 wherein R⁶¹ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 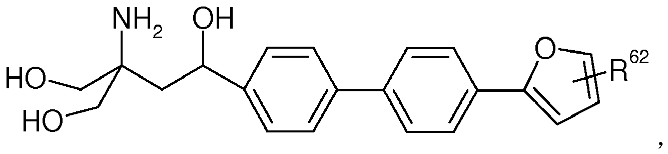 wherein R⁶² is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 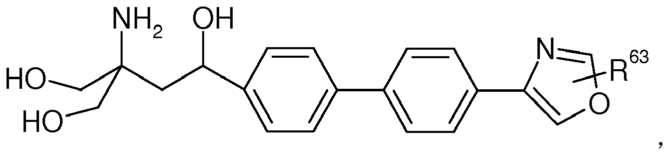 wherein R⁶³ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 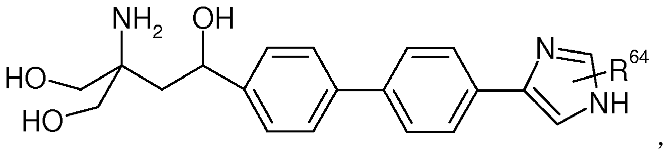 wherein R⁶⁴ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 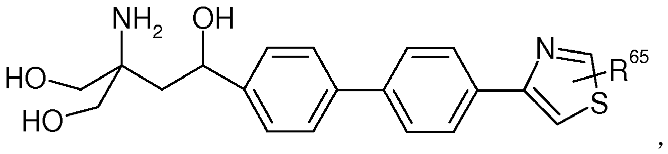 wherein R⁶⁵ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; or 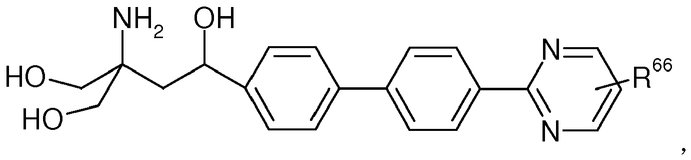 wherein R⁶⁶ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl.

14. The compound, or pharmaceutically acceptable salts or esters thereof according to claim 12, **characterized in that** the compound is represented by one of the following general formulae: 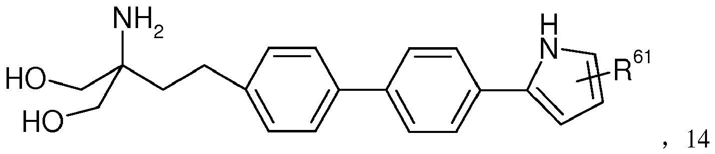 wherein R⁶¹ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 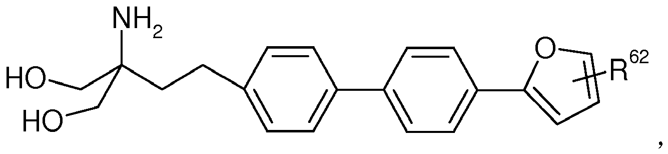 wherein R⁶² is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 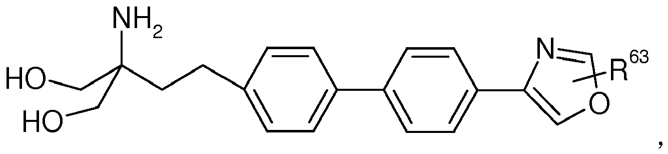 wherein R⁶³ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 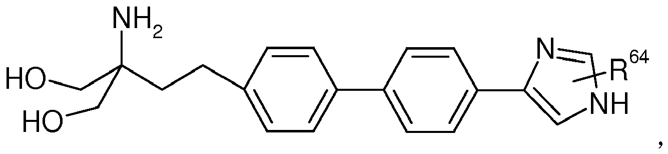 wherein R⁶⁴ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; 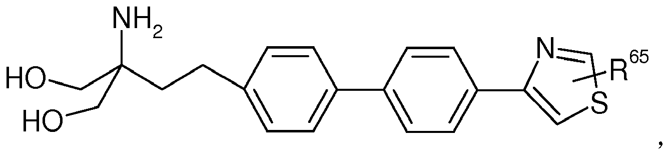 wherein R⁶⁵ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl; or 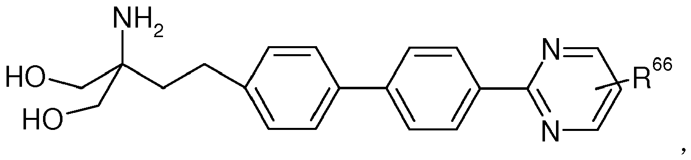 wherein R⁶⁶ is independently selected from the group consisting of hydrogen, hydroxy, mercapto, amino, aldehyde, carboxyl, carbamoyl, fluoro, chloro, bromo, nitro, cyano, methyl, ethyl, propyl, isopropyl, cyclopropyl, n-butyl, isobutyl, tert-butyl, methoxy, ethoxy, propoxy, isopropoxy, cyclopropyloxy, n-butoxy, butoxy, tert-butoxy, methylthio, ethylthio, propylthio, isopropylthio, cyclopropylthio, n-butylthio, isobutylthio, tert-butylthio, methylamino, ethylamino, propylamino, isopropylamino, cyclopropylamino, n-butylamino group, isobutyl group, tert-butylamino group, formyl group, acetyl group, propionyl group, isopropyl group, cyclopropylmethyl group, n-butyryl, isobutyryl group, t-butyl group, formylamino group, acetylamino group, propionamino group, isopropylamino group, cyclopropylmethylamino group, n-butyl amino group, isobutyryl group, amino group, tert-butyl amino group, vinyl group, propenyl, allyl, butenyl.

15. The compound, or pharmaceutically acceptable salts and esters thereof according to any one of claims 1-14, **characterized in that**, the compound is selected from the group consisting of

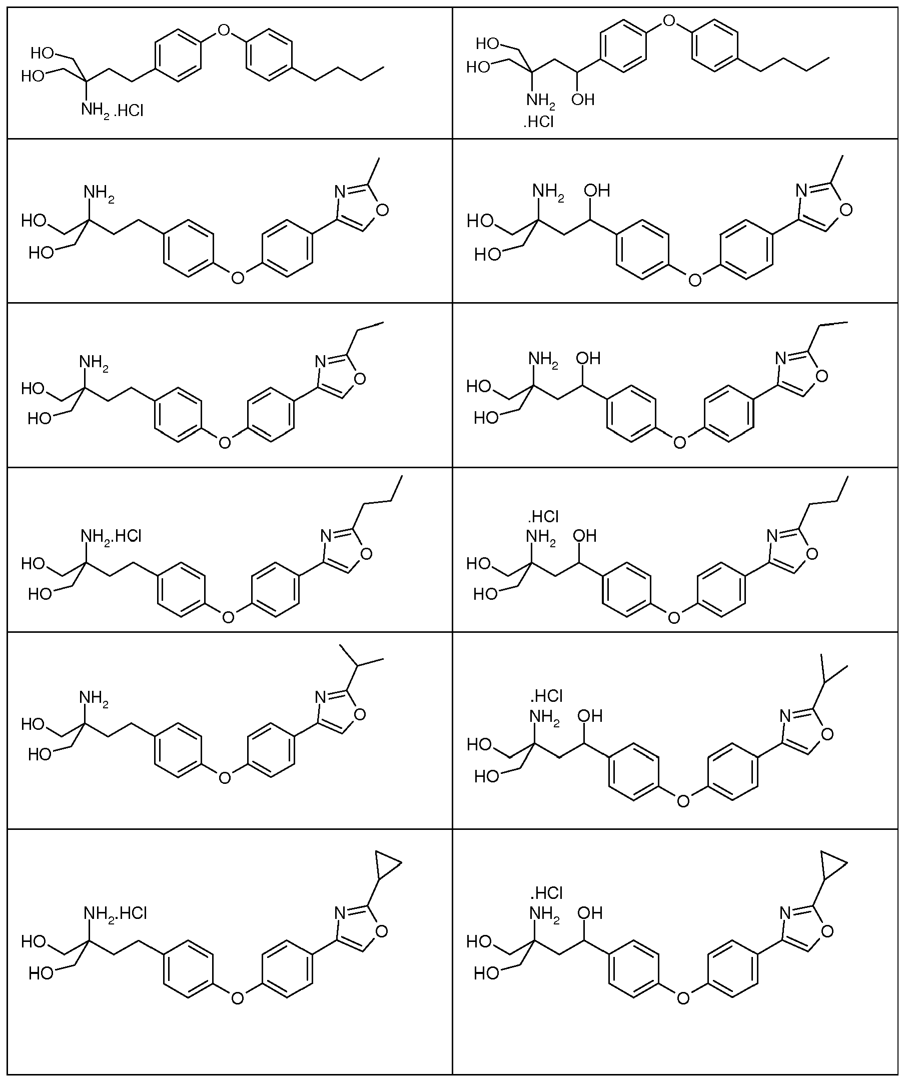

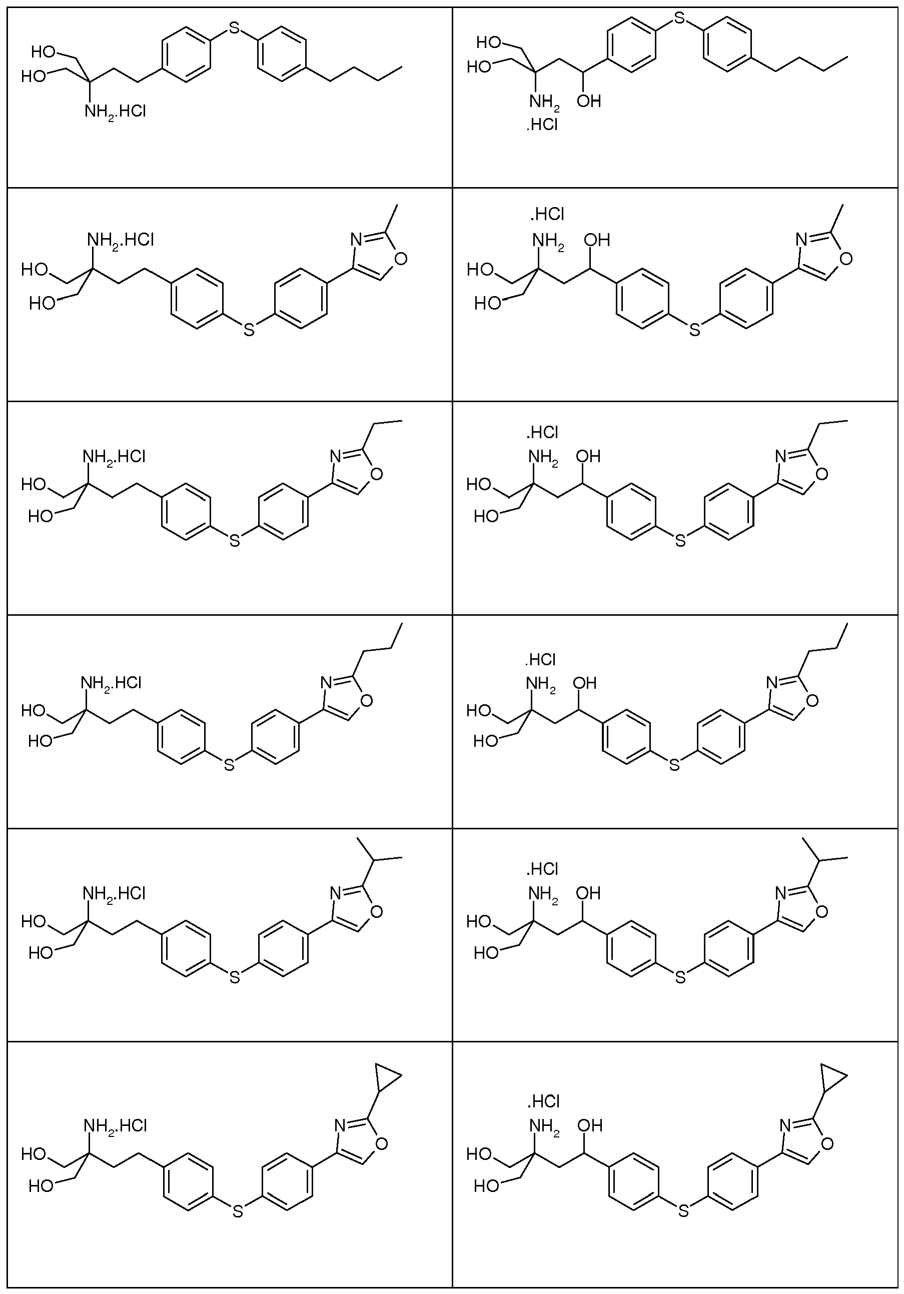

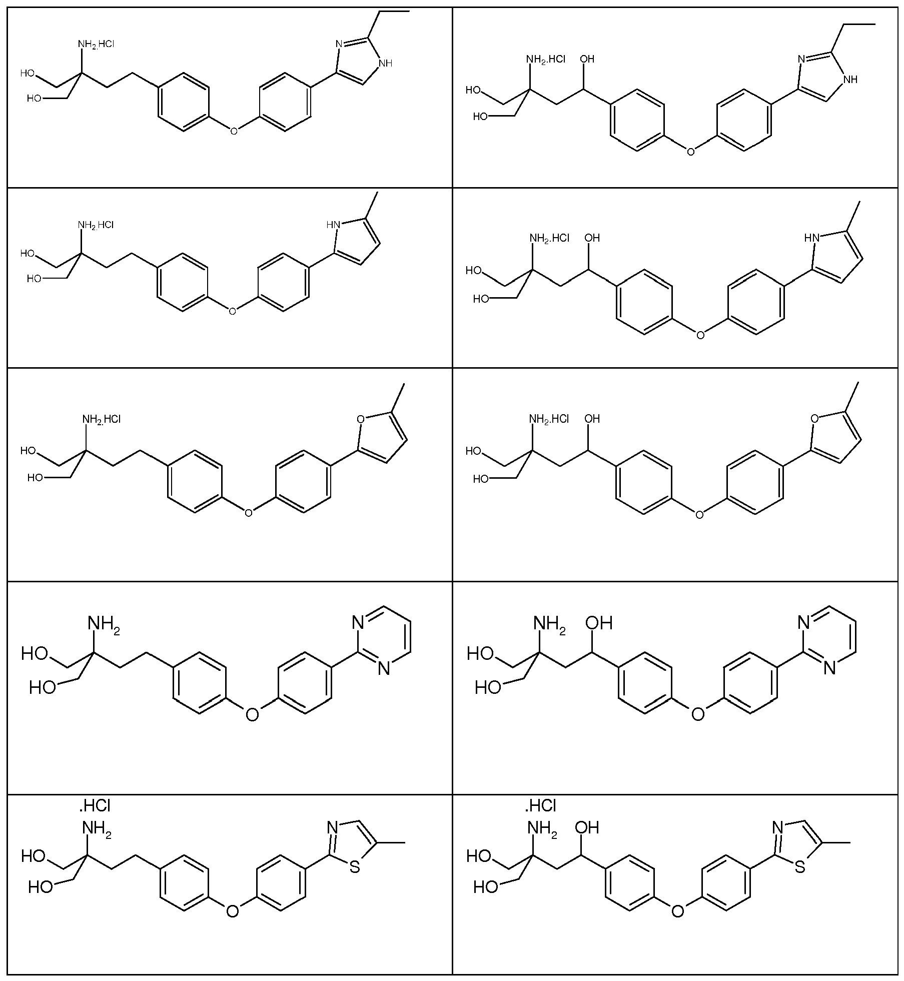

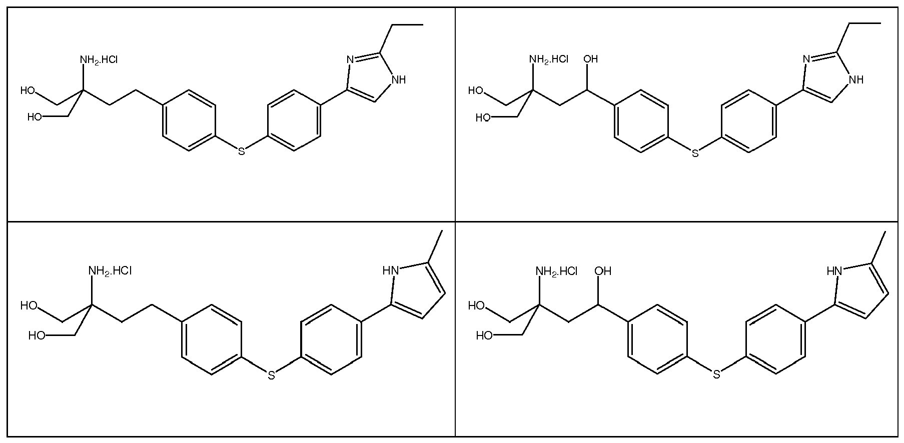

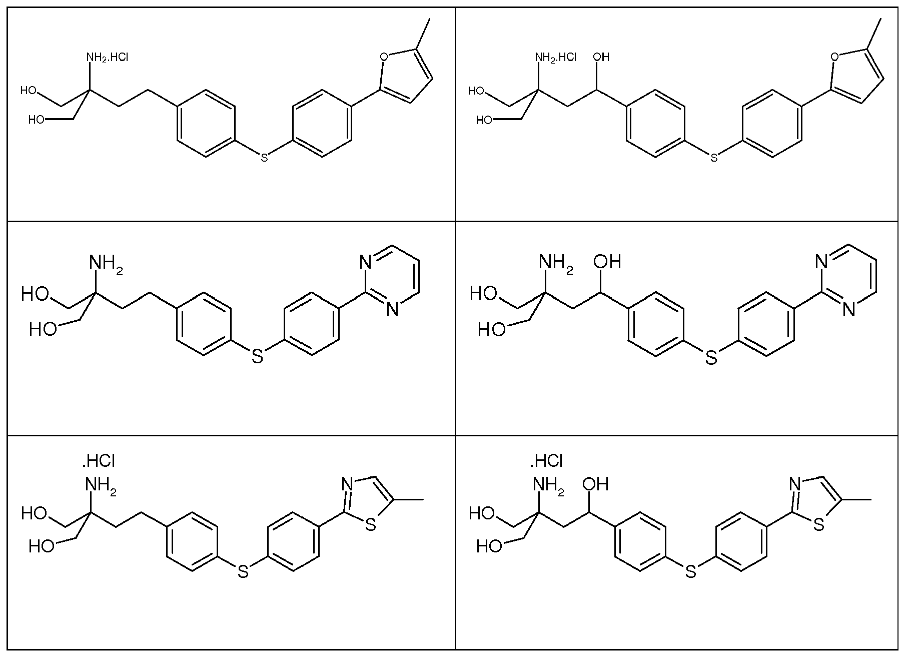

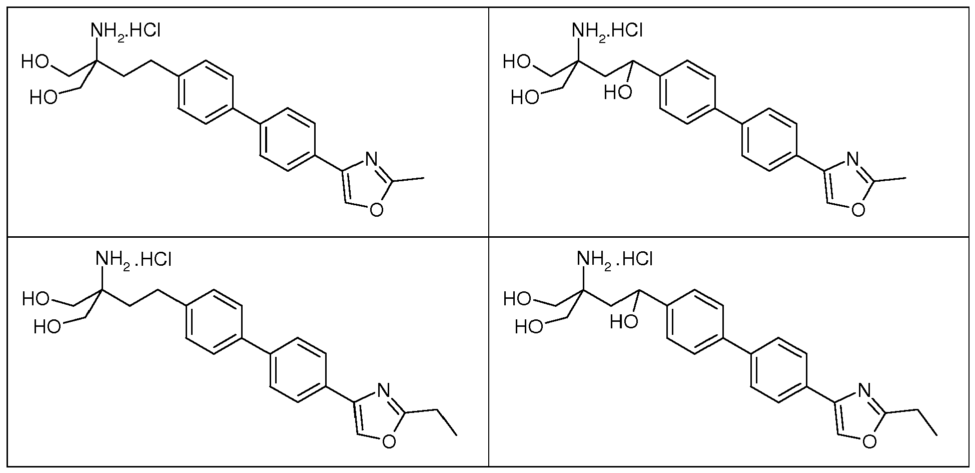

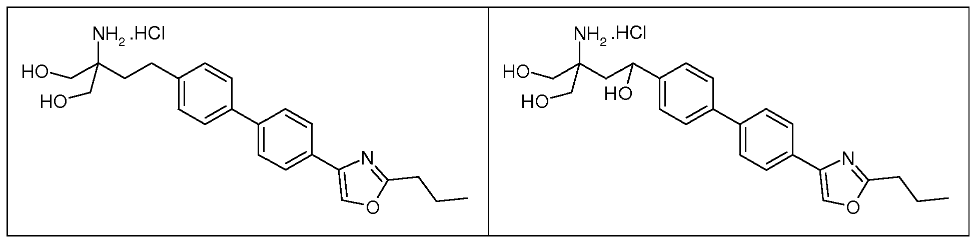

16. A pharmaceutical composition, **characterized in that** it comprises at least one compound according to any one of claims 1-15 as an active ingredient and a pharmaceutically acceptable carrier.

17. Use of the compound according to any one of claims 1-15 in the manufacture of immunomodulators.

18. Use of the compound according to any one of claims 1-15 in the manufacture of a medicament for treatment or prevention of disease relative to immune dysfunction, immunodepression, immunosuppressive, rejection reaction after organ transplantation and/or autoimmune diseases.
